# EUROPEAN PATENT APPLICATION

(11) **EP 3 425 391 A1**
(43) Date of publication of application: **09.01.2019**
(21) Application number: 18175398.9
(22) Date of filing: 05.11.2012
(51) Int. Cl.: G01N 33/50

(54) **DRUG SCREENING AND POTENCY ASSAYS**

(30) Priority: 04.11.2011 US 201161556133 P; 16.11.2011 US 201161560679 P; 01.06.2012 US 201261689245 P
(62) Divisional of application: 12787307.3
(71) Applicant: inRegen, George Town Grand Cayman KY1-1104 (KY)
(72) Inventor: BRUCE, Andrew T., Lexington, NC 27295 (US); KELLEY, Russel W., Winston-Salem, NC 27104 (US); BERTRAM, Timothy A., Winston-Salem, NC 27103 (US); CHOUDHURY, Sumana, Chapel Hill, NC 27516-1177 (US); BASU, Joydeep, Winston-Salem, NC 27103 (US)
(74) Representative: Cripps, Joanna Elizabeth

(57) **Abstract**

The present invention concerns bioactive renal cell populations, in particular a B2 cell population comprising an enriched population of tubular cells and wherein the renal cell population is depleted of a B1 cell population, renal cell constructs, and methods of screening tests agents using the bioactive renal cell populations.

## Description

### Field of the Invention

The present invention relates to methods of screening test agents using bioactive renal cell populations or fractions that lack inactive or undesired cellular components as compared to a healthy individual.

### Background of the Invention

Drug discovery and development consists of an arduous testing process, beginning with the demonstration of pharmacological effects in experimental cell and animal models and ending with drug safety and efficacy studies in patients. A significant number of drug candidates in pre-clinical development fail to progress out of this stage due to unacceptable levels of toxicity in test systems.

Multiple pharmacologic parameters are considered when evaluating a drug candidate. Knowledge of the absorption, distribution, metabolism and excretion profile ("ADME") of a drug and its metabolites in humans (and animals used in toxicology assessments) is crucial to understanding differences in effect among individuals in a population and for optimizing dosimetry. Absorption and bioavailability are standard measures of the amount of biologically active material distributed to the systemic circulation or local site of action. Duration of drug action is often dependent on how rapidly the body eliminates the active molecules, either through metabolism, which involves chemical modification by drug-metabolizing enzymes, or by excretion, which involves binding and transport away from biologically active sites in the body. Thus, typical pre-clinical studies involve monitoring permeation across epithelial membranes (e.g., gastrointestinal mucosa), studies of drug metabolism, identification of plasma protein binding and evaluation of transport into and out of tissues, especially organs that eliminate drug products, such as the kidney and liver.

The high attrition rate of drug candidates is a major economic deterrent in the pharmaceutical industry, as drug failure may be identified only after great time and expense are invested. These failures can be attributed, in part, to a lack of effective pre-clinical models and assay systems. Accordingly, there is a great need in the art to develop an in vitro human system that can effectively evaluate the pharmacologic and toxicologic properties of drug candidates. Improved in vitro model systems will allow the drug development process to reliably predict the in vivo response before the drug reaches the clinic, decreasing time, expense and significant risks to patient health.

### SUMMARY OF THE INVENTION

In one aspect, the present invention provides methods of determining a level of renal toxicity of an test agent comprising a) culturing a plurality of concentrations of a test agent with a heterogeneous renal cell population comprising a B2 cell population comprising an enriched population of tubular cells, and wherein the heterogeneous renal cell population is depleted of a B1 cell population; and b) determining a level of toxicity of said test agent, wherein the presence of at least one toxicity indicator is indicative of the level of renal toxicity of said test agent.

In one embodiment, the heterogeneous renal cell population further comprises a B4 cell population comprising one or more of erythropoietin (EPO)-producing cells, glomerular cells and vascular cells. In another embodiment, the heterogeneous renal cell population further comprises a B3 cell population. In still another embodiment, the heterogeneous renal cell population further comprises a B5 cell population.

In certain embodiments, the cell population is cultured as spheroids. In certain other embodiments, the heterogeneous renal cell population is cultured on a matrix. In one embodiment, the matrix is a three-dimensional (3-D) matrix.

In some embodiments, the toxicity indicator is decreased GGT expression. In other embodiments, the toxicity indicator is a change Aquaporin-1 expression relative to control. In certain other embodiments, the toxicity indicator is a change in Aquaporin-2 expression relative to control. In a further embodiment, the toxicity indicator is LDH.

In another embodiment, the toxicity indicator is a change in phenotype of the cell population relative to control.

In certain embodiments, the determination of the level of renal toxicity of the test agent comprises calculating a TC50 and/or IC50 for the test agent.

In another aspect, the invention provides methods for determining metabolism of a test agent comprising a) incubating a test agent and an enzyme with a heterogeneous renal cell population comprising a B2 cell population comprising an enriched population of tubular cells, and wherein the heterogeneous renal cell population is depleted of a B1 cell population; and b) detecting one or more metabolites of the test agent.

In one embodiment, the heterogeneous renal cell population further comprises a B4 cell population comprising one or more of erythropoietin (EPO)-producing cells, glomerular cells and vascular cells. In another embodiment, the heterogeneous renal cell population further comprises a B3 cell population. In still another embodiment, the heterogeneous renal cell population further comprises a B5 cell population.

In certain embodiments, the cell population is cultured as spheroids. In certain other embodiments, the heterogeneous renal cell population is cultured on a matrix. In one embodiment, the matrix is a three-dimensional (3-D) matrix.

In yet another aspect, the invention provides *in vitro* methods for identifying a test agent suitable for therapeutic use in a human subject having a kidney disorder comprising a) contacting a test agent with a heterogeneous renal cell population comprising a B2 cell population that is characterized by a phenotype selected from the group consisting of expression of a proliferative marker and an M2 phenotype, wherein the B2 cell population comprises an enriched population of tubular cells; and b) determining whether the test agent modulates the expression of a proliferative marker and/or an M2 phenotype of the heterogeneous renal cell population relative to a non-contacted control cell population.

In one embodiment, the heterogeneous renal cell population further comprises a B4 cell population comprising one or more of erythropoietin (EPO)-producing cells, glomerular cells and vascular cells. In another embodiment, the heterogeneous renal cell population further comprises a B3 cell population. In still another embodiment, the heterogeneous renal cell population further comprises a B5 cell population.

In certain embodiments, the cell population is cultured as spheroids. In certain other embodiments, the heterogeneous renal cell population is cultured on a matrix. In one embodiment, the matrix is a three-dimensional (3-D) matrix.

In another aspect, the instant invention provides an organoid comprising a heterogeneous renal cell population comprising a B2 cell population comprising an enriched population of tubular cells, and wherein the heterogeneous renal cell population is depleted of a B1 cell population.

In still another aspect, the instant invention provides a method of forming an organoid comprising a heterogeneous renal cell population comprising a B2 cell population comprising an enriched population of tubular cells, and wherein the heterogeneous renal cell population is depleted of a B1 cell population, comprising culturing the heterogenerous renal cell population in a culture system selected from the group consisting of i) 2D culture; ii) 3D culture: COL(I) gel; iii) 3D culture: Matrigel; iv) 3D culture: spinners, followed by COL(I)/Matrigel; and v) 3D culture: COL(IV) gel.

In still another embodiment, the instant invention provides a method of determining a regenerative potential of a heterogeneous cell population comprising a B2 cell population comprising an enriched population of tubular cells, and wherein the heterogeneous renal cell population is depleted of a B1 cell population, comprising a) culturing the heterogeneous renal cell population; and b) determining the regenerative potential of the heterogeneous cell population, wherein the formation of tubules and/or organoids is indicative of a regenerative potential.

### BRIEF DESCRIPTION OF THE DRAWINGS

Figure 1 shows enrichment of epo-producing cell fraction from freshly-dissociated kidney tissue using a multi-layered step gradient technique (A - left panel) or a single-layer mixing gradient technique (B - right panel). Both methods result in the partial depletion of non epo-producing cell components (predominantly tubular cells) from the epo band, which appears between 1.025 g/mL and 1.035 g/mL.
Figure 2 shows step gradients of "normoxic" (21% oxygen) and "hypoxic" (2% oxygen) rodent cultures that were harvested separately and applied side-by-side to identical step gradients.
Figure 3 shows step gradients of "normoxic" (21% oxygen) and "hypoxic" (2% oxygen) canine cultures that were harvested separately and applied side-by-side to identical step gradients.
Figure 4 shows histopathologic features of the HK17 and HK19 samples.
Figure 5 shows high content analysis (HCA) of albumin transport in human NKA cells defining regions of interest (ROI).
Figure 6 shows quantitative comparison of albumin transport in NKA cells derived from non-CKD and CKD kidney.
Figure 7A depicts comparative analysis of marker expression between tubular-enriched B2 and tubular cell-depleted B4 subfractions.
Figure 7B depicts the phenotypic characterization of selected renal cells (SRCs) across species.
Figure 8 depicts comparative functional analysis of albumin transport between tubular-enriched B2 and tubular cell-depleted B4 subfractions.
Figure 9 depicts the procedure for exposing cells to low oxygen during processing.
Figure 10 shows that upon exposure to 2% Oxygen, the following was observed: alters distribution of cells across a density gradient, improves overall post-gradient yield
Figures 11A depicts an assay developed to observe repair of tubular monolayers in vitro. Figure 11B shows results of a Quantitative Image Analysis (BD Pathway 855 BioImager). Figure 11C shows cells induced with 2% oxygen to be more proficient at repair of tubular epithelial monolayers.
Figure 12A depicts an assay developed to observe repair of tubular monolayers in vitro. Figure 12B shows that the induction of cells with 2% Oxygen enhanced the migration and wound repair compared to un-induced (21 % oxygen). Figure 12C plots the % of migrated cells against migration time.
Figure 13A shows that osteopontin is secreted by tubular cells and is upregulated in response to injury (Osteopontin Immunocytochemistry: Hoechst nuclear stain (blue), Osteopontin (Red), 10x). Osteopontin is upregulated by injury in established tubular cell monolayers as shown by immunoflluorescence (Figure 13A) and ELISA (Figure 13B).
Figure 14A shows that the migratory response of cells is mediated in part by osteopontin (Green = migrated cells (5x)). Figure 14B shows that neutralizing antibodies (NAb) to osteopontin reduce renal cell migration response by 50%.
Figure 15 shows that low-oxygen induction of cells modulates expression of tissue remodeling genes.
Figure 16 depicts a putative mechanism for low oxygen augmentation of bioactivity of cells leading to renal regeneration.
Figure 17 shows Orbital Roatator with low bind plates.
Figure 18 shows Spinner flasks with Human cells.
Figure 19 depicts Spheroids.

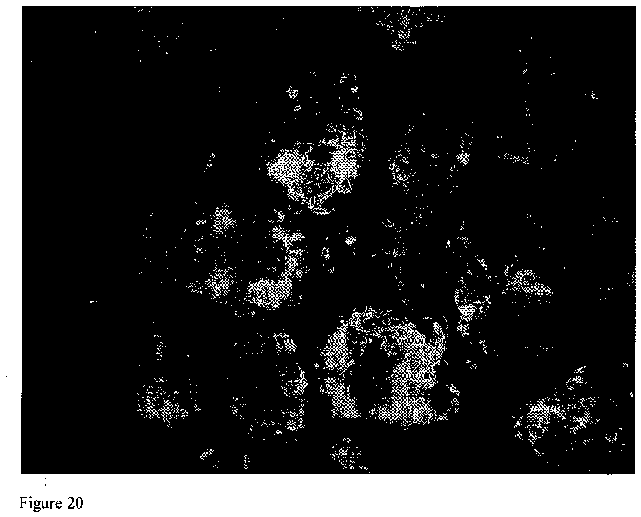

Figure 20 depicts NKCC2 green; nucleus- blue.

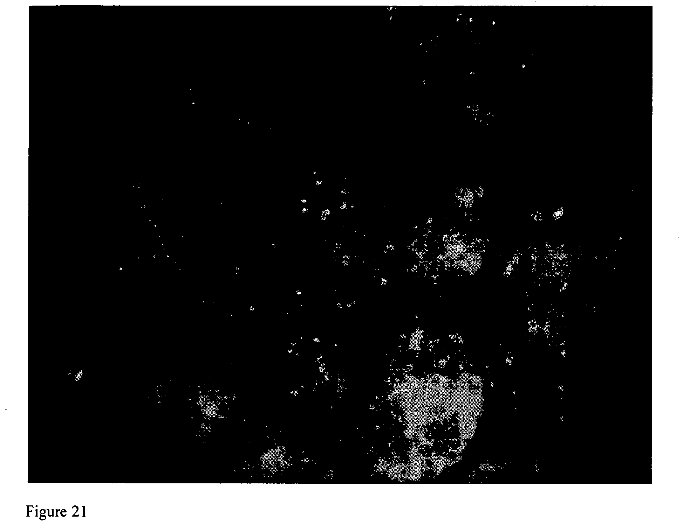

Figure 21 depicts GGT-1 green; nucleus- blue.

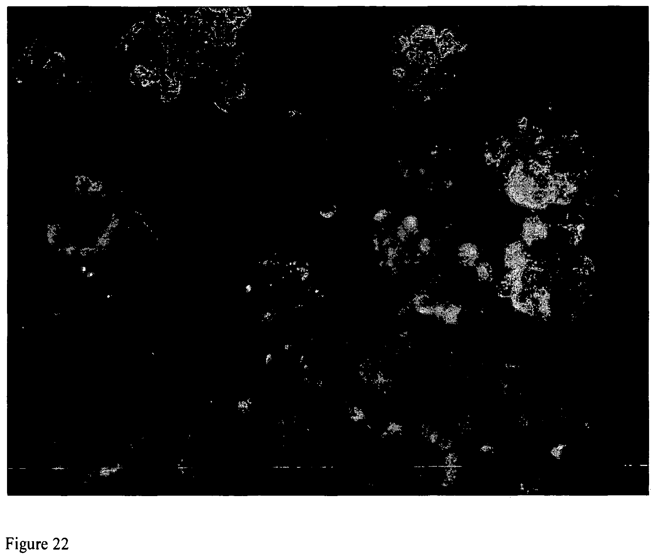

Figure 22 depicts Aquaporin1 green; nucleus- blue.

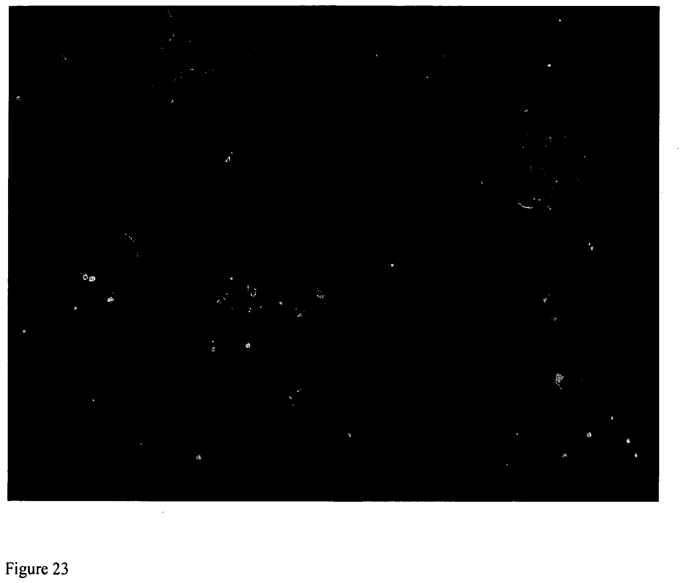

Figure 23 depicts Leucine Aminopeptidase 3 red; nucleus blue.

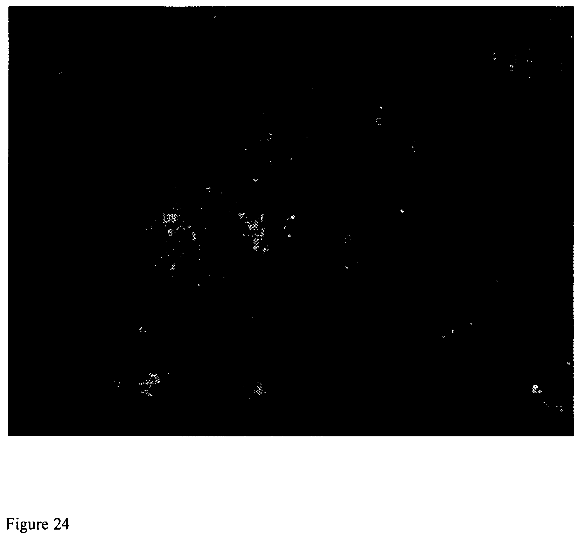

Figure 24 depicts Organic Ion Transporter 1(OAT1) red; nucleus blue.

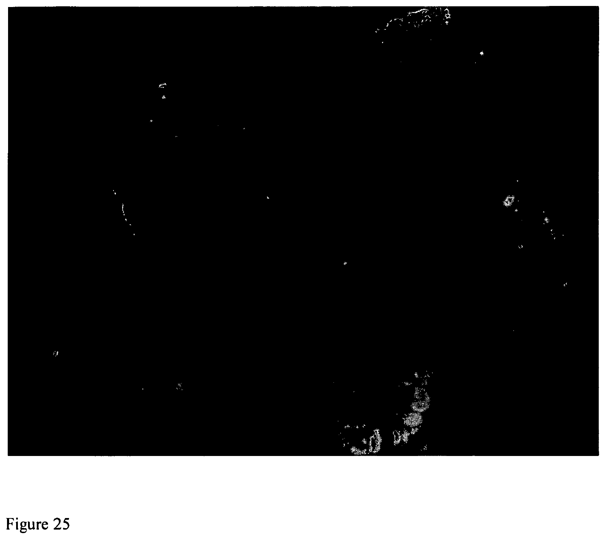

Figure 25 depicts Cubilin red; nucleus blue.
Figure 26 depicts Cisplatin effects on viability in spheroid cultures after 48 hour exposure.
Figure 27 shows a quantitation of the GGT-1 activity following 48 hours of Cisplatin exposure to 3D tubular organoid cultures.
Figure 28 depicts TC₅₀ from semilogarithmic dose response curve with Amphotericin B.
Figure 29A depicts morphological and viability changes post 72hr exposure to Amphotericin B.
Figure 29B shows the the percent change from untreated control by Presto Blue post 72hr exposure to Amphotericin B.
Figure 30 depicts 2D tube formation from 2-week HK cultures.
Figure 31 shows 2D culture at 18 days.
Figure 32 depicts 2D basal culture at 18 days.
Figure 33 shows 2D basal culture at 14 days.
Figure 34 shows characterization of 2D cultured tubular structures with renal tubule markers.
Figure 35 depicts 3D tube formation in COL(I) gels at day 5.
Figure 36 shows 3D COL(I) gel culture at 7 days.
Figure 37 shows 3D COL(I) gel culture at 11 days.
Figure 38 depicts 3D tube formation at 2 weeks in COL(I) gel culture.
Figure 39 shows 3D tube formation at 2 weeks in COL(I) gel culture.
Figure 40 shows human foreskin fibroblasts (HFF) in COL(I) gel culture at two weeks.
Figure 41 depicts four day spinner culture, 2 days Matrigel.
Figure 42 shows four day spinner culture, 3 days Matrigel.
Figure 43 depicts four day spinner culture, 7 days Matrigel.
Figure 44 depicts low bind plate 4 days, 7 days Matrigel.

### DETAILED DESCRIPTION OF THE INVENTION

The present invention is directed to heterogenous mixtures or fractions of bioactive renal cells (BRCs), methods of isolating and culturing the same, as well as methods of screening test agents using BRCs as described herein. The present invention is also directed to organoids comprising and/or formed from heterogenous mixtures or fractions of bioactive renal cells, methods of forming and culturing the same, as well as methods of determining the bioactive potential or potency of the heterogenous mixtures or fractions of bioactive renal cells. The bioactive renal cells may be isolated renal cells including tubular and erythropoietin (EPO)-producing kidney cells. The BRC cell populations may include enriched tubular and EPO-producing cell populations. The BRCs may be derived from or are themselves renal cell fractions from healthy individuals. In addition, the present invention provides renal cell fractions obtained from an unhealthy individual may lack certain cellular components when compared to the corresponding renal cell fractions of a healthy individual, yet still retain therapeutic properties. The present invention also provides therapeutically-active cell populations lacking cellular components compared to a healthy individual, which cell populations can be, in one embodiment, isolated and expanded from autologous sources in various disease states.

### Definitions

Unless defined otherwise, technical and scientific terms used herein have the same meaning as commonly understood by one of ordinary skill in the art to which this invention belongs. Principles of Tissue Engineering, 3rd Ed. (Edited by R Lanza, R Langer, & J Vacanti), 2007 provides one skilled in the art with a general guide to many of the terms used in the present application. One skilled in the art will recognize many methods and materials similar or equivalent to those described herein, which could be used in the practice of the present invention. Indeed, the present invention is in no way limited to the methods and materials described.

The term "cell population" as used herein refers to a number of cells obtained by isolation directly from a suitable tissue source, usually from a mammal. The isolated cell population may be subsequently cultured *in vitro.* Those of ordinary skill in the art will appreciate that various methods for isolating and culturing cell populations for use with the present invention and various numbers of cells in a cell population that are suitable for use in the present invention. A cell population may be an unfractionated, heterogeneous cell population derived from the kidney. For example, a heterogeneous cell population may be isolated from a kidney biopsy or from whole kidney tissue. Alternatively, the heterogeneous cell population may be derived from *in vitro* cultures of mammalian cells, established from kidney biopsies or whole kidney tissue. An unfractionated heterogeneous cell population may also be referred to as a non-enriched cell population.

The term "native kidney" shall mean the kidney of a living subject. The subject may be healthy or un-healthy. An unhealthy subject may have a kidney disease.

The term "regenerative effect" shall mean an effect which provides a benefit to a native kidney. The effect may include, without limitation, a reduction in the degree of injury to a native kidney or an improvement in, restoration of, or stabilization of a native kidney function. Renal injury may be in the form of fibrosis, inflammation, glomerular hypertrophy, etc. and related to kidney disease in the subject.

The term "regenerative potential" or "potential regenerative bioactivity" as used herein refers to the potential of the bioactive cell preparations and/or admixtures described herein to provide a regenerative effect.

The term "admixture" as used herein refers to a combination of two or more isolated, enriched cell populations derived from an unfractionated, heterogeneous cell population. According to certain embodiments, the cell populations of the present invention are renal cell populations.

An "enriched" cell population or preparation refers to a cell population derived from a starting kidney cell population (e.g., an unfractionated, heterogeneous cell population) that contains a greater percentage of a specific cell type than the percentage of that cell type in the starting population. For example, a starting kidney cell population can be enriched for a first, a second, a third, a fourth, a fifth, and so on, cell population of interest. As used herein, the terms "cell population", "cell preparation" and "cell prototype" are used interchangeably.

In one aspect, the term "enriched" cell population as used herein refers to a cell population derived from a starting kidney cell population (e.g., a cell suspension from a kidney biopsy or cultured mammalian kidney cells) that contains a percentage of cells capable of producing EPO that is greater than the percentage of cells capable of producing EPO in the starting population. For example, the term "B4" is a cell population derived from a starting kidney cell population that contains a greater percentage of EPO-producing cells, glomerular cells, and vascular cells as compared to the starting population. The cell populations of the present invention may be enriched for one or more cell types and depleted of one or more other cell types. For example, an enriched EPO-producing cell population may be enriched for interstitial fibroblasts and depleted of tubular cells and collecting duct epithelial cells relative to the interstitial fibroblasts and tubular cells in a non-enriched cell population, i.e. the starting cell population from which the enriched cell population is derived. In all embodiments citing EPO-enriched or "B4" populations, the enriched cell populations are heterogeneous populations of cells containing cells that can produce EPO in an oxygen-regulated manner, as demonstrated by oxygen-tunable EPO expression from the endogenous native EPO gene.

In another aspect, an enriched cell population, which contains a greater percentage of a specific cell type, *e.g*., vascular, glomerular, or endocrine cells, than the percentage of that cell type in the starting population, may also lack or be deficient in one or more specific cell types, e.g., vascular, glomerular, or endocrine cells, as compared to a starting kidney cell population derived from a healthy individual or subject. For example, the term "B4'," or B4 prime," in one aspect, is a cell population derived from a starting kidney cell population that lacks or is deficient in one or more cell types, *e.g*., vascular, glomerular or endocrine, depending on the disease state of the starting specimen, as compared to a healthy individual. In one embodiment, the B4' cell population is derived from a subject having chronic kidney disease. In one embodiment, the B4' cell population is derived from a subject having focal segmental glomerulosclerosis (FSGS). In another embodiment, the B4' cell population is derived from a subject having autoimmune glomerulonephritis. In another aspect, B4' is a cell population derived from a starting cell population including all cell types, *e*.*g*., vascular, glomerular, or endocrine cells, which is later depleted of or made deficient in one or more cell types, *e.g.,* vascular, glomerular, or endocrine cells. In yet another aspect, B4' is a cell population derived from a starting cell population including all cell types, *e*.*g*., vascular, glomerular, or endocrine cells, in which one or more specific cell types *e*.*g*., vascular, glomerular, or endocrine cells, is later enriched. For example, in one embodiment, a B4' cell population may be enriched for vascular cells but depleted of glomerular and/or endocrine cells. In another embodiment, a B4' cell population may be enriched for glomerular cells but depleted of vascular and/or endocrine cells. In another embodiment, a B4' cell population may be enriched for endocrine cells but depleted of vascular and/or glomerular cells. In another embodiment, a B4' cell population may be enriched for vascular and endocrine cells but depleted of glomerular cells. In preferred embodiments, the B4' cell population, alone or admixed with another enriched cell population, *e.g*., B2 and/or B3, retains therapeutic properties. A B4' cell population, for example, is described herein in the Examples, e.g., Examples 7-9.

In another aspect, an enriched cell population may also refer to a cell population derived from a starting kidney cell population as discussed above that contains a percentage of cells expressing one or more vascular, glomerular and proximal tubular markers with some EPO-producing cells that is greater than the percentage of cells expressing one or more vascular, glomerular and proximal tubular markers with some EPO-producing cells in the starting population. For example, the term "B3" refers to a cell population derived from a starting kidney cell population that contains a greater percentage of proximal tubular cells as well as vascular and glomerular cells as compared to the starting population. In one embodiment, the B3 cell population contains a greater percentage of proximal tubular cells as compared to the starting population but a lesser percentage of proximal tubular cells as compared to the B2 cell population. In another embodiment, the B3 cell population contains a greater percentage of vascular and glomerular cells markers with some EPO-producing cells as compared to the starting population but a lesser percentage of vascular and glomerular cells markers with some EPO-producing cells as compared to the B4 cell population.

In another aspect, an enriched cell population may also refer to a cell population derived from a starting kidney cell population as discussed above that contains a percentage of cells expressing one or more tubular cell markers that is greater than the percentage of cells expressing one or more tubular cell markers in the starting population. For example, the term "B2" refers to a cell population derived from a starting kidney cell population that contains a greater percentage of tubular cells as compared to the starting population. In addition, a cell population enriched for cells that express one or more tubular cell markers (or "B2") may contain some epithelial cells from the collecting duct system. Although the cell population enriched for cells that express one or more tubular cell markers (or "B2") is relatively depleted of EPO-producing cells, glomerular cells, and vascular cells, the enriched population may contain a smaller percentage of these cells (EPO-producing, glomerular, and vascular) in comparison to the starting population. In general, a heterogeneous cell population is depleted of one or more cell types such that the depleted cell population contains a lesser proportion of the cell type(s) relative to the proportion of the cell type(s) contained in the heterogeneous cell population prior to depletion. The cell types that may be depleted are any type of kidney cell. For example, in certain embodiments, the cell types that may be depleted include cells with large granularity of the collecting duct and tubular system having a density of < about 1.045 g/ml, referred to as "B1". In certain other embodiments, the cell types that may be depleted include debris and small cells of low granularity and viabilty having a density of > about 1.095 g/ml, referred to as "B5". In some embodiments, the cell population enriched for tubular cells is relatively depleted of all of the following: "B1", "B5", oxygen-tunable EPO-expressing cells, glomerular cells, and vascular cells.

The term "hypoxic" culture conditions as used herein refers to culture conditions in which cells are subjected to a reduction in available oxygen levels in the culture system relative to standard culture conditions in which cells are cultured at atmospheric oxygen levels (about 21%). Non-hypoxic conditions are referred to herein as normal or normoxic culture conditions.

The term "oxygen-tunable" as used herein refers to the ability of cells to modulate gene expression (up or down) based on the amount of oxygen available to the cells. "Hypoxia-inducible" refers to the upregulation of gene expression in response to a reduction in oxygen tension (regardless of the pre-induction or starting oxygen tension).

The term "biomaterial" as used here refers to a natural or synthetic biocompatible material that is suitable for introduction into living tissue. A natural biomaterial is a material that is made by a living system. Synthetic biomaterials are materials which are not made by a living system. The biomaterials disclosed herein may be a combination of natural and synthetic biocompatible materials. As used herein, biomaterials include, for example, polymeric matrices and scaffolds. Those of ordinary skill in the art will appreciate that the biomaterial(s) may be configured in various forms, for example, as liquid hydrogel suspensions, porous foam, and may comprise one or more natural or synthetic biocompatible materials.

The term "construct" refers to one or more cell populations deposited on or in a surface of a scaffold or matrix made up of one or more synthetic or naturally-occurring biocompatible materials. The one or more cell populations may be coated with, deposited on, embedded in, attached to, seeded, or entrapped in a biomaterial made up of one or more synthetic or naturally-occurring biocompatible polymers, proteins, or peptides. The one or more cell populations may be combined with a biomaterial or scaffold or matrix *in vitro* or *in vivo.* In general, the one or more biocompatible materials used to form the scaffold/biomaterial is selected to direct, facilitate, or permit the formation of multicellular, three-dimensional, organization of at least one of the cell populations deposited thereon. The one or more biomaterials used to generate the construct may also be selected to direct, facilitate, or permit dispersion and/or integration of the construct or cellular components of the construct with the endogenous host tissue, or to direct, facilitate, or permit the survival, engraftment, tolerance, or functional performance of the construct or cellular components of the construct.

The term "marker" or "biomarker" refers generally to a DNA, RNA, protein, carbohydrate, or glycolipid-based molecular marker, the expression or presence of which in a cultured cell population can be detected by standard methods (or methods disclosed herein) and is consistent with one or more cells in the cultured cell population being a particular type of cell. The marker may be a polypeptide expressed by the cell or an identifiable physical location on a chromosome, such as a gene, a restriction endonuclease recognition site or a nucleic acid encoding a polypeptide (e.g., an mRNA) expressed by the native cell. The marker may be an expressed region of a gene referred to as a "gene expression marker", or some segment of DNA with no known coding function. The biomarkers may be cell-derived, e.g., secreted, products.

The terms "differentially expressed gene," "differential gene expression" and their synonyms, which are used interchangeably, refer to a gene whose expression is activated to a higher or lower level in a first cell or cell population, relative to its expression in a second cell or cell population. The terms also include genes whose expression is activated to a higher or lower level at different stages over time during passage of the first or second cell in culture. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example. Differential gene expression may include a comparison of expression between two or more genes or their gene products, or a comparison of the ratios of the expression between two or more genes or their gene products, or even a comparison of two differently processed products of the same gene, which differ between the first cell and the second cell. Differential expression includes both quantitative, as well as qualitative, differences in the temporal or cellular expression pattern in a gene or its expression products among, for example, the first cell and the second cell. For the purpose of this invention, "differential gene expression" is considered to be present when there is a difference between the expression of a given gene in the first cell and the second cell. The differential expression of a marker may be in cells from a patient before administration of a cell population, admixture, or construct (the first cell) relative to expression in cells from the patient after administration (the second cell).

The terms "inhibit", "down-regulate", "under-express" and "reduce" are used interchangeably and mean that the expression of a gene, or level of RNA molecules or equivalent RNA molecules encoding one or more proteins or protein subunits, or activity of one or more proteins or protein subunits, is reduced relative to one or more controls, such as, for example, one or more positive and/or negative controls. The under-expression may be in cells from a patient before administration of a cell population, admixture, or construct relative to cells from the patient after administration.

The term "up-regulate" or "over-express" is used to mean that the expression of a gene, or level of RNA molecules or equivalent RNA molecules encoding one or more proteins or protein subunits, or activity of one or more proteins or protein subunits, is elevated relative to one or more controls, such as, for example, one or more positive and/or negative controls. The over-expression may be in cells from a patient after administration of a cell population, admixture, or construct relative to cells from the patient before administration.

The term "subject" shall mean any single human subject, including a patient, eligible for treatment, who is experiencing or has experienced one or more signs, symptoms, or other indicators of a kidney disease, anemia, or EPO deficiency. Such subjects include without limitation subjects who are newly diagnosed or previously diagnosed and are now experiencing a recurrence or relapse, or are at risk for a kidney disease, anemia, or EPO deficiency, no matter the cause. The subject may have been previously treated for a kidney disease, anemia, or EPO deficiency, or not so treated.

The term "patient" refers to any single animal, more preferably a mammal (including such non-human animals as, for example, dogs, cats, horses, rabbits, zoo animals, cows, pigs, sheep, and non-human primates) for which treatment is desired. Most preferably, the patient herein is a human.

The term "sample" or "patient sample" or "biological sample" shall generally mean any biological sample obtained from a subject or patient, body fluid, body tissue, cell line, tissue culture, or other source. The term includes tissue biopsies such as, for example, kidney biopsies. The term includes cultured cells such as, for example, cultured mammalian kidney cells. Methods for obtaining tissue biopsies and cultured cells from mammals are well known in the art. If the term "sample" is used alone, it shall still mean that the "sample" is a "biological sample" or "patient sample", *i.e*., the terms are used interchangeably.

The term "test sample" refers to a sample from a subject that has been treated by a method of the present invention. The test sample may originate from various sources in the mammalian subject including, without limitation, blood, semen, serum, urine, bone marrow, mucosa, tissue, etc.

The term "control" or "control sample" refers a negative or positive control in which a negative or positive result is expected to help correlate a result in the test sample. Controls that are suitable for the present invention include, without limitation, a sample known to exhibit indicators characteristic of normal erythroid homeostasis, a sample known to exhibit indicators characteristic of anemia, a sample obtained from a subject known not to be anemic, and a sample obtained from a subject known to be anemic. Additional controls suitable for use in the methods of the present invention include, without limitation, samples derived from subjects that have been treated with pharmacological agents known to modulate erythropoiesis (e.g., recombinant EPO or EPO analogs). In addition, the control may be a sample obtained from a subject prior to being treated by a method of the present invention. An additional suitable control may be a test sample obtained from a subject known to have any type or stage of kidney disease, and a sample from a subject known not to have any type or stage of kidney disease. A control may be a normal healthy matched control. Those of skill in the art will appreciate other controls suitable for use in the present invention.

As used herein, the term "toxicity" is defined as any unwanted effect on human cells or tissue caused by a test agent, or test agent used in combination with other pharmaceuticals, including unwanted or overly exaggerated pharmacological effects. An analogous term used in this context is "adverse reaction."

In the pharmaceutical arts, the term "efficacy" can describe the strength of a response in a tissue produced from a single drug-receptor complex. In the context of this disclosure, "efficacy" can also be defined as a response elicited by a drug or test agent that improves the phenotype of a cell or tissue.

A "test agent" or "test compound" is any substance that is evaluated for its ability to diagnose, cure, mitigate, treat, or prevent disease in a subject, or is intended to alter the structure or function of the body of a subject. A test agent in an embodiment can be a "drug" as that term is defined under the Food Drug and Cosmetic Act, §321(g)(1). Test agents include, but are not limited to, chemical compounds, biologic agents, proteins, peptides, nucleic acids, lipids, polysaccharides, supplements, diagnostic agents and immune modulators.

"Pharmacokinetics" refers to the actions of the body on a drug. Pharmacokinetic processes include, but are not limited to, absorption, distribution, metabolism, and elimination of drugs.

"Pharmacodynamics" refers to the actions of a drug on the body. Because certain classes of drugs exhibit similar effects on the body, pharmacodynamic properties determine the group in which a drug or agent is classified.

"Phase I metabolism" refers to biochemical reactions that usually convert the parent drug, agent, or compound by introducing or unmasking a functional group, including but not limited to, hydroxyl, amino, or sulfhydryl groups. The products of Phase I metabolism are often inactive, though in some instances activity is only modified or even higher than the parent drug.

"Phase II metabolism" encompasses biochemical reactions that couple or conjugate polar molecules to parent drugs or their phase I metabolites that contain suitable functional groups for conjugation. Phase II metabolic reactions require energy. Phase II metabolism can occur before or in the absence of Phase I reactions.

The term "potency" refers to a measure of a biological activity of a therapeutic agent, *e.g*., a cell-based therapy. In one embodiment, potency assays are quantitative metrics that reflect the mechanism of action underlying the therapeutic response as a validation of the manufacturing process. In other embodiments, potency assays may be used to evaluate modifications to manufacturing or to the nature of the product itself.

The term "spheroid" refers to an aggregate or assembly of cells cultured to allow 3D growth as opposed to growth as a monolayer. It is noted that the term "spheroid" does not imply that the aggregate is a geometric sphere. The aggregate may be highly organized with a well defined morphology or it may be an unorganized mass; it may include a single cell type or more than one cell type. The cells may be primary isolates, or a permanent cell line, or a combination of the two. Included in this definition are organoids and organotypic cultures.

The term "organoid" as used herein refers to a heterogeneous 3D agglomeration of cells that recapitulates aspects of cellular self-organization, architecture and signaling interactions present in the native organ. The term "organoid" includes spheroids or cell clusters formed from suspension cell cultures.

### Cell populations

Isolated, heterogeneous populations of kidney cells, and admixtures thereof, enriched for specific bioactive components or cell types and/or depleted of specific inactive or undesired components or cell types for use in the treatment of kidney disease, *i.e*., providing stabilization and/or improvement and/or regeneration of kidney function, were previously described in U.S. Application No. 12/617,721 filed November 12, 2009. The present invention provides methods of screening test agents using isolated renal cell fractions that lack cellular components as compared to a healthy individual yet retain therapeutic properties, *i.e*., provide stabilization and/or improvement and/or regeneration of kidney function. The cell populations, cell fractions, and/or admixtures of cells described herein may be derived from healthy individuals, individuals with a kidney disease, or subjects as described herein.

### Bioactive cell populations

The present invention contemplates methods of screening test agents using certain subfractions of a heterogeneous population of renal cells, enriched for bioactive components and depleted of inactive or undesired components, which provide superior therapeutic and regenerative outcomes than the starting population. The present invention further contemplates organoids comprising certain subfractions of a heterogeneous population of renal cells, enriched for bioactive components and depleted of inactive or undesired components. For example, bioactive components of the invention, *e*.*g*., B2, B4, and B3, which are depleted of inactive or undesired components, *e*.*g*., B1 and B5, alone or admixed, provide unexpected improvement in drug screening. Organoids comprising and/or formed from bioactive components of the invention, *e.g*., B2, B4, and B3, which are depleted of inactive or undesired components, *e.g*., B1 and B5, alone or admixed, are surprisingly useful in *in vitro* potency assays for determining the potential regenerative bioactivity *in vivo* (i.e., potency) of the bioactive components.

In one aspect, the present invention provides methods of screening test agents using a specific subfraction, B4, depleted of or deficient in one or more cell types, *e*.*g*., vascular, endocrine, or endothelial, *i.e*., B4', retains therapeutic properties, *e.g*., stabilization and/or improvement and/or regeneration of kidney function, alone or when admixed with other bioactive subfractions, *e*.*g*., B2 and/or B3. In a preferred embodiment, the bioactive cell population is B2. In certain embodiments, the B2 cell population is admixed with B4 or B4'. In other embodiments, the B2 cell population is admixed with B3. In other embodiments, the B2 cell population is admixed with both B3 and B4, or specific cellular components of B3 and/or B4.

The B2 cell population is characterized by expression of a tubular cell marker selected from the group consisting of one or more of the following: megalin, cubilin, hyaluronic acid synthase 2 (HAS2), Vitamin D3 25-Hydroxylase (CYP2D25), N-cadherin (Ncad), E-cadherin (Ecad), Aquaporin-1 (Aqp1), Aquaporin-2 (Aqp2), RAB17, member RAS oncogene family (Rab17), GATA binding protein 3 (Gata3), FXYD domain-containing ion transport regulator 4 (Fxyd4), solute carrier family 9 (sodium/hydrogen exchanger), member 4 (Slc9a4), aldehyde dehydrogenase 3 family, member B1 (Aldh3b1), aldehyde dehydrogenase 1 family, member A3 (Aldh1a3), and Calpain-8 (Capn8), and collecting duct marker Aquaporin-4 (Aqp4). B2 is larger and more granulated than B3 and/or B4 and thus having a buoyant density between about 1.045 g/ml and about 1.063 g/ml (rodent), between about 1.045 g/ml and 1.052 g/ml (human), and between about 1.045 g/ml and about 1.058 g/ml (canine).

The B3 cell population is characterized by the expression of vascular, glomerular and proximal tubular markers with some EPO-producing cells, being of an intermediate size and granularity in comparison to B2 and B4, and thus having a buoyant density between about 1.063 g/ml and about 1.073 g/ml (rodent), between about 1.052 g/ml and about 1.063 g/ml (human), and between about 1.058 g/ml and about 1.063 g/ml (canine). B3 is characterized by expression of markers selected from the group consisting of one or more of the following: aquaporin 7 (Aqp7), FXYD domain-containing ion transport regulator 2 (Fxyd2), solute carrier family 17 (sodium phosphate), member 3 (Slc17a3), solute carrier family 3, member 1 (Slc3a1), claudin 2 (Cldn2), napsin A aspartic peptidase (Napsa), solute carrier family 2 (facilitated glucose transporter), member 2 (Slc2a2), alanyl (membrane) aminopeptidase (Anpep), transmembrane protein 27 (Tmem27), acyl-CoA synthetase medium-chain family member 2 (Acsm2), glutathione peroxidase 3 (Gpx3), fructose-1,6- biphosphatase 1 (Fbp1), and alanine-glyoxylate aminotransferase 2 (Agxt2). B3 is also characterized by the vascular expression marker Platelet endothelial cell adhesion molecule (Pecam) and the glomerular expression marker podocin (Podn).

The B4 cell population is characterized by the expression of a vascular marker set containing one or more of the following: PECAM, VEGF, KDR, HIF1a, CD31, CD146; a glomerular marker set containing one or more of the following: Podocin (Podn), and Nephrin (Neph); and an oxygen-tunable EPO enriched population compared to unfractionated (UNFX), B2 and B3. B4 is also characterized by the expression of one or more of the following markers: chemokine (C-X-C motif) receptor 4 (Cxcr4), endothelin receptor type B (Ednrb), collagen, type V, alpha 2 (Col5a2), Cadherin 5 (Cdh5), plasminogen activator, tissue (Plat), angiopoietin 2 (Angpt2), kinase insert domain protein receptor (Kdr), secreted protein, acidic, cysteine-rich (osteonectin) (Sparc), serglycin (Srgn), TIMP metallopeptidase inhibitor 3 (Timp3), Wilms tumor 1 (Wt1), wingless-type MMTV integration site family, member 4 (Wnt4), regulator of G-protein signaling 4 (Rgs4), Platelet endothelial cell adhesion molecule (Pecam), and Erythropoietin (Epo). B4 is also characterized by smaller, less granulated cells compared to either B2 or B3, with a buoyant density between about 1.073 g/ml and about 1.091g/ml (rodent), between about 1.063 g/ml and about 1.091 g/mL (human and canine).

The B4' cell population is defined as having a buoyant density of between 1.063 g/mL and 1.091 g/mL and expressing one or more of the following markers: PECAM, vEGF, KDR, HIF1a, podocin, nephrin, EPO, CK7, CK8/18/19. In one embodiment, the B4' cell population is characterized by the expression of a vascular marker set containing one or more of the following: PECAM, vEGF, KDR, HIF1a, CD31, CD146. In another embodiment, the B4' cell population is characterized by the expression of an endocrine marker EPO. In one embodiment, the B4' cell population is characterized by the expression of a glomerular marker set containing one or more of the following: Podocin (Podn), and Nephrin (Neph). In certain embodiments, the B4' cell population is characterized by the expression of a vascular marker set containing one or more of the following: PECAM, vEGF, KDR, HIF1a and by the expression of an endocrine marker EPO. In another embodiment, B4' is also characterized by smaller, less granulated cells compared to either B2 or B3, with a buoyant density between about 1.073 g/ml and about 1.091g/ml (rodent), between about 1.063 g/ml and about 1.091 g/mL (human and canine).

In one aspect, the present invention provides methods of screening a test agent using an isolated, enriched B4' population of human renal cells comprising at least one of erythropoietin (EPO)-producing cells, vascular cells, and glomerular cells having a density between 1.063 g/mL and 1.091 g/mL. In one embodiment, the B4' cell population is characterized by expression of a vascular marker. In certain embodiments, the B4' cell population is not characterized by expression of a glomerular marker. In some embodiments, the B4' cell population is capable of oxygen-tunable erythropoietin (EPO) expression.

In one embodiment, the B4' cell population does not include a B2 cell population comprising tubular cells having a density between 1.045 g/mL and 1.052 g/mL. In another embodiment, the B4' cell population does not include a B1 cell population comprising large granular cells of the collecting duct and tubular system having a density of < 1.045 g/ml. In yet another embodiment, the B4' cell population does not include a B5 cell population comprising debris and small cells of low granularity and viability with a density > 1.091 g/ml.

In one embodiment, the B4' cell population does not include a B2 cell population comprising tubular cells having a density between 1.045 g/mL and 1.052 g/mL; a B1 cell population comprising large granular cells of the collecting duct and tubular system having a density of < 1.045 g/ml; and a B5 cell population comprising debris and small cells of low granularity and viability with a density > 1.091 g/ml. In some embodiments, the B4' cell population may be derived from a subject having kidney disease.

In one aspect, the present invention provides methods of screening a test agent using an admixture of human renal cells comprising a first cell population, B2, comprising an isolated, enriched population of tubular cells having a density between 1.045 g/mL and 1.052 g/mL, and a second cell population, B4', comprising erythropoietin (EPO)-producing cells and vascular cells but depleted of glomerular cells having a density between about 1.063 g/mL and 1.091 g/mL, wherein the admixture does not include a B1 cell population comprising large granular cells of the collecting duct and tubular system having a density of < 1.045 g/ml, or a B5 cell population comprising debris and small cells of low granularity and viability with a density > 1.091 g/ml. In certain embodiments, the B4' cell population is characterized by expression of a vascular marker. In one embodiment, the B4' cell population is not characterized by expression of a glomerular marker. In certain embodiments, B2 further comprises collecting duct epithelial cells. In one embodiment, the admixture of cells is capable of receptor-mediated albumin uptake. In another embodiment, the admixture of cells is capable of oxygen-tunable erythropoietin (EPO) expression. In one embodiment, the admixture contains HAS-2-expressing cells capable of producing and/or stimulating the production of high-molecular weight species of hyaluronic acid (HA) both *in vitro* and *in vivo.* In all embodiments, the first and second cell populations may be derived from kidney tissue or cultured kidney cells.

In another aspect, the present invention provides methods of screening a test agent using a heterogeneous renal cell population comprising a combination of cell fractions or enriched cell populations (e.g., B1, B2, B3, B4 (or B4'), and B5). In one embodiment, the combination has a buoyant density between about 1.045 g/ml and about 1.091 g/ml. In one other embodiment, the combination has a buoyant density between less than about 1.045 g/ml and about 1.099 g/ml or about 1.100 g/ml. In another embodiment, the combination has a buoyant density as determined by separation on a density gradient, e.g., by centrifugation. In yet another embodiment, the combination of cell fractions contains B2, B3, and B4 (or B4') depleted of B1 and/or B5. In some embodiments, the combination of cell fractions contains B2, B3, B4 (or B4'), and B5 but is depleted of B1. Once depleted of B1 and/or B5, the combination may be subsequently cultured *in vitro* prior to the preparation of a drug screening assay using the combination of B2, B3, and B4 (or B4') cell fractions.
The inventors of the present invention have surprisingly discovered that *in vitro* culturing of a B1-depleted combination of B2, B3, B4, and B5 results in depletion of B5. In one embodiment, B5 is depleted after at least one, two, three, four, or five passages. In one other embodiment, the B2, B3, B4, and B5 cell fraction combination that is passaged under the conditions described herein provides a passaged cell population having B5 at a percentage that is less than about 5%, less than about 4%, less than about 3%, less than about 2%, less than about 1%, or less than about 0.5% of the passaged cell population.

In another embodiment, B4' is part of the combination of cell fractions. In one other embodiment, the *in vitro* culturing depletion of B5 is under hypoxic conditions.

In one embodiment, the admixture is capable of providing a regenerative stimulus upon *in vivo* delivery. In other embodiments, the admixture is capable of reducing the decline of, stabilizing, or improving glomerular filtration, tubular resorption, urine production, and/or endocrine function upon *in vivo* delivery.

In one aspect, the present invention provides methods of screening a test agent using an isolated, enriched B4' population of human renal cells comprising at least one of erythropoietin (EPO)-producing cells, vascular cells, and glomerular cells having a density between 1.063 g/mL and 1.091 g/mL. In one embodiment, the B4' cell population is characterized by expression of a vascular marker. In certain embodiments, the B4' cell population is not characterized by expression of a glomerular marker. The glomerular marker that is not expressed may be podocin (see Example 7). In some embodiments, the B4' cell population is capable of oxygen-tunable erythropoietin (EPO) expression.

In one embodiment, the B4' cell population does not include a B2 cell population comprising tubular cells having a density between 1.045 g/mL and 1.052 g/mL. In another embodiment, the B4' cell population does not include a B1 cell population comprising large granular cells of the collecting duct and tubular system having a density of < 1.045 g/ml. In yet another embodiment, the B4' cell population does not include a B5 cell population comprising debris and small cells of low granularity and viability with a density > 1.091 g/ml.

In one embodiment, the B4' cell population does not include a B2 cell population comprising tubular cells having a density between 1.045 g/mL and 1.052 g/mL; a B1 cell population comprising large granular cells of the collecting duct and tubular system having a density of < 1.045 g/ml; and a B5 cell population comprising debris and small cells of low granularity and viability with a density > 1.091 g/ml.

In some embodiments, the B4' cell population may be derived from a subject having kidney disease. In one aspect, the present invention provides methods of screening a test agent using an admixture of human renal cells comprising a first cell population, B2, comprising an isolated, enriched population of tubular cells having a density between 1.045 g/mL and 1.052 g/mL, and a second cell population, B4', comprising erythropoietin (EPO)-producing cells and vascular cells but depleted of glomerular cells having a density between about 1.063 g/mL and 1.091 g/mL, wherein the admixture does not include a B1 cell population comprising large granular cells of the collecting duct and tubular system having a density of < 1.045 g/ml, or a B5 cell population comprising debris and small cells of low granularity and viability with a density > 1.091 g/ml. In certain embodiment, the B4' cell population is characterized by expression of a vascular marker. In one embodiment, the B4' cell population is not characterized by expression of a glomerular marker. In certain embodiments, B2 further comprises collecting duct epithelial cells. In one embodiment, the admixture of cells is capable of receptor-mediated albumin uptake. In another embodiment, the admixture of cells is capable of oxygen-tunable erythropoietin (EPO) expression. In one embodiment, the admixture contains HAS-2-expressing cells capable of producing and/or stimulating the production of high-molecular weight species of hyaluronic acid (HA) both *in vitro* and *in vivo.* In all embodiments, the first and second cell populations may be derived from kidney tissue or cultured kidney cells.

In one embodiment, the admixture is capable of providing a regenerative stimulus upon *in vivo* delivery. In other embodiments, the admixture is capable of reducing the decline of, stabilizing, or improving glomerular filtration, tubular resorption, urine production, and/or endocrine function upon *in vivo* delivery. In one embodiment, the B4' cell population is derived from a subject having kidney disease.

In a preferred embodiment, the admixture comprises B2 in combination with B3 and/or B4. In another preferred embodiment, the admixture comprises B2 in combination with B3 and/or B4'. In other preferred embodiments, the admixture consists of or consists essentially of (i) B2 in combination with B3 and/or B4; or (ii) B2 in combination with B3 and/or B4'.

The admixtures that contain a B4' cell population may contain B2 and/or B3 cell populations that are also obtained from a non-healthy subject. The non-healthy subject may be the same subject from which the B4' fraction was obtained. In contrast to the B4' cell population, the B2 and B3 cell populations obtained from non-healthy subjects are typically not deficient in one or more specific cell types as compared to a starting kidney cell population derived from a healthy individual.

As described in Presnell et al. WO/2010/056328, it has been found that the B2 and B4 cell preparations are capable of expressing higher molecular weight species of hyaluronic acid (HA) both *in vitro* and *in vivo,* through the actions of hyaluronic acid synthase-2 (HAS-2) - a marker that is enriched more specifically in the B2 cell population. Treatment with B2 in a 5/6 Nx model was shown to reduce fibrosis, concomitant with strong expression HAS-2 expression *in vivo* and the expected production of high-molecular-weight HA within the treated tissue. Notably, the 5/6 Nx model left untreated resulted in fibrosis with limited detection of HAS-2 and little production of high-molecular-weight HA. Without wishing to be bound by theory, it is hypothesized that this anti-inflammatory high-molecular weight species of HA produced predominantly by B2 (and to some degree by B4) acts synergystically with the cell preparations in the reduction of renal fibrosis and in the aid of renal regeneration. Accordingly, the methods of the instant invention include use of the cellular prototypes of the invention in a biomaterial comprising hyaluronic acid. Also comtemplated by the instant invention is the provision of a biomaterial component of the regenerative stimulus via direct production or stimulation of production by the implanted cells.

In another aspect, the present invention provides methods of screening a test agent using isolated populations of erythropoietin (EPO)-producing kidney cells that are further enriched such that the proportion of EPO-producing cells in the enriched subpopulation is greater relative to the proportion of EPO-producing cells in the starting or initial cell population. In one embodiment, the enriched EPO-producing cell fraction contains a greater proportion of interstitial fibroblasts and a lesser proportion of tubular cells relative to the interstitial fibroblasts and tubular cells contained in the unenriched initial population. In certain embodiments, the enriched EPO-producing cell fraction contains a greater proportion of glomerular cells and vascular cells and a lesser proportion of collecting duct cells relative to the glomerular cells, vascular cells and collecting duct cells contained in the unenriched initial population. In such embodiments, these populations are referred to herein as the "B4" cell population.

In another aspect, the present invention provides methods of screening a test agent using an EPO-producing kidney cell population that is admixed with one or more additional kidney cell populations. In one embodiment, the EPO-producing cell population is a first cell population enriched for EPO-producing cells, *e.g*., B4. In another embodiment, the EPO-producing cell population is a first cell population that is not enriched for EPO-producing cells, *e.g*., B2. In another embodiment, the first cell population is admixed with a second kidney cell population. In some embodiments, the second cell population is enriched for tubular cells, which may be demonstrated by the presence of a tubular cell phenotype. In another embodiment, the tubular cell phenotype may be indicated by the presence of one tubular cell marker. In another embodiment, the tubular cell phenotype may be indicated by the presence of one or more tubular cell markers. The tubular cell markers include, without limitation, megalin, cubilin, hyaluronic acid synthase 2 (HAS2), Vitamin D3 25-Hydroxylase (CYP2D25), N-cadherin (Ncad), E-cadherin (Ecad), Aquaporin-1 (Aqp1), Aquaporin-2 (Aqp2), RAB17, member RAS oncogene family (Rab17), GATA binding protein 3 (Gata3), FXYD domain-containing ion transport regulator 4 (Fxyd4), solute carrier family 9 (sodium/hydrogen exchanger), member 4 (Slc9a4), aldehyde dehydrogenase 3 family, member B1 (Aldh3b1), aldehyde dehydrogenase 1 family, member A3 (Aldh1a3), and Calpain-8 (Capn8). In another embodiment, the first cell population is admixed with at least one of several types of kidney cells including, without limitation, interstitium-derived cells, tubular cells, collecting duct-derived cells, glomerulus-derived cells, and/or cells derived from the blood or vasculature. The EPO-producing kidney cell population may contain B4 or B4' in the form of an admixture with B2 and/or B3, or in the form of an enriched cell population, e.g., B2+B3+B4/B4'.

In one aspect, the EPO-producing kidney cell populations used in the methods of the present invention are characterized by EPO expression and bioresponsiveness to oxygen, such that a reduction in the oxygen tension of the culture system results in an induction in the expression of EPO. In one embodiment, the EPO-producing cell populations are enriched for EPO-producing cells. In one embodiment, the EPO expression is induced when the cell population is cultured under conditions where the cells are subjected to a reduction in available oxygen levels in the culture system as compared to a cell population cultured at normal atmospheric (∼21%) levels of available oxygen. In one embodiment, EPO-producing cells cultured in lower oxygen conditions express greater levels of EPO relative to EPO-producing cells cultured at normal oxygen conditions. In general, the culturing of cells at reduced levels of available oxygen (also referred to as hypoxic culture conditions) means that the level of reduced oxygen is reduced relative to the culturing of cells at normal atmospheric levels of available oxygen (also referred to as normal or normoxic culture conditions). In one embodiment, hypoxic cell culture conditions include culturing cells at about less than 1% oxygen, about less than 2% oxygen, about less than 3% oxygen, about less than 4% oxygen, or about less than 5% oxygen. In another embodiment, normal or normoxic culture conditions include culturing cells at about 10% oxygen, about 12% oxygen, about 13% oxygen, about 14% oxygen, about 15% oxygen, about 16% oxygen, about 17% oxygen, about 18% oxygen, about 19% oxygen, about 20% oxygen, or about 21% oxygen.

In one other embodiment, the induction or increased expression of EPO is obtained and can be observed by culturing cells at about less than 5% available oxygen and comparing EPO expression levels to cells cultured at atmospheric (about 21%) oxygen. In another embodiment, the induction of EPO is obtained in a culture of cells capable of expressing EPO by a method that includes a first culture phase in which the culture of cells is cultivated at atmospheric oxygen (about 21%) for some period of time and a second culture phase in which the available oxygen levels are reduced and the same cells are cultured at about less than 5% available oxygen. In another embodiment, the EPO expression that is responsive to hypoxic conditions is regulated by HIF1α. Those of ordinary skill in the art will appreciate that other oxygen manipulation culture conditions known in the art may be used for the cells described herein.

In one aspect, the enriched populations of EPO-producing mammalian cells used in the methods of the invention are characterized by bio-responsiveness (e.g., EPO expression) to perfusion conditions. In one embodiment, the perfusion conditions include transient, intermittent, or continuous fluid flow (perfusion). In one embodiment, the EPO expression is mechanically-induced when the media in which the cells are cultured is intermittently or continuously circulated or agitated in such a manner that dynamic forces are transferred to the cells via the flow. In one embodiment, the cells subjected to the transient, intermittent, or continuous fluid flow are cultured in such a manner that they are present as three-dimensional structures in or on a material that provides framework and/or space for such three-dimensional structures to form. In one embodiment, the cells are cultured on porous beads and subjected to intermittent or continuous fluid flow by means of a rocking platform, orbiting platform, or spinner flask. In another embodiment, the cells are cultured on three-dimensional scaffolding and placed into a device whereby the scaffold is stationary and fluid flows directionally through or across the scaffolding. Those of ordinary skill in the art will appreciate that other perfusion culture conditions known in the art may be used for the cells described herein.

### Inactive cell populations

As described herein, the present invention is based, in part, on the surprising finding that certain subfractions of a heterogeneous population of renal cells, enriched for bioactive components and depleted of inactive or undesired components, provide superior therapeutic and regenerative outcomes than the starting population, and further provide drug screening platforms to more accurately predict *in vivo* or clinical toxicity and efficacy. In preferred embodiments, the cellular populations of the instant invention are depleted of B1 and/or B5 cell populations. For instance, the following may be depleted of B1 and/or B5: admixtures of two or more of B2, B3, and B4'; an enriched cell population of B2, B3, and B4'.

The B1 cell population comprises large, granular cells of the collecting duct and tubular system, with the cells of the population having a buoyant density less than about 1.045 g/m. The B5 cell population is comprised of debris and small cells of low granularity and viability and having a buoyant density greater than about 1.091 g/ml.

### Methods of isolating and culturing cell populations

In one aspect, the present invention provides methods of screening a test agent using cell populations that have been isolated and/or cultured from kidney tissue. In another aspect, the present invention provides organoids comprising and/or formed from cell populations that have been isolated and/or cultured from kidney tissue, as well as methods of forming and methods of using the organoids of the invention. Methods are provided herein for separating and isolating the renal cellular components, e.g., enriched cell populations that will be used in the screening assays. In one embodiment, the cell populations are isolated from freshly digested, i.e., mechanically or enzymatically digested, kidney tissue or from heterogeneous in vitro cultures of mammalian kidney cells.

In one aspect, the invention provides methods of screening a test agent using heterogeneous mixtures of renal cells that have been cultured in hypoxic culture conditions prior to separation on a density gradient, which provides for enhanced distribution and composition of cells in both B4, including B4', and B2 and/or B3 fractions. The enrichment of oxygen-dependent cells in B4 from B2 was observed for renal cells isolated from both diseased and non-diseased kidneys. Without wishing to be bound by theory, this may be due to one or more of the following phenomena: 1) selective survival, death, or proliferation of specific cellular components during the hypoxic culture period; 2) alterations in cell granularity and/or size in response to the hypoxic culture, thereby effecting alterations in buoyant density and subsequent localization during density gradient separation; and 3) alterations in cell gene / protein expression in response to the hypoxic culture period, thereby resulting in differential characteristics of the cells within any given fraction of the gradient. Thus, in one embodiment, the cell populations enriched for tubular cells, *e.g.*, B2, are hypoxia-resistant.

Exemplary techniques for separating and isolating the cell populations of the invention include separation on a density gradient based on the differential specific gravity of different cell types contained within the population of interest. The specific gravity of any given cell type can be influenced by the degree of granularity within the cells, the intracellular volume of water, and other factors. In one embodiment, optimal gradient conditions, as described herein, are used for isolation of the cell preparations of the instant invention, *e*.*g*., B2 and B4, including B4', across multiple species including, but not limited to, human, canine, and rodent. In a preferred embodiment, a density gradient is used to obtain a novel enriched population of tubular cells fraction, *i.e*., B2 cell population, derived from a heterogeneous population of renal cells. In one embodiment, a density gradient is used to obtain a novel enriched population of EPO-producing cells fraction, *i.e*., B4 cell population, derived from a heterogeneous population of renal cells. In other embodiments, a density gradient is used to obtain enriched subpopulations of tubular cells, glomerular cells, and endothelial cells of the kidney. In one embodiment, both the EPO-producing and the tubular cells are separated from the red blood cells and cellular debris. In one embodiment, the EPO-producing, glomerular, and vascular cells are separated from other cell types and from red blood cells and cellular debris, while a subpopulation of tubular cells and collecting duct cells are concomitantly separated from other cell types and from red blood cells and cellular debris. In one other embodiment, the endocrine, glomerular, and/or vascular cells are separated from other cell types and from red blood cells and cellular debris, while a subpopulation of tubular cells and collecting duct cells are concomitantly separated from other cell types and from red blood cells and cellular debris.

In one aspect, the methods of the instant invention use cell populations generated by using, in part, the OPTIPREP® (Axis-Shield) density gradient medium, comprising 60% nonionic iodinated compound iodixanol in water, based on certain key features described below. One of skill in the art, however, will recognize that any density gradient or other means, e.g., immunological separation using cell surface markers known in the art, comprising necessary features for isolating the cell populations of the instant invention may be used in accordance with the invention. It should also be recognized by one skilled in the art that the same cellular features that contribute to separation of cellular subpopulations via density gradients (size and granularity) can be exploited to separate cellular subpopulations via flow cytometry (forward scatter = a reflection of size via flow cytometry, and side scatter = a reflection of granularity). Importantly, the density gradient medium should have low toxicity towards the specific cells of interest. While the density gradient medium should have low toxicity toward the specific cells of interest, the instant invention contemplates the use of gradient mediums which play a role in the selection process of the cells of interest. Without wishing to be bound by theory, it appears that the cell populations of the instant invention recovered by the gradient comprising iodixanol are iodixanol-resistant, as there is an appreciable loss of cells between the loading and recovery steps, suggesting that exposure to iodixanol under the conditions of the gradient leads to elimination of certain cells. The cells appearing in the specific bands after the iodixanol gradient are resistant to any untoward effects of iodixanol and/or density gradient exposure. Accordingly, the present invention also contemplates the use of additional contrast medias which are mild to moderate nephrotoxins in the isolation and/or selection of the cell populations of the instant invention. In addition, the density gradient medium should also not bind to proteins in human plasma or adversely affect key functions of the cells of interest.

In another aspect, the methods of the instant invention use cell populations generated by enriching and/or depleting kidney cell types using fluorescent activated cell sorting (FACS). In one embodiment, kidney cell types may be enriched and/or depleted using BD FACSAria™ or equivalent.

In another aspect, the methods of the instant invention use cell populations generated by enriching and/or depleting kidney cell types using magnetic cell sorting. In one embodiment, kidney cell types may be enriched and/or depleted using the Miltenyi autoMACS® system or equivalent.

In another aspect, t the methods of the instant invention use cell populations generated by three-dimensional culturing of the renal cell populations. In one embodiment, the cell populations are cultured via continuous perfusion. In one embodiment, the cell populations cultured via three-dimensional culturing and continuous perfusion demonstrate greater cellularity and interconnectivity when compared to cell populations cultured statically. In another embodiment, the cell populations cultured via three dimensional culturing and continuous perfusion demonstrate greater expression of EPO, as well as enhanced expression of renal tubule-associate genes such as e-cadherin when compared to static cultures of such cell populations.

In yet another embodiment, the cell populations cultured via continuous perfusion demonstrate greater levels of glucose and glutamine consumption when compared to cell populations cultured statically.

As described herein (including Example 3), low or hypoxic oxygen conditions may be used in the methods to prepare the cell populations of the present invention. However, the methods of the present invention may be used without the step of low oxygen conditioning. In one embodiment, normoxic conditions may be used.

Those of ordinary skill in the art will appreciate that other methods of isolation and culturing known in the art may be used for the cells described herein.

### Biomaterials (Polymeric matrices or scaffolds)

As described in Bertram et al. U.S. Published Application 20070276507, polymeric matrices or scaffolds may be shaped into any number of desirable configurations to satisfy any number of overall system, geometry or space restrictions. In one embodiment, the matrices or scaffolds of the present invention may be three-dimensional and shaped to conform to the dimensions and shapes of an organ or tissue structure. For example, in the use of the polymeric scaffold for use in drug screening, a three-dimensional (3-D) matrix may be used. A variety of differently shaped 3-D scaffolds may be used. Naturally, the polymeric matrix may be shaped in different sizes and shapes to conform to differently sized patients. The polymeric matrix may also be shaped in other ways to accommodate the special needs of the patient. In another embodiment, the polymeric matrix or scaffold may be a biocompatible, porous polymeric scaffold. The scaffolds may be formed from a variety of synthetic or naturally-occurring materials including, but not limited to, open-cell polylactic acid (OPLA®), cellulose ether, cellulose, cellulosic ester, fluorinated polyethylene, phenolic, poly-4-methylpentene, polyacrylonitrile, polyamide, polyamideimide, polyacrylate, polybenzoxazole, polycarbonate, polycyanoarylether, polyester, polyestercarbonate, polyether, polyetheretherketone, polyetherimide, polyetherketone, polyethersulfone, polyethylene, polyfluoroolefin, polyimide, polyolefin, polyoxadiazole, polyphenylene oxide, polyphenylene sulfide, polypropylene, polystyrene, polysulfide, polysulfone, polytetrafluoroethylene, polythioether, polytriazole, polyurethane, polyvinyl, polyvinylidene fluoride, regenerated cellulose, silicone, ureaformaldehyde, collagens, laminins, fibronectin, silk, elastin, alginate, hyaluronic acid, agarose, or copolymers or physical blends thereof. Scaffolding configurations may range from liquid hydrogel suspensions to soft porous scaffolds to rigid, shape-holding porous scaffolds.

Hydrogels may be formed from a variety of polymeric materials and are useful in a variety of biomedical applications. Hydrogels can be described physically as three-dimensional networks of hydrophilic polymers. Depending on the type of hydrogel, they contain varying percentages of water, but altogether do not dissolve in water. Despite their high water content, hydrogels are capable of additionally binding great volumes of liquid due to the presence of hydrophilic residues. Hydrogels swell extensively without changing their gelatinous structure. The basic physical features of hydrogel can be specifically modified, according to the properties of the polymers used and the additional special equipments of the products.

Preferably, the hydrogel is made of a polymer, a biologically derived material, a synthetically derived material or combinations thereof, that is biologically inert and physiologically compatible with mammalian tissues. The hydrogel material preferably does not induce an inflammatory response. Examples of other materials which can be used to form a hydrogel include (a) modified alginates, (b) polysaccharides (e.g. gellan gum and carrageenans) which gel by exposure to monovalent cations, (c) polysaccharides (e.g., hyaluronic acid) that are very viscous liquids or are thixotropic and form a gel over time by the slow evolution of structure, and (d) polymeric hydrogel precursors (e.g., polyethylene oxide-polypropylene glycol block copolymers and proteins). U.S. Pat. No. 6,224,893 B1 provides a detailed description of the various polymers, and the chemical properties of such polymers, that are suitable for making hydrogels in accordance with the present invention.

Scaffolding or biomaterial characteristics may enable cells to attach and interact with the scaffolding or biomaterial material, and/or may provide porous spaces into which cells can be entrapped. In one embodiment, the porous scaffolds or biomaterials of the present invention allow for the addition or deposition of one or more populations or admixtures of cells on a biomaterial configured as a porous scaffold (e.g., by attachment of the cells) and/or within the pores of the scaffold (e.g., by entrapment of the cells). In another embodiment, the scaffolds or biomaterials allow or promote for cell:cell and/or cell:biomaterial interactions within the scaffold to form constructs as described herein.

In one embodiment, the biomaterial used in accordance with the present invention is comprised of hyaluronic acid (HA) in hydrogel form, containing HA molecules ranging in size from 5.1 kDA to >2 x 10⁶ kDa. In another embodiment, the biomaterial used in accordance with the present invention is comprised of hyaluronic acid in porous foam form, also containing HA molecules ranging in size from 5.1 kDA to >2 x 10⁶ kDa. In yet another embodiment, the biomaterial used in accordance with the present invention is comprised of of a poly-lactic acid (PLA)-based foam, having an open-cell structure and pore size of about 50 microns to about 300 microns. In yet another embodiment, the specific cell populations, preferentially B2 but also B4, provide directly and/or stimulate synthesis of high molecular weight Hyaluronic Acid through Hyaluronic Acid Synthase-2 (HAS-2), especially after intra-renal implantation.

Those of ordinary skill in the art will appreciate that other types of synthetic or naturally-occurring materials known in the art may be used to form scaffolds as described herein.

In one aspect, the present invention provides constructs as described herein made from the above-referenced scaffolds or biomaterials.

### Screening Methods

The cells of the invention are useful for studying several parameters of a test agent, including metabolism, toxicity and efficacy. Methods of the invention can be used to screen experimental drugs or "test agents" that have no known metabolic or pharmacokinetic profile, in order to obtain such information, including information necessary to assess toxicity. Toxicity can often occur as a result of drug-to-drug interactions. Thus, methods of the invention can be used to study the combination of test agents with known drugs or other test agents.

Many drugs and xenobiotics (e.g. antibiotics, p-aminohippurate) cannot be cleared efficiently in the kidney by glomerular filtration. Such substances are then actively transported by the cells of the proximal tubule from the bloodstream into the glomerular filtrate flowing in the lumen of the renal tubule. Organic anion transporters, organic cation transporters and the p-glycoprotein coded by the multi-drug resistance locus play a major role in such transcellular transport processes. Due to their functions in drug transport, proximal tubule cells are a major target for toxic drug effects in the kidney; as well, other cell types of the renal tubule can be affected by drugs in the filtrate. Thus, it is important to assess the nephrotoxic effects of newly developed drugs and their effects on the cells of the renal tubule.

Thus, in one aspect, the present invention provides methods for the screening and the pharmacological profiling of test agents or compounds, *e*.*g*., new chemical entities (NCEs), modulating a cellular or organoid response, e.g. a physiological response and/or the activities of cells or organoids. The present invention further provides methods for determining a level of renal toxicity or nephrotoxicity of one or more test agents using the cell populations of the invention. In another aspect, the present invention provides methods for assessing changes in the phenotype of the cultured cell populations of the invention in the presence of a test agent.

### A. Functional Assays

Functional assays can be used to determine the cell health and viability of the cell populations of the invention in the presence of a test compound. In certain embodiments, the indicators of cell health and viability include but are not limited to, indicators of cellular replication, mitochondrial function, energy balance, membrane integrity and cell mortality. In other embodiments, the indicators of cell health and viability further include indicators of oxidative stress, metabolic activation, metabolic stability, enzyme induction, enzyme inhibition, and interaction with cell membrane transporters.

### 1. Cell-based Proliferation Assays

Cell-based proliferation assays using the cell populations of the present invention can be used to determine the effect on cell proliferative activity of a test agent. In general, cell proliferation and cell viability assays are designed to provide a detectable signal when cells are metabolically active.

A number of proliferation assays are based on the incorporation of labeled nucleotide or nucleotide analogs into the DNA of proliferating cells. In these assays, cells are exposed to a candidate compound and to a labeled nucleotide, e.g., 14C-thymidine, 3H-thymidine, or 5-bromo-2-deoxyuridine (BrdU). Proliferation is quantified by measuring the amount of labeled nucleotide taken up by the cells. Radiolabeled nucleotides can be measured by radiodetection methods; antibodies can be used to detect incorporation of BrdU. Other assays rely on the conversion of chemical precursors to a dye in dividing cells. Some assays measure the conversions of tetrazolium salts (e.g., methyl thiazole tetrazolium (MTT), 2-(4-Iodophenyl)-3-(4-nitrophenyl)-5-(2,4-disulfophenyl)-2H-tetraz- olium (WST-1), or 3'-{1-[(phenylamino)-carbonyl]-3,4-tetrazolium}-bis(4-methoxy-6-nitro) benzene-sulfonic acid hydrate (XTT)) to formazan by cellular mitochondrial dehydrogenases. Mitochondrial dehydrogenase activity increases in proliferating cells, thereby increasing the amount of formazan dye. The amount of formazan dye measured by absorbance is an indication of proliferation. Still other assays measure cellular proliferation as a function of ATP production. For example, the luciferase enzyme catalyzes a bioluminescent reaction using the substrate luciferin. The amount of bioluminescence produced by a sample of cells measures the amount of ATP present in the sample, which is an indicator of the number of cells. Some cell proliferation assays directly measure the number of cells produced by a number of founder cells in the presence of a candidate compound, *e*.*g*., soft-agar colony formation assays. Furthermore, commercially available kits, including reagents and protocols, are available for example, from Promega Corporation (Madison, Wis.), Sigma-Aldrich (St. Louis, Mo.), and Trevigen (Gaithersburg, Md.).

### 2. Cell Migration Assays

When a cell is exposed to chemical stimuli, its behavior is an important consideration, particularly when developing and evaluating therapeutic candidates and their effectiveness. By documenting the reaction of a cell or a group of cells to a chemical stimulus, such as a therapeutic test agent, the effectiveness of the chemical stimulus can be better understood.

Scratch wound assays or similar alternatives are commonly used to assess the effects of drugs and drug candidates on cellular migration associated with wound closing. In a typical scratch wound assay, cells are plated to confluence in a multiwell plate. A single scratch wound is created in each well. The plate is then imaged at fixed time intervals. Cell number in the area of the scratch wound can be quantified to evaluate the characteristics of wound closing in the presence of pharmacological agents. Commercially available kits, including reagents and protocols, are available, for example, from Essen Bioscience (Ann Arbor, Michigan).

### 3. GGT assay

γ-Glutamyl transpeptidase (GGT1), a tubular cell marker, has been shown to play a key role in the gamma-glutamyl cycle, a pathway for the synthesis and degradation of glutathione (GSH) and drug and xenobiotic detoxification. Siest G, et al., (1992) Biochem. Pharmacol. 43 (12): 2527-2533. The typical GGT assay measures nitroaniline production by GGT1 and is useful to determine the functional characterization of the cell populations of the invention in the presence of a test compound. Functional assays using GGT1 are well known in the art, for example, Kelley et al., Am J Physiol Renal Physiol. 2010 Nov;299(5):F 1026-39.

### 4. Albumin uptake assay

Measurement of albumin uptake can be used to determine the functional characterization of the cells of the invention in the presence of a test compound. Albumin uptake assays have been described in the literature, for example, Kelley et al., Am J Physiol Renal Physiol. 2010 Nov;299(5):F1026-39, and in Example 10 below.

### 5. LAP assay

In other embodiments, detection of biomarkers as diagnostic of kidney injury, include detection of leucine aminopeptidase (LAP). LAP, a tubular enzyme, is released from damaged proximal and/or distal tubular cells.

### B. Determining Nephrotoxicity of a Test Agent

In one embodiment, the present invention provides a method of determining the nephrotoxicity of a test agent or compound, comprising contacting a heterogeneous renal cell population comprising a B2 cell population, wherein the B2 cell population comprises an enriched population of tubular cells, with the test compound, and determining the level of an indicator of nephrotoxicity, wherein the level of the indicator is indicative of nephrotoxicity.

In certain embodiments, the cell population comprises a B2 cell population, wherein the B2 cell population comprises an enriched population of tubular cells, and is depleted of a B1 cell population, and/or a B5 cell population.

Compounds that alter mitochondrial function or cellular energy balance will ultimately produce cell death. Therefore it is important to monitor the effects of the test compounds on mitochondrial function and energy balance. The reduction of tetrazolium dyes such as 3-[4,5-dimethylthiazol-2-yl]-2,5-diphenyltetrazolium bromide (MTT) (Mosmann, 1983; Huveneers-Oorsprong, et al., 1997) and Alamar blue (A B) (Goegan et al., 1995) to chromaphores detectable by spectrophotometric or fluorometric analysis have been used extensively as indicators of cell viability. Accordingly, in one embodiment, the level of MTT is measured in determining the level of renal toxicity.

In other embodiments of the present invention, the indicator of *in vitro* nephrotoxicity is a measure of cell death. The nephrotoxic effects of test compounds may occur via several possible mechanisms, including inhibition of protein synthesis, mitochondrial injury, and DNA damage. These cellular insults ultimately lead to activation of programmed cell death pathways and apoptosis. Thus, indicators of *in vitro* nephrotoxicity suitable for use with the present invention are indicators of apoptosis. Indicators of apoptosis can be characterized by distinct morphologic changes consisting of cell shrinkage, nuclear condensation, and internucleosomal DNA fragmentation.

Preferably, in one embodiment, the *in vitro* indicator of nephrotoxicity is a biochemical indicator of apoptosis. For example, biochemical indicators of apoptosis may monitor caspase 8 activity, which is induced predominantly from apoptotic stimuli received via integral membrane death receptors such as Fas and TNFR1.

In another embodiment, biochemical indicators of apoptosis initiated from mitochondria may be assayed, such as caspase-9 activity or cytochrome C release. Alternatively, rather than monitoring apoptosis from a single pathway, a particular embodiment monitors the common effector of different apoptosis pathways, for example, caspase-3 activity. It has been established in the art that once activated, both caspases 8 and 9 participate in a cascade that culminates in the activation of caspase 3, which cleaves several substrates, resulting in chromosomal DNA fragmentation and cellular morphologic changes characteristic of apoptosis. Thus, it would be understood by one ordinarily skilled in the art that when the *in vitro* indicator of nephrotoxicity monitors caspase-3 activity, effectively all apoptotic pathway are being monitored.

In one embodiment, the indicator of *in vitro* nephrotoxicity is a morphological or biochemical indicator of apoptosis. In a particular embodiment, the indicator of in vitro nephrotoxicity is a biochemical marker of apoptosis such as cytochrome C oxidase activity, and caspase activity.

In a more particular embodiment, the caspase activity for use as an *in vitro* indicator of nephrotoxicity is selected from the group consisting of: caspase-9 activity, caspase-8 activity, caspase-7 activity, and caspase-3 activity. In another embodiment, the activities of one or more caspases selected from group consisting of caspase-9 activity, caspase-8 activity, caspase-7 activity, and caspase-3 activity are used as indicators of in vitro nephrotoxicity. In yet a more particular embodiment, the *in vitro* indicator of nephrotoxicity is caspase-3 activity.

In related embodiments, caspase activity is determined by using a conditionally activated luciferase substrate, wherein caspase cleavage of the luciferase substrate makes the substrate available to luciferase. In a particular embodiment, the luciferase substrate is specific for measuring caspase-3 activity. Such assays are provided by Promega under the name CASPASEGLO, which is a commercially available assay kit for monitoring caspase activity based upon luminescence.

The skilled artisan would appreciate that many other enzymes activated in apoptosis, and which are known in the art are equally suited for use as an in vitro indicator of nephrotoxicity in the in vitro nephrotoxicity assays described herein.

Receptor for Advanced Glycation End Products (RAGE) can also be used in determining the toxicity of a test agent. Assays for determining RAGE are commercially available, *e.g*., RAGE Human ELISA Kit - 1 x 96 Well Plate (ab100632) from AbCAM (San Francisco, CA) and QUANTIKINE® Human RAGE Immunoassay from R&D Systems (Minneapolis, MN).

Biotransformation of drugs by multiple renal enzyme systems, including CYP450, results in the formation of toxic metabolites and reactive oxygen species (ROS), thus resulting in oxidative stress and a consequential increase in renal injury via nucleic acid alkylation or oxidation, protein damage, lipid peroxidation, and DNA strand breaks Cummings BS, Schnellmann RG: Pathophysiology of nephrotoxic cell injury. In Diseases of the Kidney and Urogenital Tract, edited by Schrier RW, Philadelphia PA, Lippincott Williams & Wilkinson, 2001, pp 1071-1136; Kaloyanides GJ, Bosmans J-L, DeBroe ME: Antibiotic and Immunosuppression-related renal failure. In Schrier RW (ed): Diseases of the Kidney and Urogenital Tract, edited by Schrier RW, Philadelphia PA, Lippincott Williams & Wilkinson, 2001, pp 1137-1174; and Aleksa K, et al., Pediatr Nephrol 20: 872- 885, 2005. Tissue damage due to production of reactive oxygen species (ROS) occurs when highly reactive chemical species with unpaired electrons are generated both endogenously and by metabolism of parent chemicals. The most biologically significant free radical species are superoxide free radical anion (O'₂.⁻), hydroxyl radical (OH), and hydrogen peroxide (H₂O₂). The cellular targets of these ROS are proteins, phospholipids (produces highly reactive aldehyde molecules), and DNA. The result of these interactions is membrane damage, enzyme malfunction, and hydroxylation of DNA, which can lead to mutagenesis. Oxidative stress can also occur when a chemical is either a direct electrophile or is metabolized to an electrophilic entity. Electrophiles can produce oxidative damage indirectly by depleting cellular antioxidants such as glutathione (GSH) and vitamin E. Once depleted the cell would be considerably more susceptible to oxidant damage from endogenously produced ROS. The production of reactive oxygen species (ROS) can be measured using 2'-7'dichloro-dihydrofluorescein diacetate (DCFDA). Fluorescence signal is directly proportional to production of ROS (Fabiani et al., 2005; Mracek et al., 2006; Gonzalez et al., 2006). Reactive oxygen species (ROS) assays are commercially available, for example, by Cell Biolabs, Inc. (San Diego, CA).

Assays to determine DNA integrity or ploidy are well known in the art. For example, the *in vitro* micronucleus assay is a mutagenic test system for the detection of compounds which induce compounds that produce chromosomal aberrations, including breakage (clastogens) or abnormal copy number (aneugens). The *in vitro* micronucleus assay has been described in detail previously (M. Fenech, Mutation Res (2000) 455(1-2):81-95) and is commercially available, for example, by BD (Franklin Lakes, NJ).

General methods and assays for determining DNA damage by cell cycle or karytyping are well known in the art and can easily be adapted by one of skill in the art using the cell populations of the invention. te Poele et al.,Cancer Res. 2002 Mar 15;62(6):1876-83; Gilbert and Hemann, Cell. 2010 Oct 29;143(3):355-66; Lopez de Mesa et al.Cancer Genet Cytogenet. 2000 Aug;121(1):78-85; Ribas et al., FASEB J. 2003 Feb;17(2):289-91; Jones and Ravid, J Biol Chem. 2004 Feb 13;279(7):5306-13; and Davoli et al., Cell. 2010 Apr 2;141(1):81-93.

Leakage of N-acetylglucosaminidase (NAG), a lysosomal enzyme involved in the breakdown metabolism of glycoproteins, may also be employed as an indicator of nephrotoxicity. Assays measuring NAG leakage are known in the art and are commercially available, *e*.*g*., N-acetyl-β-D-glucosaminidase (NAG) Colorimetric Assay Kit from Bio-Quant Inc.

Assays to detect steatosis, abnormal lipid accumulation, as an indicator of toxicity are also known in the art and are commercially available, *e*.*g*., Cayman Chemical Steatosis Colorimetric Assay Kit #10012643.

A variety of different molecules may be employed as indicators of nephrotoxicity in the in vitro assays of the present invention. In particular embodiments, an indicator of nephrotoxicity is a measure of cell injury. In related embodiments damage to kidney cells is monitored by the release of brush border enzymes. In other embodiments, changes in kidney cell gene expression in genes such as osteopontin, inositol polyphospate multikinase, I-arginine glycine amidinotransferase, prosaposin, lipocalin, synaptogyrin 2, kallikrein, KIM-1, kidney injury molecule 1 (Kim1), lipocalin 2 (Lcn2) can be used to indicate nephrotoxicity of a compound. Examples of other genes that whose expression levels can be measure to indicate nephrotoxicity of a compound are described in Amin et al., Environ Health Perspect. 2004 March; 112(4):465-79 and Wang et al., Toxicology. 2008 Apr 18;246(2-3):91-100. One having ordinary skill in the art would appreciate that a variety of methods can be used to measure changes in gene expression, for example, DNA slot blot, RT-PCR, real-time PCR, oligonucleotide and cDNA microarrays, primer extension, S1 nuclease assay, and RNAse protection assays, among others.

In another embodiment, an indicator of nephrotoxicity is clusterin, also known as testosterone-repressed prostate message 2 (TRPM-2), which is an ubiquitous, secreted glycoprotein induced in many organs, including the kidney, at times of tissue injury and/or remodeling, and it has been found in the tubular lumen of epithelial ducts. Jenne D E & Tschopp J, Trends Biochem. Sci. 14: 154-159 (1992). Clusterin may preserve cell interactions that are otherwise perturbed by renal insults. Silkensen J R et al., J. Am. Soc. Nephrol. 8(2): 302-305 (1997). Furthermore, cyclosporine A (CsA) increases clusterin mRNA levels in the rat kidney. Darby I A et al., Exp. Nephrol. 3(4): 234-239 (1995).

In another embodiment, an indicator of nephrotoxicity is alpha-2u globulin related-protein (Alpha-2u), also known as lipocalin 2 (LCN2) or neutrophil gelatinase-associated lipocalin (NGAL) in humans. NGAL is stored in granules of neutrophils and binds to small lipophilic substances and is believed to play a role in inflammation. Bundgaard J R et al., Biochem. Biophys. Res. Commun. 202: 1468-1475 (1994); Cowland J B & Borregaard N, Genomics 45: 17-23 (1997); Zerega B et al., Eur. J. Cell Biol. 79, 165-172 (2000).

Measurements of α-GST leakage from cultured cells into the media can be used to assess membrane integrity. This assay is specific for the alpha form of GST found in the proximal tubule. ELISA kits to measure GST are available commercially, *e*.*g*., from Biotrin Inc.. GST leakage assays, have been described in the literature, for example, Redick et al., J Biol. Chem. 257, 15200-15203. Oberley et al., Toxicol. Appl. Pharmacol. 131, 94-107, 1995; Feinfeld, J Clin Chem Clin Biochem. 24, 529-532, 1986. Other assays for determining membrane integrity include, but are not limited to, assays that determine lactate dehydrogenase (LDH) activity, aspartyl aminotransferase, alanine aminotransferase, isocitrate dehydrogenase, sorbitol dehydrogenase, glutamate dehydrogenase, ornithine carbamyl transferase, γ-glutamyl transferase, and alkaline phosphatase.

DNA integrity (ploidy) may also be useful in determining the toxicity of a test agent. Assays to determine DNA integrity are well known in the art and are commercially available, *e*.*g*., GUAVA® Cell Cycle Assay from Millipore (Billerica, Ma).

In yet another embodiment, an indicator of nephrotoxicity is collagen IV. The collagen IV assay is well known to those of skill in the art and has been described in, for example, Nonyl et al., AJPRP 281 :F443-F435, 2001.

In another embodiment, an indicator of nephrotoxicity is OCT4. The OCT4 assay is well known to those of skill in the art and has been described in, for example, Fuchs and Hewitt, Toxicol Pathol. 2010;38(6):943-56 and Gautier et al., Toxicol Pathol. 2010;38(6):943-56.

In still another embodiment, an indicator of nephrotoxicity is RPA. Assays for RPA are well known in the art and have been described in, for example, Zhang et al., Toxicologic Pathology, 37: 629-643, 2009.

In another embodiment, an indicator of nephrotoxicity is tissue inhibitor of matrix metalloproteinase-1 (Timp-1). The Timp-1 assay is well known to those of skill in the art and has been described in, for example, Toxicological Sciences 103(2), 371-381 (2008) Evaluation of Putative Biomarkers for Nephrotoxicity after exposure to Ochratoxin A In-Vivo and In-Vitro.

In various embodiments, the level of an *in vitro* indicator of nephrotoxicity is measured continuously. The levels of *in vitro* indicators of nephrotoxicity can be monitored at the time the compound or mixture of compounds are added to a culture of cells of the present invention. Monitoring can be done periodically, for example, at about 10 minutes, about 30 minutes, about 1 hour, about 2 hours, about 5 hours, about 12 hours, about 18 hours, about 24 hours, about 30 hours, about 36 hours, about 48 hours, about 56 hours, about 64 hours, about 72 hours or more after the compound or mixture of compounds are added to a culture of cells of the present invention. Monitoring can also be performed at a time between 0 hours and about 96 hours, 0 hours and about 84 hours, 0 hours and about 72 hours, 0 hours and about 60 hours, 0 hours and about 48 hours, 0 hours and about 36 hours, 0 hours and about 30 hours, or 0 hours and about 24 hours.

In particular embodiments, methods of the present invention comprise determining a dose response curve and/or an TC50 of a test compound or compound mixture. As used herein, the term "dose response curve" describes a relationship between the amount of a compound or mixture assayed and the resulting measured response. The term "dose" is commonly used to indicate the amount of the compound or mixture used in the experiment, while the term "response" refers to the measurable effect of the compound or compound mixture being tested. Dose-response relationships are determined graphically by plotting the varying compound or mixture concentration on the X-axis in log scale and the measurable response on the Y-axis. As used herein, the term "TC50" means the concentration of a compound or mixture of compounds that induces a response halfway between the baseline response and the maximum response of that compound or compound mixture.

In related embodiments, the nephrotoxicity of a test compound is determined by comparing the TC50 determined for a test compound by an in vitro assay of the present invention to the TC50 of one or more known nephrotoxic compounds, as determined using an in vitro assay of the present invention.

In other related embodiments, the nephrotoxicity of a test compound mixture is determined by comparing the TC50 determined for a test compound mixture by an in vitro assay of the present invention to the TC50 of one or more known nephrotoxic compounds or compound mixtures, as determined using an in vitro assay of the present invention. Generally, a dose-response curve for the test compound is determined by measuring the level of an indicator of nephrotoxicity produced using various concentrations of a test compound, such as a set of serial dilutions of the test compound. The goal of determining the nephrotoxicity of a test compound over a serially diluted concentration range is to provide for the construction of a dose response curve. The X-axis of a dose response curve generally represents the concentration of the test compound on a log scale, whereas the Y-axis represents the response of the in vitro indicator of nephrotoxicity to a particular concentration of test compound.

Those skilled in the art would understand that a standard dose-response curve is generally defined by four parameters: the baseline response, wherein the indicator of nephrotoxicity does not increase above the lowest concentration of test compound tested; the maximum response, wherein there is no additional increase in the in vitro indicator of nephrotoxicity with increasing concentrations of test compound; the slope of the curve, wherein changes in the in vitro indicator of nephrotoxicity increase with increasing test compound concentrations; and the TC50, wherein the concentration of test compound produces a half-maximal response in the in vitro indicator of nephrotoxicity for that given compound. More simply stated, the TC50 is the concentration of test compound that provokes a response half-way between the baseline response and maximum response.

Thus, the TC50 is a convenient measure of the inherent nephrotoxicity of a test compound. In addition, it would be understood by the skilled artisan that the relative nephrotoxicity of a test compound to a control nephrotoxic compound may be determined by comparing the TC50s of the test compound to the control nephrotoxic compound. Thus, a test compound is said to have a relatively high level of nephrotoxicity when the TC50 of the test compound is less than the TC50 of a control nephrotoxic compound. Likewise, the test compound is said to have a relatively low level of nephrotoxicity when the TC50 of the test compound is greater than the TC50 of a control nephrotoxic compound. The skilled artisan would understand that the methods of the present invention are able to determine all degrees of relative nephrotoxicity of a test compound to any given control nephrotoxic compound and that such methods are not limited to the examples herein.

In other embodiments, methods of the present invention comprise determining an IC50 of a test agent. The term "IC50" as used herein, is intended to refer to the dose of a biologically active moiety, *e*.*g*., test agent, that inhibits a biological activity by 50%.

The present methods provide for testing the nephrotoxicity of a test compound. It would be understood by one of ordinary skill in the art that a test compound may be any pharmaceutical compound including small molecules and peptides that cause renal damage upon administration to a host. Such drugs include, by way of example, diuretics, NSAIDs, ACE inhibitors, cyclosporin, tacrolimus, radiocontrast media, interleukin-2, vasodilators (hydralazine, calcium-channel blockers, minoxidil, diazoxide), mitomycin C, conjugated estrogens, quinine, 5-fluorouracil, ticlopidine, clopidogrel, interferon, valacyclovir, gemcitabine, bleomycin, heparin, warfarin, streptokinase, nedaplatin, methoxyflurane, tetracycline, amphotericin B, cephaloridine, streptozocin, tacrolimus, carbamazepine, mithramycin, quinolones, foscamet, pentamidine, intravenous gammaglobulin, fosfamide, zoledronate, cidofovir, adefovir, tenofovir, mannitol, dextran, hydroxyethylstarch, lovastatin, ethanol, codeine, barbiturates, diazepam, quinine, quinidine, sulfonamides, hydralazine, triamterene, nitrofurantoin, mephenyloin, penicillin, methicillin ampicillin, rifampin, sulfonamides, thiazides, cimetidine, phenyloin, allopurinol, cephalosporins, cytosine arabinoside, furosemide, interferon, ciprofloxacin, clarithromycin, telithromycin, rofecoxib, pantoprazole, omeprazole, atazanavir, gold, penicillamine, captopril, lithium, mefenamate, fenoprofen, mercury, interferon, pamidronate, fenclofenac, tolmetin, foscamet, aciclovir, methotrexate, sulfanilamide, triamterene, indinavir, foscamet, ganciclovir, methysergide, ergotamine, dihydroergotamine, methyldopa, pindolol, hydralazine, atenolol, taxol, tumor necrosis factor, chlorambucil, interleukins, bleomycin, etoposide, fluorouracil, vinblastine, doxorubicin, cisplatin, aminoglycosides, and the like (see, generally, Devasmita et al., Nature Clinical Practice Nephrology (2006) 2, 80-91).

The compounds to be tested may include fragments or parts of naturally-occurring compounds or may be derived from previously known compounds through a rational drug design scheme. It is proposed that compounds isolated from natural sources, such as animals, bacteria, fungi, plant sources, including leaves and bark, and marine samples may be assayed as candidates for the presence of potentially useful pharmaceutical compounds. Alternatively, pharmaceutical compounds to be screened for toxicity could also be synthesized (i.e., man-made compounds).

### C. Pharmacokinetics

"Pharmacokinetics" refers to the actions of the body on a drug. Pharmacokinetic processes include, but are not limited to, absorption, distribution, metabolism, and elimination of drugs. Uptake transporter assays are useful in determining the pharmacokinetic profile of a test agent. Exemplary transporter uptake assays include determination of test agent interactions with the following transporters using the cell populations of the invention.

Organic Anion Transporter 1 (OAT1), a transmembrane protein expressed predominantly in the basolateral membrane of proximal tubular cells, is involved in the uptake of a wide range of relatively small and hydrophilic organic anions from plasma into the cytoplasm of the proximal tubular cells of the kidneys for subsequent exit across the apical membrane for excretion via the urine. El-Sheikh AAK et al., (2008) Eur J Pharmacol 585; 245-255. The OAT1 assay is well known in the art and has been described in, for example, J. Cell. Mol. Vol 15, No 6, 2011 pp.1287-1298.

Organic Cation Transporter 1 (OCT1), located on the basolateral membrane of renal proximal tubular cells, mediates facilitated transport of small (hydrophilic) organic cations. Jonker JW and Schinkel AH (2004) J Pharmacol Exp Ther 308(1); 2-9; Jonker JW et al, (2001) Mol Cell Biol 21(16); 5471-5477. The *in vitro* transporter OCT1 assay is well known in the art and has been described in, for example, Expert Opin Drug Metab. Toxicol. (2005) 1(3):409-427.

Multidrug resistance-associated protein (MRP) belongs to the ATP-binding cassette (ABC) transporter superfamily, and plays a role in detoxification in tissues including kidney. Inui et al. Kidney Int. 2000 Sep;58(3):944-58. The MRP assay is well known in the art and is commercially available, for example, ATP Bioluminescence Assay to Quantify Cell Toxicity BMG Microplate Luminometer LUMIstarlabtech from BMG Labtech (Cary, NC) and CELLTITER-GLO® assay (Promega Madison WI), and also as described by W. Li et al. (Toxicology in Vitro 20 (2006) 669-676).

Further transporter uptake assays, using the cell populations of the invention, include determination of test agent interactions with the following transporters: Protein kinase C (PKC), LAP, GGT, and MDR1 (J. Sahi, Expert Opin Drug Metab. Toxicol. (2005) 1(3):409-427).

### D. Cell culture and assay format

The foregoing methods require the use of the cell populations described herein. Techniques employed in mammalian primary cell culture and cell line cultures are well known to those of skill in that art.

In one embodiment, a level of renal toxicity of an agent is determined by culturing a plurality of concentrations of a test agent with a heterogeneous renal cell population comprising a B2 cell population, wherein the B2 cell population comprises an enriched population of tubular cells. In another embodiment, the heterogenous renal cell population further comprises a B4 cell population. In yet another embodiment, the heterogeneous renal cell population further comprises a B3 population. In still another embodiment, the heterogeneous renal cell population further comprises a B5 population.

In certain embodiments, the cell population comprises a B2 cell population, wherein the B2 cell population comprises an enriched population of tubular cells, and is depleted of a B1 cell population, and/or a B5 cell population.

The effect of various test compounds on the cell populations of the invention may be determined either in two-dimensional suspension culture or in a three-dimensional system as described herein. As used herein, a two dimensional surface (or 2D surface) refers to a surface that is not embedded within a gel matrix (or 3D gel). In particular embodiments, the cells are seeded in multiwell (e.g., 96-well), extracellular matrix (ECM)-coated plates and cultured in medium. The cell supernatant or the cells themselves may be harvested for analysis.

In certain embodiments, the cells of the invention may be cultured in 3D formats as described further herein. In some embodiments, the term "organoid" refers to an accumulation of cells, with a phenotype and/or function, consistent with a native kidney. In some embodiments, organoids comprise mixed populations of cells, of a variety of lineages, which are typically found *in vivo* in a given tissue. In some embodiments, the organoids of this invention are formed *in vitro,* via any means, whereby the cells of the invention form aggregates, which in turn may form spheroids, organoids, or a combination thereof. Such aggregates, spheroids or organoids, in some embodiments, assume a structure consistent with a particular organ. In some embodiments, such aggregates, spheroids or organoids, express surface markers, which are typically expressed by cells of the particular organ. In some embodiments, such aggregates, spheroids or organoids, produce compounds or materials, which are typically expressed by cells of the particular organ. For example, multicellular spheroids, the most commonly used 3D cell culture system, have been previously proposed as a drug-screening tool (Schughart et al., J.Biomol.Screen.9, 273-285 (2004)). In certain embodiments, the cells of the invention may be cultured on natural substrates, *e*.*g*., gelatin. In other embodiments, the cells of the invention may be cultured on synthetic substrates, *e*.*g*., PGLA.

Once the cell cultures are thus established, various concentrations of the compound being tested are added to the media and the cells are allowed to grow exposed to the various concentrations for 24 hours. While the 24 hour exposure period is described, it should be noted that this is merely an exemplary time of exposure and testing the specific compounds for longer or shorter periods of time is contemplated to be within the scope of the invention. As such it is contemplated that the cells may be exposed for 6, 12, 24, 36, 48 or more hours.

Furthermore, the cells may be exposed to the test compound at any given phase in the growth cycle. For example, in some embodiments, it may be desirable to contact the cells with the compound at the same time as a new cell culture is initiated. Alternatively, it may be desirable to add the compound when the cells have reached confluent growth or arc in log growth phase. Determining the particular growth phase cells are in is achieved through methods well known to those of skill in the art.

The varying concentrations of the given test compound are selected with the goal of including some concentrations at which no toxic effect is observed and also at least two or more higher concentrations at which a toxic effect is observed. A further consideration is to run the assays at concentrations of a compound that can be achieved in vivo. For example, assaying several concentrations within the range from 0 micromolar to about 300 micromolar is commonly useful to achieve these goals. It will be possible or even desirable to conduct certain of these assays at concentrations higher than 300 micromolar, such as, for example, 350 micromolar, 400 micromolar, 450 micromolar, 500 micromolar, 600 micromolar, 700 micromolar, 800 micromolar, 900 micromolar, or even at millimolar concentrations. The estimated therapeutically effective concentration of a compound provides initial guidance as to upper ranges of concentrations to test.

High throughput assays for screening numerous compounds for toxicity are specifically contemplated. In certain embodiments, the high throughput screens may be automated. In high throughput screening assays, groups of compounds are exposed to a biological target. These groups may be assembled from collections of compounds previously individually prepared and since stored in a compound bank, the assembly being random or guided by the use of similarity programs from which similar structures are formed.

Procedures for designing and conducting toxicity tests in *in vitro* and *in vivo* systems are well known, and are described in many texts on the subject, such as Loomis et al. Loomis's Essentials of Toxicology, 4th Ed. (Academic Press, New York, 1996); Echobichon, The Basics of Toxicity Testing (CRC Press, Boca Raton, 1992); Frazier, editor, In Vitro Toxicity Testing (Marcel Dekker, New York, 1992); and the like. Typically, the various assays described in the present specification may employ cells seeded in 96 well plates, 384 cell plates, or any other suitable cell culture means. The cells are then exposed to the test compounds over a concentration range, for example, 0-300 micromolar. The cells are incubated in these concentrations for a given period of, for example, 6 and/or 24 hours. Subsequent to the incubation, the assays are performed for each test compound. In one embodiment, all the assays are performed at the same time such that a complete set of data are generated under similar conditions of culture, time and handling. However, it may be that the assays are performed in batches within a few days of each other.

### Organoids and Potency Assays

The instant invention further provides organoids comprising and/or formed from the bioactive components described herein, *e*.*g*., B2, B4, and B3, which are depleted of inactive or undesired components, *e*.*g*., B1 and B5, alone or admixed. In one aspect, the present invention provides organiods comprising and/or formed from a specific subfraction, B4, depleted of or deficient in one or more cell types, *e.g.,* vascular, endocrine, or endothelial, *i.e*., B4', retains therapeutic properties, *e*.*g*., stabilization and/or improvement and/or regeneration of kidney function, alone or when admixed with other bioactive subfractions, *e*.*g*., B2 and/or B3. In a preferred embodiment, the bioactive cell population is B2. In certain embodiments, the B2 cell population is admixed with B4 or B4'. In other embodiments, the B2 cell population is admixed with B3. In other embodiments, the B2 cell population is admixed with both B3 and B4, or specific cellular components of B3 and/or B4. In all embodiments, the organoids of the invention are formed and cultured *ex vivo.*

In one embodiment, the organoids of the invention comprise or are formed from a B2 cell population, wherein the B2 cell population comprises an enriched population of tubular cells. In another embodiment, the heterogenous renal cell population further comprises a B4 cell population. In yet another embodiment, the heterogeneous renal cell population further comprises a B3 population. In still another embodiment, the heterogeneous renal cell population further comprises a B5 population.

In certain embodiments, the cell population comprises a B2 cell population, wherein the B2 cell population comprises an enriched population of tubular cells, and is depleted of a B1 cell population, and/or a B5 cell population.

In another aspect, the invention provides methods of forming organoids comprising and/or formed from bioactive components of the invention, *e*.*g*., B2, B4, and B3, which are depleted of inactive or undesired components, *e*.*g*., B1 and B5, alone or admixed. n one aspect, the present invention provides organiods comprisind and/or formed from a specific subfraction, B4, depleted of or deficient in one or more cell types, *e*.*g*., vascular, endocrine, or endothelial, *i.e*., B4', retains therapeutic properties, *e.g*., stabilization and/or improvement and/or regeneration of kidney function, alone or when admixed with other bioactive subfractions, *e*.*g*., B2 and/or B3. In a preferred embodiment, the bioactive cell population is B2. In certain embodiments, the B2 cell population is admixed with B4 or B4'. In other embodiments, the B2 cell population is admixed with B3. In other embodiments, the B2 cell population is admixed with both B3 and B4, or specific cellular components of B3 and/or B4.

In one embodiment, the organoids of the invention comprise and/or are formed from a B2 cell population, wherein the B2 cell population comprises an enriched population of tubular cells. In another embodiment, the heterogenous renal cell population further comprises a B4 cell population. In yet another embodiment, the heterogeneous renal cell population further comprises a B3 population. In still another embodiment, the heterogeneous renal cell population further comprises a B5 population.

In certain embodiments, the cell population comprises a B2 cell population, wherein the B2 cell population comprises an enriched population of tubular cells, and is depleted of a B1 cell population, and/or a B5 cell population.

In another aspect, the present invention provides methods of forming organoids using the bioactive cell preparations and/or admixtures described herein. General methods for generating tubules from primary renal cell populations using 3D COL(I) gel culture are known in the art, for example, as in Joraku et al., Methods. 2009 Feb;47(2):129-33. In all embodiments, the organoids of the invention are formed and cultured *ex vivo.*

In some embodiments, formation of organoids and tubules from the bioactive cell preparations and/or admixtures described herein may be induced, for example and without limitation, using the following culture methods or systems: i) 2D culture; ii) 3D culture: COL(I) gel; iii) 3D culture: Matrigel; iv) 3D culture: spinners, then COL(I)/Matrigel; and v) 3D culture: COL(IV) gel. Specific examples of formation of organoids and tubules from NKA are provided in Example 18 below.

In one embodiment, organoids formed from the bioactive cell preparations and/or admixtures described herein may be induced in 2D culture. In one embodiment, the bioactive cell preparations and/or admixtures described herein are seeded on standard 2D plastic-ware. In one embodiment, cells are seeded at a density of about 5000 cells/cm². Cells may be seeded in an appropriate medium, such as, for example Renal Cell Complete Growth Media (RCGM). In general, cell populations may be grown past confluence for about 7, 8, 9, 10, 11, 12, 13, 14, 15 days or more, with regular changes of media about every 3-4 days. In one embodiment, cells demonstrate spontaneous self-organization into spheroidal structures, *i.e.,* organoids, and tubules between about 7 to about 15 days.

In another embodinment, organoids formed from the bioactive cell preparations and/or admixtures described herein may be induced in 3D culture. In one embodiment, formulated the bioactive cell preparations and/or admixtures described herein may be incorporated into a collagen (I) gel, collagen (IV) gel, Matrigel or a mixture of any of these as previously described (see Guimaraes-Souza et al., 2012. In vitro reconstitution of human kidney structures for renal cell therapy. Nephrol Dial Transplant 0: 1-9). The liquid gel may be brought to a neutral pH and the bioactive cell preparations and/or admixtures described herein mixed in at about 500-2500 cells/ul. In one embodiment, about 1000 cells/ul are mixed in. The cell/gel mixture may be aliquoted into a well of a 24 well plate, for example, (about 200 to about 400ul/well) and allowed to solidify at 37 degrees C for several hours. Cell culture media may then added and the cultures allowed to mature for about 4, about 5, about 6, about 7, about 8, about 9, or about 10 days with regular changes of media. In one embodiment networks of tubular structures organize as lattices and rings form throughout the gel matrix by the bioactive cell preparations and/or admixtures described herein.

In another embodiment, organoids may be formed by suspension culture of the bioactive cell preparations and/or admixtures described herein in spinner flasks or low-bind plasticware. In one embodiment, cells may be cultured in media in spinner flasks for up to 4 days at about 80rpm. Spheroids may then be further cultured for about 7, about 8, about 9, or about 10 days on Matrigel coated plates, for example. In one embodiment, spheroids formed from the bioactive cell preparations and/or admixtures described herein show tubulogenic potential as shown by de novo budding of tubular structures from cultured spheroids.

The *in vitro* formation of renal organoids and tubules from cultured bioactive cell preparations and/or admixtures described herein has been found to be an indicator of *in vivo* potency of the bioactive cell preparations and/or admixtures described herein. Thus, in one aspect, the invention provides organoid/tubulogenesis-based *in vitro* potency assays for determining or predicting the potential regenerative bioactivity *in vivo* (i.e., potency) of the bioactive cell preparations and/or admixtures described herein. Site specific engraftment and *de novo* regeneration, *i.e*., formation of organoids and/or tubulogenesis and/or glomerulogenesis, of the bioactive cell preparations and/or admixtures described herein may be determined through the use of these *in vitro* potency assays. In one aspect, the invention provides a method of determining a regenerative potential of a heterogeneous cell population comprising a B2 cell population comprising an enriched population of tubular cells, and wherein the heterogeneous renal cell population is depleted of a B1 cell population, comprising a) culturing the heterogeneous renal cell population; and b) determining the regenerative potential of the heterogeneous cell population, wherein the formation of tubules and/or organoids is indicative of a regenerative potential. In one embodiment, tubule or organoid formation may be quantitated by determining the total number of tubules/organoids per field (microscope view) or per unit area of 2D cell surface growth or per unit volume of 3D culture. For example, in a certain embodiment, a photograph may be taken of a microscope field, the photo may be divided into a grid pattern, and the number of tubules or organoids in 50-100 grid squares may be scored. In another embodiment, tubule formation may be quantitated by determining the number of lattices or closed networks of tubules, the number of intersection points between tubules per unit area, volume or microscope field. In yet another embodiment, formation of spheroidal organoids may be quantitated by determining the number of tubules budding per spheroid or the number of branches formed from budded tubules. In another embodiment, tubulogenic potential may be determined by staining the tubules/organoids with a fluorescent dye, such as, for example, calcein which allows the tubules to be imaged by confocal microscopy. Software packages which allow the number of tubules, organoids, lattices, intersection points to be scored by the software are known in the art and commercially available.

### Kits

The instant invention further includes kits comprising the polymeric matrices of the invention and related materials, and/or cell culture media, and/or drug screening assay reagents and instructions for use. The instructions for use may contain, for example, instructions for culture of the cells and the methods of screening test agents using the cells. The instructions for use further may contain, for example, instructions for culture of the cells and the *in vitro* methods for determining the potential regenerative bioactivity *in vivo* (i.e., potency) of the bioactive cell preparations and/or admixtures described herein.

The following examples are offered for illustrative purposes only, and are not intended to limit the scope of the present invention in any way.

### EXAMPLES

### EXAMPLE 1 - Isolation & Characterization of Bioresponsive Renal Cells

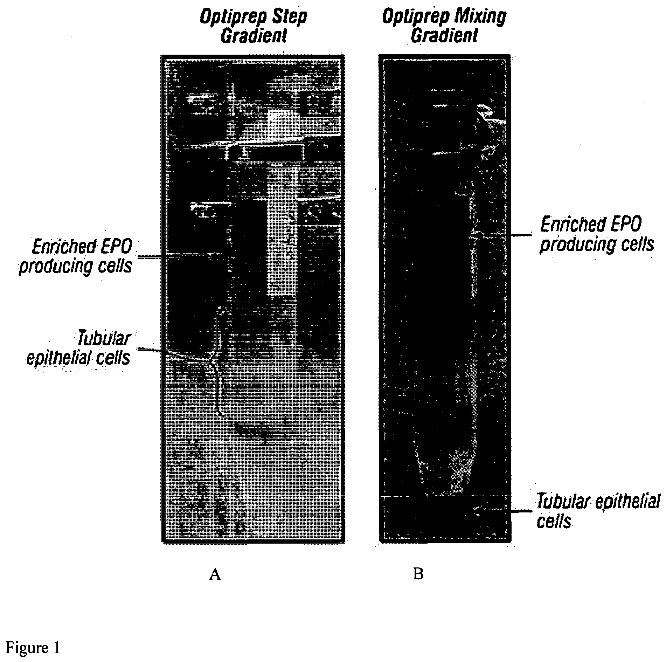

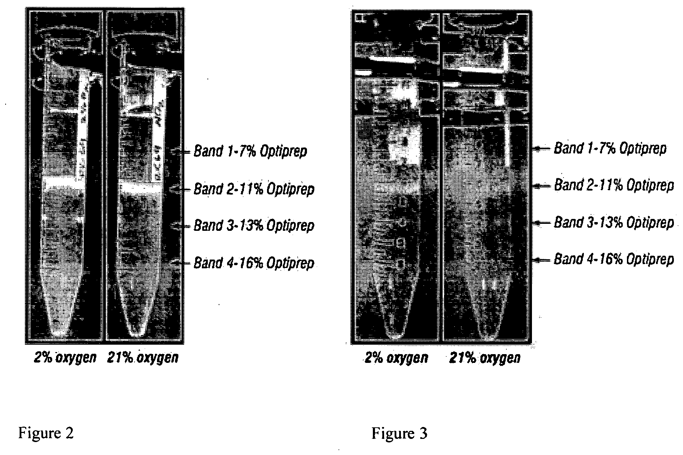

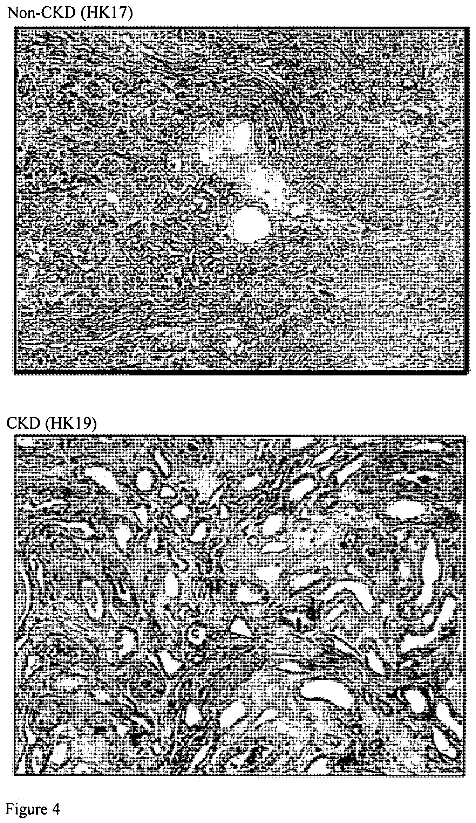

A case of idiopathic progressive chronic kidney disease (CKD) with anemia in an adult male swine (*Sus scrofa*) provided fresh diseased kidney tissue for the assessment of cellular composition and characterization with direct comparison to age-matched normal swine kidney tissue. Histological examination of the kidney tissue at the time of harvest confirmed renal disease characterized by severe diffuse chronic interstitial fibrosis and crescentic glomerulonephritis with multifocal fibrosis. Clinical chemistry confirmed azotemia (elevation of blood urea nitrogen and serum creatinine), and mild anemia (mild reduction in hematocrit and depressed hemoglobin levels). Cells were isolated, expanded, and characterized from both diseased and normal kidney tissue. As shown in Figure 1 of Presnell et al. WO/2010/056328, a Gomori's Trichrome stain highlights the fibrosis (blue staining indicated by arrows) in the diseased kidney tissue compared to the normal kidney tissue. Functional tubular cells, expressing cubulin:megalin and capable of receptor-mediated albumin transport, were propagated from both normal and diseased kidney tissue. Erythropoietin (EPO)-expressing cells were also present in the cultures and were retained through multiple passages and freeze/thaw cycles. Furthermore, molecular analyses confirmed that the EPO-expressing cells from both normal and diseased tissue responded to hypoxic conditions *in vitro* with HIF1α-driven induction of EPO and other hypoxia-regulated gene targets, including vEGF. Cells were isolated from the porcine kidney tissue via enzymatic digestion with collagenase + dispase, and were also isolated in separate experiments by performing simple mechanical digestion and explant culture. At passage two, explant-derived cell cultures containing epo-expressing cells were subjected to both atmospheric (21%) and varying hypoxic (<5%) culture conditions to determine whether exposure to hypoxia culminated in upregulation of EPO gene expression. As noted with rodent cultures (see Example 3), the normal pig displayed oxygen-dependent expression and regulation of the EPO gene. Surprisingly, despite the uremic / anemic state of the CKD pig (Hematocrit <34, Creatinine >9.0) EPO expressing cells were easily isolated and propagated from the tissue and expression of the EPO gene remained hypoxia regulated, as shown in Figure 2 of Presnell et al. WO/2010/056328. As shown in Figure 3 of Presnell et al. WO/2010/056328, cells in the propagated cultures demonstrated the ability to self-organize into tubule-like structures. As shown in Figure 4 of Presnell et al. WO/2010/056328, the presence of functional tubular cells in the culture (at passage 3) was confirmed by observing receptor-mediated uptake of FITC-conjugated Albumin by the cultured cells. The green dots (indicated by thin white arrows) represent endocytosed fluorescein-conjugated albumin which is mediated by tubular cell-specific receptors, Megalin and Cubilin, indicating protein reabosroption by functional tubular cells. The blue staining (indicated by thick white arrows) is Hoescht-stained nuclei. Taken together, these data suggest that functional tubular and endocrine cells can be isolated and propagated from porcine renal tissues, even in renal tissues that have been severely compromised with CKD. Furthermore, these findings support the advancement of autologous cell-based therapeutic products for the treatment of CKD.

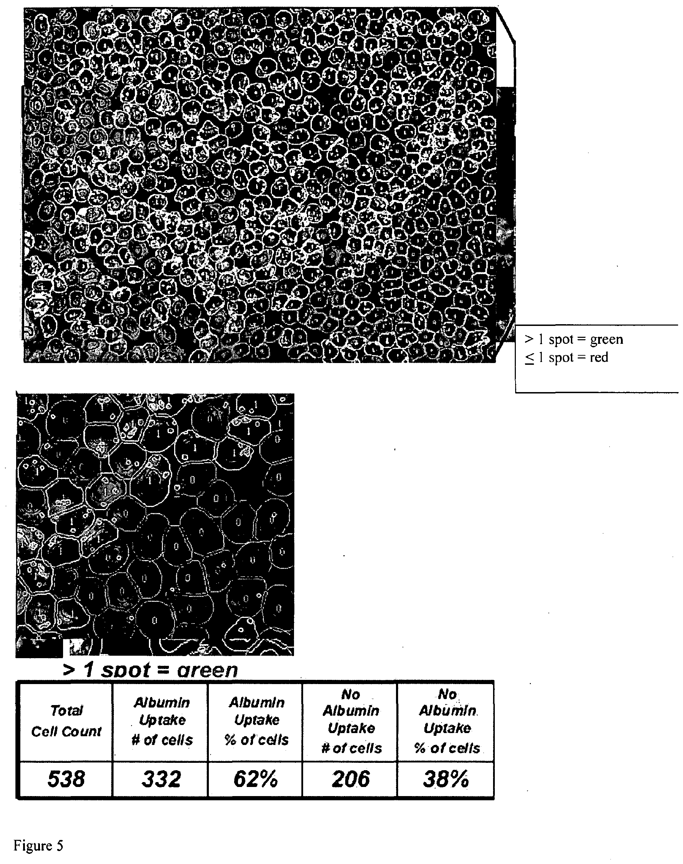

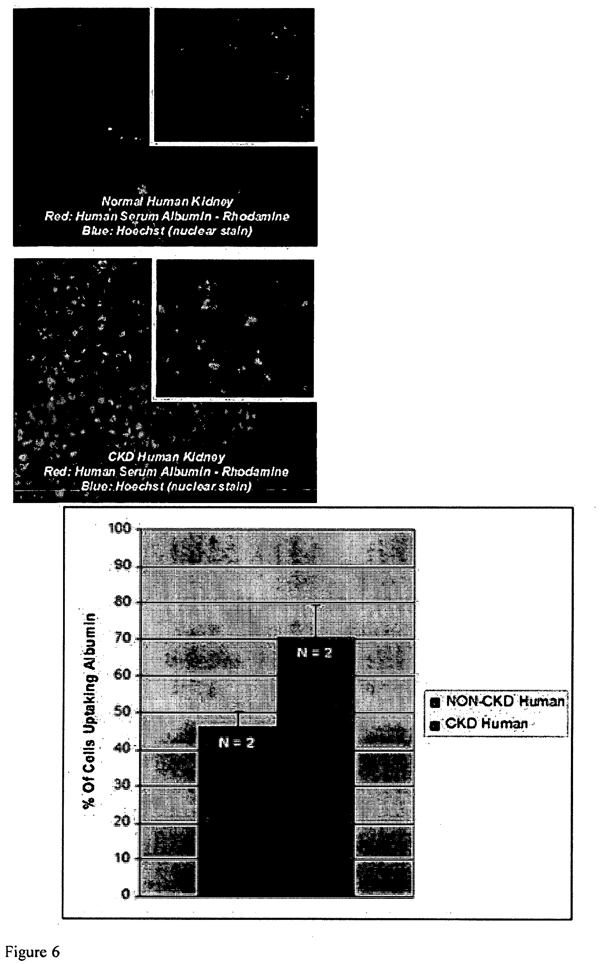

In addition, EPO-producing cells were isolated enzymatically from normal adult human kidney (as described above in Example 1). As shown in Figure 5 of Presnell et al. WO/2010/056328, the isolation procedure resulted in more relative EPO expression after isolation than in the initial tissue. As shown in Figure 6 of Presnell et al. WO/2010/056328, it is possible to maintain the human EPO producing cells in culture with retention of EPO gene expression. Human cells were cultured/propagated on plain tissue-culture treated plastic or plastic that had been coated with some extracellular matrix, such as, for instance, fibronectin or collagen, and all were found to support EPO expression over time.

### EXAMPLE 2 - Isolation & enrichment of specific bioreactive renal cells

Kidney cell isolation: Briefly, batches of 10, 2-week-old male Lewis rat kidneys were obtained from a commercial supplier (Hilltop Lab Animals Inc.) and shipped overnight in Viaspan preservation medium at a temperature around 4°C. All steps described herein were carried out in a biological safety cabinet (BSC) to preserve sterility. The kidneys were washed in Hank's balanced salt solution (HBSS) 3 times to rinse out the Viaspan preservation medium. After the third wash the remaining kidney capsules were removed as well as any remaining stromaltissue. The major calyx was also removed using micro dissection techniques. The kidneys were then finely minced into a slurry using a sterile scalpel. The slurry was then transferred into a 50ml conical centrifuge tube and weighed. A small sample was collected for RNA and placed into an RNAse-free sterile 1.5ml micro-centrifuge tube and snap frozen in liquid nitrogen. Once frozen, it was then transferred to the -80 degree freezer until analysis. The tissue weight of 10 juvenile kidneys equaled approximately 1 gram. Based on the weight of the batch, the digestion medium was adjusted to deliver 20mls of digestion medium per 1 gram of tissue. Digestion buffer for this procedure contained 4 Units of Dispase 1(Stem Cell Tech) in HBSS, 300Units/ml of Collagenase type IV (Worthington) with 5mM CaCl₂ (Sigma).

The appropriate volume of pre-warmed digestion buffer was added to the tube, which was then sealed and placed on a rocker in a 37°C incubator for 20 minutes. This first digestion step removes many red blood cells and enhances the digestion of the remaining tissue. After 20 minutes, the tube was removed and placed in the BSC. The tissue was allowed to settle at the bottom of the tube and then the supernatant was removed. The remaining tissue was then supplemented with fresh digestion buffer equaling the starting volume. The tube was again placed on a rocker in a 37°C incubator for an additional 30 minutes.

After 30 minutes the digestion mixture was pipetted through a 70µm cell strainer (BD Falcon) into an equal volume of neutralization buffer (DMEM w/ 10% FBS) to stop the digestion reaction. The cell suspension was then washed by centrifugation at 300xg for 5 min. After centrifugation, the pellet was then re-suspended in 20mls KSFM medium and a sample acquired for cell counting and viability assessment using trypan blue exclusion. Once the cell count was calculated, 1 million cells were collected for RNA, washed in PBS, and snap frozen in liquid nitrogen. The remaining cell suspension was brought up to 50mls with KSFM medium and washed again by centrifugation at 300xg for 5 minutes. After washing, the cell pellet was re-suspended in a concentration of 15 million cells per ml of KSFM.

Five milliliters of kidney cell suspension were then added to 5mls of 30% (w/v) Optiprep® in 15ml conical centrifuge tubes (BD Falcon) and mixed by inversion 6 times. This formed a final mixture of 15% (w/v) of Optiprep®. Post inversion, tubes were carefully layered with 1 mL PBS. The tubes were centrifuged at 800 x g for 15 minutes without brake. After centrifugation, the tubes were removed and a cell band was formed at the top of the mixing gradient. There was also a pellet containing red blood cells, dead cells, and a small population of live cells that included some small less granular cells, some epo-producing cells, some tubular cells, and some endothelial cells. The band was carefully removed using a pipette and transferred to another 15ml conical tube. The gradient medium was removed by aspiration and the pellet was collected by re-suspension in 1 ml KSFM. The band cells and pellet cells were then recombined and re-suspended in at least 3 dilutions of the collected band volume using KSFM and washed by centrifugation at 300xg for 5 minutes. Post washing, the cells were re-suspended in 20mls of KSFM and a sample for cell counting was collected. Once the cell count was calculated using trypan blue exclusion, 1 million cells were collected for an RNA sample, washed in PBS, and snap frozen in liquid nitrogen.

Pre-Culture 'Clean-up' to enhance viability and culture performance of Specific Bioactive Renal Cells Using Density Gradient Separation: To yield a clean, viable population of cells for culture, a cell suspension was first generated as described above in "Kidney Cell Isolation". As an optional step and as a means of cleaning up the initial preparation, up to 100 million total cells, suspended in sterile isotonic buffer were mixed thoroughly 1:1 with an equal volume of 30% Optiprep® prepared at room temperature from stock 60% (w/v) iodixanol (thus yielding a final 15% w/v Optiprep solution) and mixed thoroughly by inversion six times. After mixing, 1ml PBS buffer was carefully layered on top of the mixed cell suspension. The gradient tubes were then carefully loaded into the centrifuge, ensuring appropriate balance. The gradient tubes were centrifuged at 800 x g for 15 minutes at 25°C without brake. The cleaned-up cell population (containing viable and functional collecting duct, tubular, endocrine, glomerular, and vascular cells) segmented between 6% and 8% (w/v) Optiprep®, corresponding to a density between 1.025 - 1.045 g/mL. Other cells and debris pelleted to the bottom of the tube.

Kidney Cell Culture: The combined cell band and pellet were then plated in tissue culture treated triple flasks (Nunc T500) or equivalent at a cell concentration of 30,000 cells per cm2 in 150mls of a 50:50 mixture of DMEM(high glucose)/KSFM containing 5% (v/v)FBS, 2.5µg EGF, 25mg BPE, 1X ITS (insulin/transferrin/sodium selenite medium supplement) with antibiotic/antimycotic. The cells were cultured in a humidified 5% CO2 incubator for 2-3 days, providing a 21% atmospheric oxygen level for the cells. After two days, the medium was changed and the cultures were placed in 2% oxygen-level environment provided by a CO2 /Nitrogen gas multigas humidified incubator (Sanyo) for 24hrs. Following the 24hr incubation, the cells were washed with 60mls of 1XPBS and then removed using 40mls 0.25% (w/v) trypsin/EDTA (Gibco). Upon removal, the cell suspension was neutralized with an equal volume of KSFM containing 10% FBS. The cells were then washed by centrifugation 300xg for 10 minutes. After washing, the cells were re-suspended in 20mls of KSFM and transferred to a 50ml conical tube and a sample was collected for cell counting. Once the viable cell count was determined using trypan blue exclusion, 1 million cells were collected for an RNA sample, washed in PBS, and snap frozen in liquid nitrogen. The cells were washed again in PBS and collected by centrifugation at 300xg for 5 minutes. The washed cell pellet was re-suspended in KSFM at a concentration of 37.5 million cells/ml.

Enriching for Specific Bioactive Renal Cells Using Density Step Gradient Separation: Cultured kidney cells, predominantly composed of renal tubular cells but containing small subpopulations of other cell types (collecting duct, glomerular, vascular, and endocrine) were separated into their component subpopulations using a density step gradient made from multiple concentrations w/v of iodixanol (Optiprep). The cultures were placed into a hypoxic environment for up to 24 hours prior to harvest and application to the gradient. A stepped gradient was created by layering four different density mediums on top of each other in a sterile 15mL conical tube, placing the solution with the highest density on the bottom and layering to the least dense solution on the top. Cells were applied to the top of the step gradient and centrifuged, which resulted in segregation of the population into multiple bands based on size and granularity.

Briefly, densities of 7, 11, 13, and 16% Optiprep® (60% w/v Iodixanol) were made using KFSM medium as diluents. For example: for 50mls of 7%(w/v) Optiprep®, 5.83mls of stock 60% (w/v) Iodixanol was added to 44.17mls of KSFM medium and mixed well by inversion. A peristaltic pump (Master Flex L/S) loaded with sterile L/S 16 Tygon tubing connected to sterile capillary tubes was set to a flow rate of 2 ml per minute, and 2 mL of each of the four solutions was loaded into a sterile conical 15 mL tube, beginning with the 16% solution, followed by the 13% solution, the 11% solution, and the 7% solution. Finally, 2 mL of cell suspension containing 75 million cultured rodent kidney cells was loaded atop the step gradient (suspensions having been generated as described above in 'Kidney cell Culture'). Importantly, as the pump was started to deliver the gradient solutions to the tube, care was taken to allow the fluid to flow slowly down the side of the tube at a 45° angle to insure that a proper interface formed between each layer of the gradient. The step gradients, loaded with cells, were then centrifuged at 800 x g for 20 minutes without brake. After centrifugation, the tubes were carefully removed so as not to disturb each interface. Five distinct cell fractions resulted (4 bands and a pellet) (B1 - B4, + Pellet) (see Figure 1A, left conical tube). Each fraction was collected using either a sterile disposable bulb pipette or a 5ml pipette and characterized phenotypically and functionally (See Example 10 of Presnell et al. WO/2010/056328). When rodent kidney cell suspensions are subjected to step-gradient fractionation immediately after isolation, the fraction enriched for tubular cells (and containing some cells from the collecting duct) segments to a density between 1.062 - 1.088 g/mL. In contrast, when density gradient separation was performed after *ex vivo* culture, the fraction enriched for tubular cells (and containing some cells from the collecting duct) segmented to a density between 1.051 - 1.062 g/mL. Similarly, when rodent kidney cell suspensions are subjected to step-gradient fractionation immediately after isolation, the fraction enriched for epo-producing cells, glomerular podocytes, and vascular cells ("B4") segregates at a density between 1.025 - 1.035 g/mL. In contrast, when density gradient separation was performed after *ex vivo* culture, the fraction enriched for epo-producing cells, glomerular podocytes, and vascular cells ("B4") segregated at a density between 1.073 - 1.091 g/mL. Importantly, the post-culture distribution of cells into both the "B2" and the "B4" fractions was enhanced by exposure (for a period of about 1 hour to a period of about 24 hours) of the cultures to a hypoxic culture environment (hypoxia being defined as <21% (atmospheric) oxygen levels prior to harvest and step-gradient procedures (additional details regarding hypoxia-effects on band distribution are provided in Example 3).

Each band was washed by diluting with 3x the volume of KSFM, mixed well, and centrifuged for 5 minutes at 300 x g. Pellets were re-suspended in 2mls of KSFM and viable cells were counted using trypan blue exclusion and a hemacytometer. 1 million cells were collected for an RNA sample, washed in PBS, and snap frozen in liquid nitrogen. The cells from B2 and B4 were used for transplantation studies into uremic and anemic female rats, generated via a two-step 5/6 nephrectomy procedure at Charles River Laboratories. Characteristics of B4 were confirmed by quantitative real-time PCR, including oxygen-regulated expression of erythropoietin and vEGF, expression of glomerular markers (nephrin, podocin), and expression of vascular markers (PECAM). Phenotype of the 'B2' fraction was confirmed via expression of E-Cadherin, N-Cadherin, and Aquaporin-2. See Figures 49a and 49b of Presnell et al. WO/2010/056328.

Thus, use of the step gradient strategy allows not only the enrichment for a rare population of epo-producing cells (B4), but also a means to generate relatively enriched fractions of functional tubular cells (B2) (see Figures 50 & 51 of Presnell et al. WO/2010/056328). The step gradient strategy also allows EPO-producing and tubular cells to be separated from red blood cells, cellular debris, and other potentially undesirable cell types, such as large cell aggregates and certain types of immune cells.

The step gradient procedure may require tuning with regard to specific densities employed to provide good separation of cellular components. The preferred approach to tuning the gradient involves 1) running a continuous density gradient where from a high density at the bottom of the gradient (16-21 % Optiprep, for example) to a relatively low density at the top of the gradient (5-10%, for example). Continuous gradients can be prepared with any standard density gradient solution (Ficoll, Percoll, Sucrose, iodixanol) according to standard methods (Axis Shield). Cells of interest are loaded onto the continuous gradient and centrifuged at 800xG for 20 minutes without brake. Cells of similar size and granularity tend to segregate together in the gradients, such that the relative position in the gradient can be measured, and the specific gravity of the solution at that position also measured. Thus, subsequently, a defined step gradient can be derived that focuses isolation of particular cell populations based on their ability to transverse the density gradient under specific conditions. Such optimization may need to be employed when isolating cells from unhealthy vs. healthy tissue, or when isolating specific cells from different species. For example, optimization was conducted on both canine and human renal cell cultures, to insure that the specific B2 and B4 subpopulations that were identified in the rat were isolatable from the other species. The optimal gradient for isolation of rodent B2 and B4 subpopulations consists of (w/v) of 7%, 11%, 13%, and 16% Optiprep. The optimal gradient for isolation of canine B2 and B4 subpopulations consists of (w/v) of 7%, 10%, 11%, and 16% Optiprep. The optimal gradient for isolation of human B2 and B4 subpopulations consists of (w/v) 7%, 9%, 11%, 16%. Thus, the density range for localization of B2 and B4 from cultured rodent, canine, and human renal cells is provided in Table 2.1.

**Table 2.1. Species Density Ranges.**

| | Species Density Ranges g/ml | | |
|---|---|---|---|
| Step Gradient Band | Rodent | Canine | Human |
| B2 | 1.045-1.063g/ml | 1.045-1.058g/ml | 1.045-1.052g/ml |
| B4 | 1.073-1.091g/ml | 1.063-1.091g/ml | 1.063-1.091g/ml |

### EXAMPLE 3 - Low-oxygen culture prior to gradient affects band distribution, composition, and gene expression

To determine the effect of oxygen conditions on distribution and composition of prototypes B2 and B4, neokidney cell preparations from different species were exposed to different oxygen conditions prior to the gradient step. A rodent neo-kidney augmentation (NKA) cell preparation (RK069) was established using standard procedures for rat cell isolation and culture initiation, as described *supra.* All flasks were cultured for 2-3 days in 21% (atmospheric) oxygen conditions. Media was changed and half of the flasks were then relocated to an oxygen-controlled incubator set to 2% oxygen, while the remaining flasks were kept at the 21% oxygen conditions, for an additional 24 hours. Cells were then harvested from each set of conditions using standard enzymatic harvesting procedures described *supra.* Step gradients were prepared according to standard procedures and the "normoxic" (21% oxygen) and "hypoxic" (2% oxygen) cultures were harvested separately and applied side-by-side to identical step gradients. (Figure 2). While 4 bands and a pellet were generated in both conditions, the distribution of the cells throughout the gradient was different in 21 % and 2% oxygen-cultured batches (Table 1). Specifically, the yield of B2 was increased with hypoxia, with a concomitant decrease in B3. Furthermore, the expression of B4-specific genes (such as erythropoietin) was enhanced in the resulting gradient generated from the hypoxic-cultured cells (Figure 73 of Presnell et al. WO/2010/056328).

A canine NKA cell preparation (DK008) was established using standard procedures for dog cell isolation and culture (analogous to rodent isolation and culture procedures), as described *supra.* All flasks were cultured for 4 days in 21% (atmospheric) oxygen conditions, then a subset of flasks were transferred to hypoxia (2%) for 24 hours while a subset of the flasks were maintained at 21%. Subsequently, each set of flasks was harvested and subjected to identical step gradients (Figure 3). Similar to the rat results (Example 1), the hypoxic-cultured dog cells distributed throughout the gradient differently than the atmospheric oxygen-cultured dog cells (Table 3.1). Again, the yield of B2 was increased with hypoxic exposure prior to gradient, along with a concomitant decrease in distribution into B3.

**Table 3.1.**

| | **Rat (RK069)** | | **Dog (DK008)** | |
|---|---|---|---|---|
| | **2% O2** | **21% O2** | **2% O2** | **21% O2** |
| **B1** | 0.77% | 0.24% | 1.20% | 0.70% |
| **B2** | 88.50% | 79.90% | 64.80% | 36.70% |
| **B3** | 10.50% | 19.80% | 29.10% | 40.20% |
| **B4** | 0.23% | 0.17% | 4.40% | 21.90% |

The above data show that pre-gradient exposure to hypoxia enhances composition of B2 as well as the distribution of specific specialized cells (erythropoietin-producing cells, vascular cells, and glomerular cells) into B4. Thus, hypoxic culture, followed by density-gradient separation as described *supra,* is an effective way to generate 'B2' and 'B4' cell populations, across species.

### EXAMPLE 4 - Isolation of tubular/glomerular cells from human kidney

Tubular and glomerular cells were isolated and propagated from normal human kidney tissue by the enzymatic isolation methods described throughout. By the gradient method described above, the tubular cell fraction was enriched ex vivo and after culture. As shown in Figure 68 of of Presnell et al. WO/2010/056328, phenotypic attributes were maintained in isolation and propagation. Tubular cell function, assessed via uptake of labeled albumin, was also retained after repeated passage and cryopreservation. Figure 69 of Presnell et al. WO/2010/056328 shows that when tubular-enriched and tubular-depleted populations were cultured in 3D dynamic culture, a marked increase in expression of tubular marker, cadherin, was expressed in the tubular-enriched population. This confirms that the enrichment of tubular cells can be maintained beyond the initial enrichment when the cells are cultured in a 3D dynamic environment.

### EXAMPLE 5 - Further separation of EPO-producing cells via flow cytometry

The same cultured population of kidney cells described above in Example 2 was subjected to flow cytometric analysis to examine forward scatter and side scatter. The small, less granular EPO-producing cell population was discernable (8.15%) and was separated via positive selection of the small, less granular population using the sorting capability of a flow cytometer (see Figure 70 of Presnell et al. WO/2010/056328).

### EXAMPLE 6 - Characterization of an unfractionated mixture of renal cells isolated from an autoimmune glomerulonephritis patient sample

An unfractionated mixture of renal cells was isolated, as described above, from an autoimmune glomerulonephritis patient sample. To determine the unbiased genotypic composition of specific subpopulations of renal cells isolated and expanded from kidney tissue, quantitative real time PCR (qrtpcr) analysis (Brunskill et al., *supra* 2008) was employed to identify differential cell-type-specific and pathway-specific gene expression patterns among the cell subfractions. As shown in Table 6.1, HK20 is an autoimmune glomerulonephritis patient sample. Table 6.2 shows that cells generated from HK20 are lacking glomerular cells, as determined by qRTPCR.

### EXAMPLE 7 - Genetic profiling of therapeutically relevant renal bioactive cell populations isolated from a case of focal segmental glomerulosclerosis

To determine the unbiased genotypic composition of specific subpopulations of renal cells isolated and expanded from kidney tissue, quantitative real time PCR (qrtpcr) analysis (Brunskill et al., *supra* 2008) was employed to identify differential cell-type-specific and pathway-specific gene expression patterns among the cell subfractions. Human preparation HK023, derived from a case of focal segmental glomerulosclerosis (FSGS) in which a large portion of glomeruli had been destroyed, was evaluated for presence of glomerular cells in the B4 fraction at the time of harvest. In brief, unfractionated (UNFX) cultures were generated (Aboushwareb *et al.*, *supra* 2008) and maintained independently from each of (4) core biopsies taken from the kidney using standard biopsy procedures. After (2) passages of UNFX *ex vivo*, cells were harvested and subjected to density gradient methods (as in Example 8) to generate subfractions, including subfraction B4, which is known to be enriched for endocrine, vascular, and glomerular cells based on work conducted in rodent, dog, and other human specimens.

**Table 6.1**

| **Sample ID** | **Species** | **Age/Gender** | **Etiology of Renal Disease** | **Cause of Death (D) or Kidney Removal (KR)** | **BUN (mg/dL)** | **sCreat (mg/dL)** | **Creatinine Clearance (CC)/GFR/eGFR** | **HCT (%)** | **NB (mg/dL)** | **sPHOS (mg/dL)** | **uPRO** | **Key Histopathologic Features** |
|---|---|---|---|---|---|---|---|---|---|---|---|---|
| PK001 | Swine | >1 yr/M | Idiopathic nephropathy | (D) Renal Failure | 75 | 9.5 | na | 34.1 | 10.6 | 6.3 | na | marked fibrosis; glomerular hypertrophy with focal sclerosis; tubular dilatation with protein casts |
| PK002 | Swine | >1yr/M | no renal disease | (D) Sacrifice | na | na | na | na | na | na | na | normal kidney histology |
| DK001 | Canine | >11yr/M | age-related renal degeneration with fatty metaplasia of flomeruli | (D) Sacrifice | 24 | 1/1 | ma | 40.1 | 13.5 | 6.6 | 0 | diffuse glomerular lipidosis with focal segmental glomerular sclerosis |
| DK002 | Canine | > 2yr/M | chronic glomerulonephrit is | (D) Sacrifice | 20 | 0.8 | na | 47 | 15.9 | 3.6 | >3.0 | chronic glomerulonephriti s with chronic inflammation, glomerular sclerosis, and moderate fibrosis |
| HK016 | Human | 2 mo/F | no renal disease | (D) Head Trauma | 13 | 0.4 | na | 26.6 | 9.6 | 8.6 | trace | normal neonatal kidney histology |
| HK017 | Human | 35 yr/F | Petechial hemorrhage secondary to DIC | (D) CVA | 12 | 2.9 | na | 26 | 8.8 | 6.3 | trace | normal tubular histology; no fibrosis; fibrin thrombi throughout glomerular capillaries |
| HK018 | Human | 48 yr/F | secondary to hypertension, NIDDM, and heart disease | (D) CV/Renal Failure | 40 | 8.6 | 8.06 (CC) | 24.6 | 8.1 | 6.7 | na (anuric) | marked fibrosis; glomerular sclerosis; tubular dilatation with protein casts |
| HK019 | Human | 52 yr/F | secondary to hypertension, NIDDM, and heart disease | (D) CV/ Renal Failure | 127 | 5.7 | 14.5 (CC) | 23.7 | 8.4 | 12.4 | >300 | diffuse moderate glomerular obsolescence with thickening of Bowman's capsule; periglomerulas fibrosis; moderate tubular injury with diffuse tubulo-interstitial fibrosis, tubular dilatation with protein casts. |
| HK020 | Human | 54 yr/F | auto-immune glomerulonephrit is | (D) CV/ Stroke | 94 | 16.6 | 4.35 (CC) | 29 | 9.6 | 5.4 | na (anuric) | Severe end-stage renal disease; no functional glomeruli observed; severe glomerular sclerosis and interstitial fibrosis with chronic inflammation, tubular congestion with protein casts. |
| HK021 | Human | 15 mo/M | no renal disease | (D) trauma | 11 | 0.4 | 73.4 (CC) | 29 | 10.3 | 3.4 | trace | normal kidney histology |
| HK022 | Human | 60 yr/M | secondary to hypertension, heart disease | (D) CVA / Intracranial hemorrhag e | 53 | 3.3 | 17 (GFR) | 31.1 | 10 | 1.8 | 100 | Severe end-stage renal disease; diffuse severe glomerulosclerosis intersfitial fibrosis and tubular atrophy with protein casts. |
| HK023 | Human | 18 yr/M | focal segmental glomerulosclero sis, nephrotic syndrome, hypertension | (KR) failed kidneys removed prior to transplant | 28 | 6.4 | 13.8 (GFR) | 36 | 11.8 | 6.4 | na | focal segmental glomerulosclerosi s (10-15% of glomeruli sclerosed), associated with diffuse mesangial hypercellularity; diffuse, focally accentuated moderate to marked interstitial fibrosis and tubular atrophy; marked chronic active interstitial nephritis |
| CKD Rats (5/6Nx) n=16 | Rat (Lewis) | 4-6 mo/F | renal mass insufficiency | (D) Renal Failure | 96.5 ± 14* | 2.4 ± 0.2* | 0.480.48 ± 0.3* (eGFR) | 39.3 ± 1.8* | 13.2 ± 0.6* | 10.2 ± 1.2* | 1420 ± 535* | interstitial fibrosis; glomerular atrophy and sclerosis; tubular degeneration and dilatation |
| Healthy rats (age-n=16) matched; n=16) | Rat (Lewis) | 4-6 mo/F | None | (D) Sacrifice | 16.9 ± 0.6* | 0.4 ± 0.02* | 1.7 ± 0.1* (eGFR) | 46.1 ± 0.6* | 14.7 ± 0.3* | 6.8 ± 0.3* | 36 ± 13* | normal adult kidney histology |
| Diabetic Nephropat hy Rats (Ob/Ob ZSF1); n=10 | Rat (ZSF1) | 9 mo/M | obesity, diabetes | (D) Sacrifice | 30.9 ± 4.8* | 0.6 ± 0.5* | 3.8 ± 0.3* (eGFR) | na | na | 5.3 ± 0.4* | 931 ± 0.4* | arteriolar thickening, severe tubular degeneration, dilation, and atrophy, and protein casts in the Bowman's space and tubular lumens (REF: Prabhakar, 2007 JASN); by 20 weeks of age |
| Lean ZSF1 Rats (Age-Matched); n=10 | Rat (ZSF1) | 9 mo/M | None | (D) Sacrifice | 18.9 ± 2.9* | 0.4 ± 0.05* | 6.4 ± 1.2* (eGFR) | na | na | 4.6 ± 0.5* | 296 ± 69* | moderate arteriolar thickening; normal tubular and glomerular structures (REF: Prabhakar 2007 JASN); at 20 weeks of age |

**Table 6.2 Compartmental analysis of cultured human, swine, and rat renal cells.**

| Sample ID | TUBULAR | | | | | | GLOMERULAR | | DUCTULAR | OTHER | | | | |
|---|---|---|---|---|---|---|---|---|---|---|---|---|---|---|
| | E-CAD | N-CAD | AQP-1 | CUB | CYP24 | ALB-U | NEPH | PODO | AQP-2 | EPO | vEGF | KDR | CD31 | SSC/FSC |
| PK001 | + | nd | nd | nd | nd | ++ | nd | nd | nd | +R | + | nd | nd | + |
| PK002 | + | nd | nd | nd | nd | + | nd | nd | nd | +R | + | nd | nd | + |
| HK016 | 3.03 | 0.83 | 0.0001 | 0.0006 | 0.055 | + | 0.0004 | 0.0050 | 0.0001 | 0.020R | 0.85 | 0.001 | trace | + |
| HK017 | 0.66 | 0.83 | 0.0009 | 0.0002 | 0.046 | ++ | trace | 0.0001 | 0.0003 | 0.032R | 0.36 | 0.002 | 0.0003 | + |
| HK018 | 0.61 | 1.59 | 0.0001 | 0.0003 | 0.059 | + | 0.0002 | - | - | 0.004R | 0.36 | 0.003 | trace | + |
| HK019 | 0.62 | 2.19 | 0.026 | 0.0008 | 0.068 | +/- | 0.0009 | 0.0003 | 0.0020 | 0.076R | 0.40 | 0.002 | 0.0040 | + |
| HK020 | 0.07 | 1.65 | 0.0003 | 0.0007 | 0.060 | +++ | - | - | - | 0.011R | 0.40 | 0.002 | - | + |
| Healthy Lewis Rat (male) | + | + | + | + | + | + | + | + | + | +R | + | + | + | + |
| Rat CKD model (5/6 NX Lewis) | + | + | + | + | nd | nd | + | + | nd | +R | nd | nd | nd | + |

The B4 fractions were collected separately from each independent UNFX sample of HK023, appearing as distinct bands of cells with buoyant density between 1.063 - 1.091 g/mL. RNA was isolated from each sample and examined for expression of Podocin (glomerular cell marker) and PECAM (endothelial cell marker) by quantitative real-time PCR. As expected from a biopsy-generated sample from a case of severe FSGS, the presence of podocin(+) glomerular cells in B4 fractions was inconsistent, with podocin undetectable in 2/4 of the samples. In contrast, PECAM+ vascular cells were consistently present in the B4 fractions of 4/4 of the biopsy-initiated cultures. Thus, the B4 fraction can be isolated at the 1.063-1.091 g/mL density range, even from human kidneys with severe disease states.

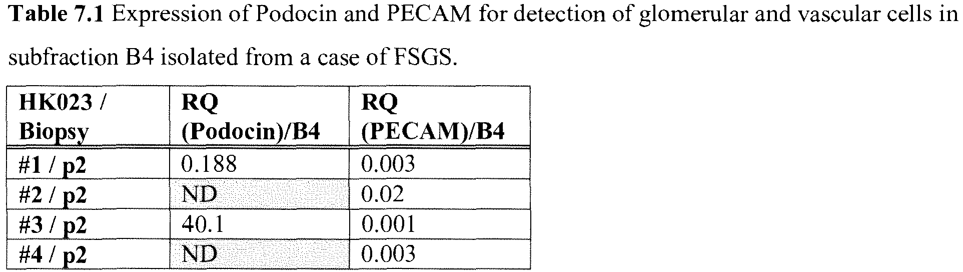

Further, as shown in Table 7.2, human sample (HK018) displayed undetected Podocin (glomerular marker) by qRTPCR after density gradient centrifugation.

**Table 7.2. HK018 Post-Gradient gene expression characterization of B2 & B4'**

| **Gene** | **RQ(Unfx)** | **RQ(B2)** | **RQ(B4)** | **B2/B4** |
|---|---|---|---|---|
| Podocin | 1 | ND | ND | - |
| VegF | 1 | 1.43 | 1.62 | 0.9 |
| Aqp1 | 1 | 1.7 | 1.2 | 1.4 |
| Epo | 1 | 0.9 | 0.5 | 1.8 |
| Cubilin | 1 | 1.2 | 0.7 | 1.7 |
| Cyp | 1 | 1.2 | 1.4 | 0.85 |
| Ecad | 1 | 1.15 | 0.5 | 2.3 |
| Ncad | 1 | 1.02 | 0.72 | 1.4 |

### EXAMPLE 8 - Enrichment/Depletion of Viable Kidney Cell Types Using Fluorescent Activated Cell Sorting (FACS)

One or more isolated kidney cells may be enriched, and/or one or more specific kidney cell types may be depleted from isolated primary kidney tissue using fluorescent activated cell sorting (FACS).

**REAGENTS:** 70% ethanol; Wash buffer (PBS); 50:50 Kidney cell medium (50%DMEM high glucose): 50% Keratinocyte-SFM; Trypan Blue 0.4%; Primary antibodies to target kidney cell population such as CD31 for kidney endothelial cells and Nephrin for kidney glomerular cells. Matched isotype specific fluorescent secondary antibodies; Staining buffer ( 0.05% BSA in PBS)

**PROCEDURE:** Following standard procedures for cleaning the biological safety cabinet (BSC), a single cell suspension of kidney cells from either primary isolation or cultured cells may be obtained from a T500 T/C treated flask and resuspend in kidney cell medium and place on ice. Cell count and viability is then determined using trypan blue exclusion method. For kidney cell enrichment/depletion of, for example, glomerular cells or endothelial cells from a heterogeneous population, between 10 and 50e6 live cells with a viability of at least 70% are obtained. The heterogeneous population of kidney cells is then stained with primary antibody specific for target cell type at a starting concentration of 1µg/0.1ml of staining buffer/1 x 10⁶ cells (titer if necessary). Target antibody can be conjugated such as CD31 PE (specific for kidney endothelial cells) or un-conjugated such as Nephrin (specific for kidney glomerular cells).

Cells are then stained for 30 minutes on ice or at 4°C protected from light. After 30 minutes of incubation, cells are washed by centrifugation at 300xg for 5 min. The pellet is then resuspended in either PBS or staining buffer depending on whether a conjugated isotype specific secondary antibody is required. If cells are labeled with a fluorochrome conjugated primary antibody, cells are resuspended in 2mls of PBS per 10e7 cells and proceed to FACS aria or equivalent cell sorter. If cells are not labeled with a fluorochrome conjugated antibody, then cells are labeled with an isotype specific fluorochrome conjugated secondary antibody at a starting concentration of 1ug/0.1ml/1e6 cells.

Cells are then stained for 30 min. on ice or at 4°C protected from light. After 30 minutes of incubation, cells are washed by centrifugation at 300xg for 5 min. After centrifugation, the pellet is resuspended in PBS at a concentration of 5e6/ml of PBS and then 4mls per 12x75mm is transferred to a sterile tube.

FACs Aria is prepared for live cell sterile sorting per manufacturer's instructions (BD FACs Aria User Manual). The sample tube is loaded into the FACs Aria and PMT voltages are adjusted after acquisition begins. The gates are drawn to select kidney specific cells types using fluorescent intensity using a specific wavelength. Another gate is drawn to select the negative population. Once the desired gates have been drawn to encapsulate the positive target population and the negative population, the cells are sorted using manufacturer's instructions.

The positive target population is collected in one 15ml conical tube and the negative population in another 15 ml conical tube filled with 1 ml of kidney cell medium. After collection, a sample from each tube is analyzed by flow cytometry to determine purity. Collected cells are washed by centrifugation at 300xg for 5 min. and the pellet is resuspended in kidney cell medium for further analysis and experimentation.

### EXAMPLE 9 - Enrichment/Depletion of Kidney Cell Types Using Magnetic Cell Sorting

One or more isolated kidney cells may be enriched and/or one or more specific kidney cell types may be depleted from isolated primary kidney tissue.

**REAGENTS:** 70% ethanol, Wash buffer (PBS), 50:50 Kidney cell medium (50%DMEM high glucose): 50% Keratinocyte-SFM, Trypan Blue 0.4%, Running Buffer(PBS, 2mM EDTA,0.5% BSA), Rinsing Buffer (PBS,2mM EDTA), Cleaning Solution (70% v/v ethanol), Miltenyi FCR Blocking reagent, Miltenyi microbeads specific for either IgG isotype, target antibody such as CD31(PECAM) or Nephrin, or secondary antibody.

**PROCEDURE:** Following standard procedures for cleaning the biological safety cabinet (BSC), a single cell suspension of kidney cells from either primary isolation or culture is obtained and resuspended in kidney cell medium. Cell count and viability is determined using trypan blue exclusion method.

For kidney cell enrichment/depletion of, for example, glomerular cells or endothelial cells from a heterogeneous population, at least 10e6 up to 4e9 live cells with a viability of at least 70% is obtained.

The best separation for enrichment/depletion approach is determined based on target cell of interest. For enrichment of a target frequency of less than 10%, for example, glomerular cells using Nephrin antibody, the Miltenyi autoMACS, or equivalent, instrument program POSSELDS (double positive selection in sensitive mode) is used. For depletion of a target frequency of greater than 10%, the Miltenyi autoMACS, or equivalent, instrument program DEPLETES (depletion in sensitive mode) is used.

Live cells are labeled with target specific primary antibody, for example, Nephrin rb polyclonal antibody for glomerular cells, by adding 1µg/10e6 cells/0,1ml of PBS with 0.05% BSA in a 15ml conical centrifuge tube, followed by incubation for 15 minutes at 4°C.

After labeling, cells are washed to remove unbound primary antibody by adding 1-2ml of buffer per 10e7 cells followed by centrifugation at 300xg for 5min. After washing, isotype specific secondary antibody, such as chicken anti-rabbit PE at 1ug/10e6/0.1ml of PBS with 0.05% BSA, is added, followed by incubation for 15 minutes at 4°C.

After incubation, cells are washed to remove unbound secondary antibody by adding 1-2ml of buffer per 10e7 cells followed by centrifugation at 300xg for 5 min. The supernatant is removed, and the cell pellet is resuspended in 60µl of buffer per 10e7 total cells followed by addition of 20µl of FCR blocking reagent per 10e7 total cells, which is then mixed well. Add 20 µl of direct MACS microbeads (such as anti-PE microbeads) and mix and then incubate for 15 min at 4°C.

After incubation, cells are washed by adding 10-20x the labeling volume of buffer and centrifuging the cell suspension at 300xg for 5 min. and resuspending the cell pellet in 500µl - 2mls of buffer per 10e8 cells.

Per manufacturer's instructions, the autoMACS system is cleaned and primed in preparation for magnetic cell separation using autoMACS. New sterile collection tubes are placed under the outlet ports. The autoMACS cell separation program is chosen. For selection the POSSELDS program is chosen. For depletion the DEPLETES program is chosen.

The labeled cells are inserted at uptake port, then beginning the program.
After cell selection or depletion, samples are collected and placed on ice until use. Purity of the depleted or selected sample is verified by flow cytometry.

### EXAMPLE 10 - Cells with therapeutic potential can be isolated and propagated from normal and chronically-diseased kidney tissue

The objective of the present study was to determine the functional characterization of human NKA cells through high content analysis (HCA). High-content imaging (HCI) provides simultaneous imaging of multiple sub-cellular events using two or more fluorescent probes (multiplexing) across a number of samples. High-content Analysis (HCA) provides simultaneous quantitative measurement of multiple cellular parameters captured in High-Content Images. In brief, unfractionated (UNFX) cultures were generated (Aboushwareb *et al*., *supra* 2008) and maintained independently from core biopsies taken from five human kidneys with advanced chronic kidney disease (CKD) and three non-CKD human kidneys using standard biopsy procedures. After (2) passages of UNFX *ex vivo*, cells were harvested and subjected to density gradient methods (as in Example 2) to generate subfractions, including subfractions B2, B3, and/or B4.

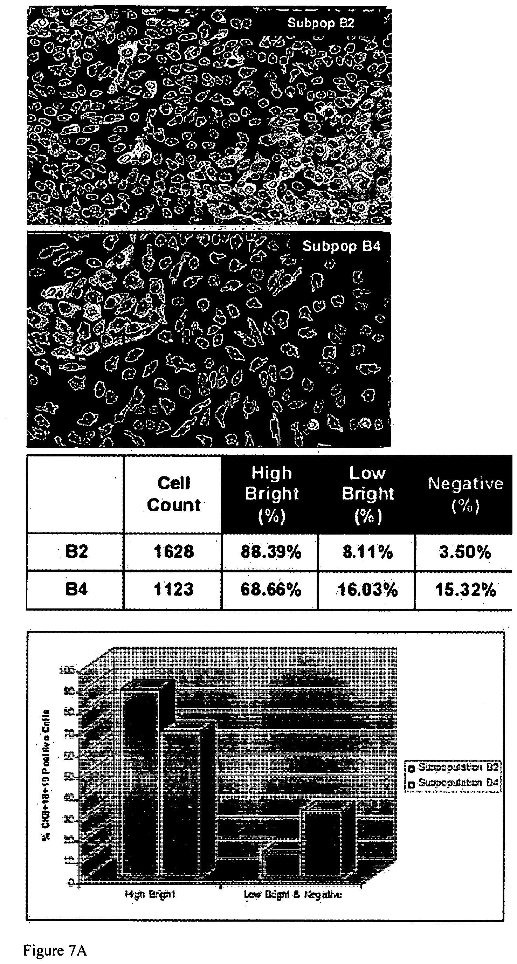

Human kidney tissues were procured from non-CKD and CKD human donors as summarized in Table 10.1. Figure 4 shows histopathologic features of the HK17 and HK19 samples. Ex vivo cultures were established from all non-CKD (3/3) and CKD (5/5) kidneys. High content analysis (HCA) of albumin transport in human NKA cells defining regions of interest (ROI) is shown in Figure 5 (HCA of albumin transport in human NKA cells). Quantitative comparison of albumin transport in NKA cells derived from non-CKD and CKD kidney is shown in Figure 6. As shown in Figure 6, albumin transport is not compromised in CKD-derived NKA cultures. Comparative analysis of marker expression between tubular-enriched B2 and tubular cell-depleted B4 subfractions is shown in Figure 7A (CK8/18/19).

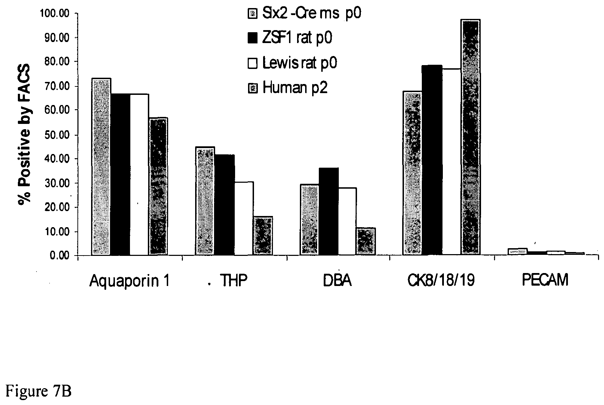

The identification of cell types within propagated cultures were also confirmed across species by detection of tubular markers: Aquaporin 1 and CK 8/18/19; vascular markers: CD31 (PECAM); and ductal markers: DBA. Figure 7B depicts the phenotypic characterization of selected renal cells (SRCs) across species.

**Table 10.1**

| Sample ID | Age/ Gender | Etiology of Renal Disease | Cause of Death (D) or Kidney Removal (KR) | BUN (mg/dL) | sCREAT (mq/dL) | Creatinine Clearance (CC) / GFR/eGFR | HCT (%) | HB (mg/dL) | sPHOS (mg/dL) | uPRO | Key Histopathologic Features |
|---|---|---|---|---|---|---|---|---|---|---|---|
| HK016 | 2 mo / F | no renal disease | (D) Trauma | 13 | 0.4 | na | 26.6 | 9.6 | 8.6 | trace | normal neonatal kidney histology |
| HK017 | 35 yr / F | Petechial hemorrhage secondary to DIC | (D) CVA | 12 | 2.9 | na | 26 | 8.8 | 6.3 | trace | normal tubular histology; no fibrosis; fibrin thrombi throughout glomerular capillaries |
| HK018 | 48 yr / F | secondary to hypertension, NIDDM, and heart disease | (D) CV / Renal Failure | 40 | 8.6 | 8.06 (CC) | 24.6 | 8.1 | 6.7 | na (anuric) | marked fibrosis; glomerular sclerosis; tubular dilatation with protein casts |
| HK019 | 52 yr / F | secondary to hypertension, NIDDM, and heart disease | (D) CV / Renal Failure | 127 | 5.7 | 14.5 (CC) | 23.7 | 8.4 | 12.4 | >300 | diffuse moderate glomerular obsolescence with thickening of Bowman's capsule; peri-glomerular fibrosis; moderate tubular injury with diffuse tubulo-interstitial fibrosis, tubular dilatation with protein casts. |
| HK020 | 54 yr / F | auto-immune glomeruloneph ritis | (D) CV / Stroke | 94 | 16.6 | 4.35 (CC) | 29 | 9.6 | 5.4 | na (anuric) | Severe end-stage renal disease; no functional glomeruli observed; severe glomerular sclerosis and interstitial fibrosis with chronic inflammation, tubular congestion with protein casts. |
| HK021 | 15mo / M | no renal disease | (D) Trauma | 11 | 0.4 | 73.4 (CC) | 29 | 10.3 | 3.4 | trace | normal kidney histology |
| HK022 | 60 yr / M | secondary to hypertension, NIDDM , and heart disease | (D) CVA / Intracranial hemorrhage | 53 | 3.3 | 17 (GFR) | 31.1 | 10 | 1.8 | 100 | Severe end-stage renal disease; diffuse severe glomerulosclerosis; interstitial fibrosis and tubular atrophy with protein casts |
| HK023 | 18 yr / M | focal segmental glomeruloscler osis, nephrotic syndrome, hypertension | (KR) failed kidneys removed prior to transplant | 28 | 6.4 | 13.8 (GFR) | 36 | 11.8 | 6.4 | na | focal segmental glomerulosclerosis (10-15% of glomeruli sclerosed), associated with diffuse mesangial hypercellularity; diffuse, focally accentuated moderate to marked interstitial fibrosis and tubular atrophy; marked chronic active interstitial nephritis |

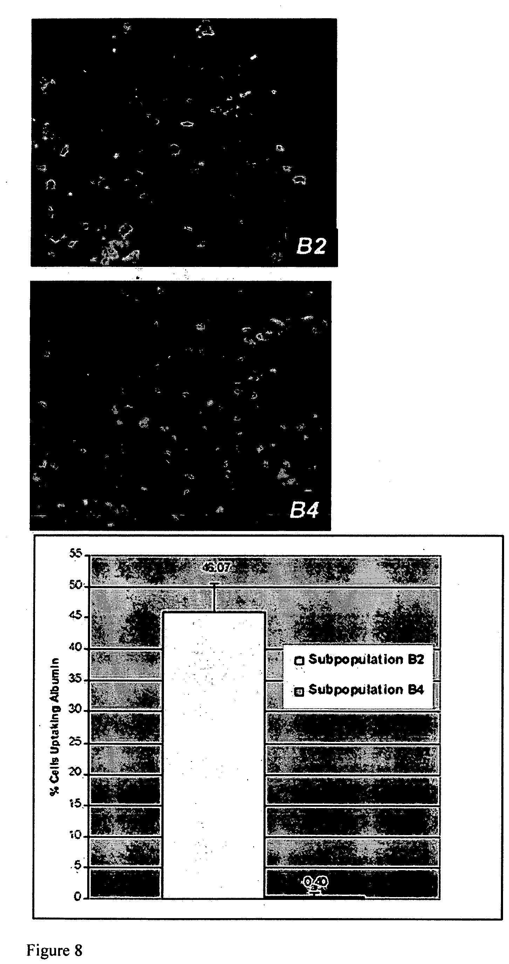

Comparative functional analysis of albumin transport between tubular-enriched B2 and tubular cell-depleted B4 subfractions is shown in Figure 8. Subfraction B2 is enriched in proximal tubule cells and thus exhibits increased albumin-transport function.

*Albumin uptake*: Culture media of cells grown to confluency in 24-well, collagen IV plates (BD Biocoat ™) was replaced for 18-24 hours with phenol red-free, serum-free, low-glucose DMEM (pr-/s-/lg DMEM) containing 1X antimycotic/antibiotic and 2mM glutamine. Immediately prior to assay, cells were washed and incubated for 30 minutes with pr-/s-/lg DMEM + 10mM HEPES, 2mM glutamine,1.8mM CaC12, and 1mM MgC12. Cells were exposed to 25µg/mL rhodamine-conjugated bovine albumin (Invitrogen) for 30 min, washed with ice cold PBS to stop endocytosis and fixed immediately with 2% paraformaldehyde containing 25 µg/mL Hoechst nuclear dye. For inhibition experiments, 1µM receptor-associated protein (RAP) (Ray Biotech, Inc., Norcross GA) was added 10 minutes prior to albumin addition. Microscopic imaging and analysis was performed with a BD Pathway™ 855 High-Content BioImager (Becton Dickinson) (see Kelley et al. Am J Physiol Renal Physiol. 2010 Nov;299(5):F1026-39. Epub Sep 8, 2010).

In conclusion, HCA yields cellular level data and can reveal populations dynamics that are undetectable by other assays, *i.e*., gene or protein expression. A quantifiable *ex-vivo* HCA assay for measuring albumin transport (HCA-AT) function can be utilized to characterize human renal tubular cells as components of human NKA prototypes. HCA-AT enabled comparative evaluation of cellular function, showing that albumin transport-competent cells were retained in NKA cultures derived from human CKD kidneys. It was also shown that specific subfractions of NKA cultures, B2 and B4, were distinct in phenotype and function, with B2 representing a tubular cell-enriched fraction with enhanced albumin transport activity. The B2 cell subpopulation from human CKD are phenotypically and functionally analogous to rodent B2 cells that demonstrated efficacy *in vivo* (as shown above).

### Example 11 - Hypoxic exposure of cultured human renal cells induces mediators of cell migration and attachment and facilitates the repair of tubular cell monolayers in vitro

The role of oxygen tension in the isolation and function of a selected population of renal epithelial cells (B2) with demonstrated therapeutic function in models of chronic kidney disease (CKD) was investigated. This study examined whether low oxygen exposure during processing alters composition and function of selected human selected renal cells (SRCs) or bioactive renal cells (BRCs). Upon exposure to 2% Oxygen, the following was observed: an alteration of the distribution of cells across a density gradient (see Presnell et al. WO 10/056328), improvement in overall post-gradient yield, modulation of oxygen-regulated gene expression (previously reported in Kelley et al. *supra* (2010)), increased expression of erythropoietin, VEGF, HIF1-alpha, and KDR(VEGFR2). In-process exposure to low oxygen enhances the ability of selected bioactive renal cells to repair/regenerate damaged renal tubules.

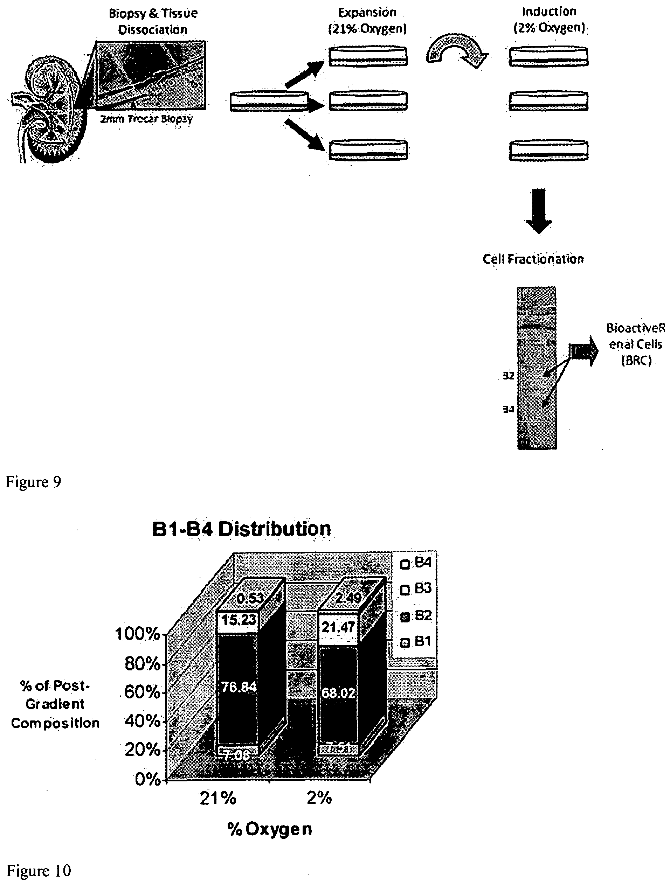

Figure 9 depicts the procedure for exposing cells to low oxygen during processing. Figure 10 shows that upon exposure to 2% Oxygen, the following was observed: alters distribution of cells across a density gradient, improves overall post-gradient yield. Hypoxic exposure (<3%) increased recovery of cultured human CKD-derived renal cells from iodixanol-based density gradients relative to atmospheric oxygen tension (21%) (96% vs. 74%) and increased the relative distribution of selected cells (B2) into high-density (>9% iodixanol) fractions (21.6% vs. 11.2%).

Competitive *in vitro* assays demonstrated that B2 cells pre-exposed for 24 hours to hypoxic conditions were more proficient in repairing damaged renal proximal tubular monolayer cultures than B2 cells cultured at 21% oxygen tension, with 58.6% ± 3% of the repair occurring within two hours of injury.

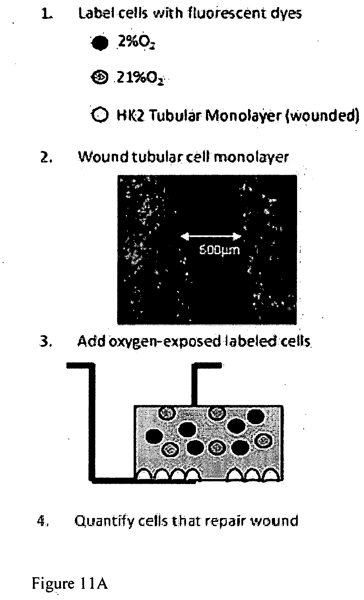

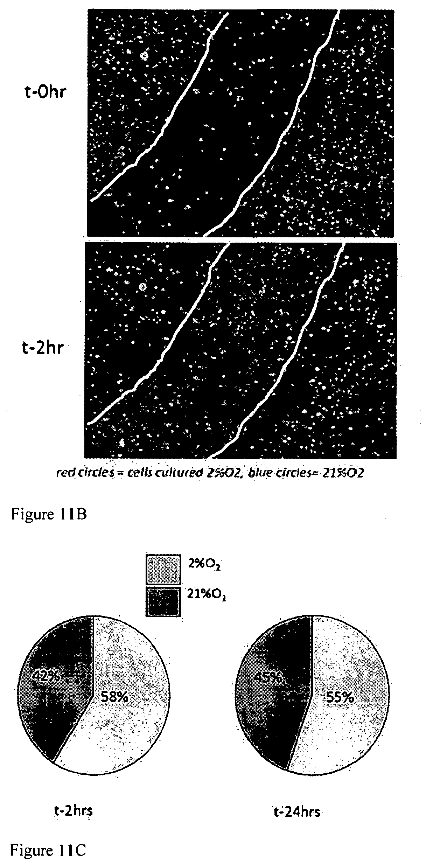

Figures 11A depicts an assay developed to observe repair of tubular monolayers in vitro. 1. Cells are labeled with fluorescent dyes (2% oxygen, 21% oxygen, and HK2 tubular cells). 2. The tubular cell monolayer was established and wounded. 3. Oxygen-exposed labeled cells are added (2% and 21% exposed cells). They are seeded equally at 20,000/cm2. Culturing is in serum-free media at 5%O2 for 24hrs. 4. Cells that repair wounding are quantified. Figure 11B - Quantitative Image Analysis (BD Pathway 855 BioImager) - red circles = cells cultured 2% O2, blue circles= 21% O2. Figure 11C - it was observed that 2% oxygen-induced cells attached more rapidly (2 hrs) and sustained a mild advantage for 24 hrs. Cells induced with 2% oxygen were more proficient at repair of tubular epithelial monolayers.

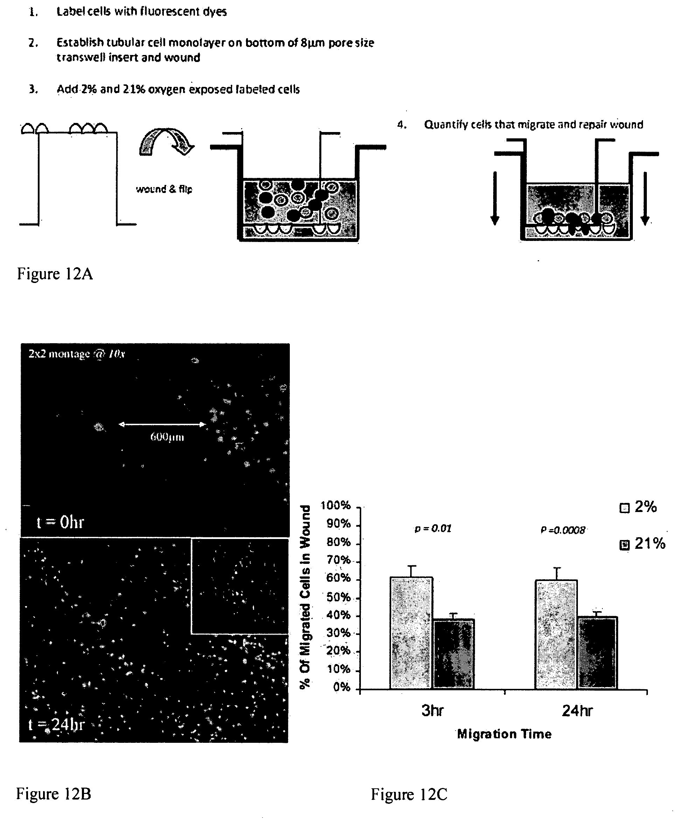

Figure 12A depicts an assay developed to observe repair of tubular monolayers in vitro. 1. Cells were labeled with fluorescent dyes. 2. The tubular cell monolayer was established on the bottom of 8 µm pore size transwell inserts and wounded. 3. The inserts are flipped and oxygen-exposed labeled cells are added (2% and 21% exposed cells). They are seeded equally at 50,000/cm2. Culturing is in serum-free media at 5%O2 for 24hrs. 4. Cells that repair wounding are quantified.

Figure 12B shows that the induction of cells with 2% Oxygen enhanced the migration and wound repair compared to un-induced (21 % oxygen). Figure 12C plots the % of migrated cells against the migration time. The average number of cells and average percentage of cells are provided in Table 11.1.

Hypoxia also induced mRNA expression of CXCR4, MMP9, ICAM1, and dystroglycan; genes that mediate cell migration and attachment. Focal accumulation of MMP9 and an increase in Connexin 43 aggregates on the cells' plasma membrane was confirmed by immunocytochemistry.

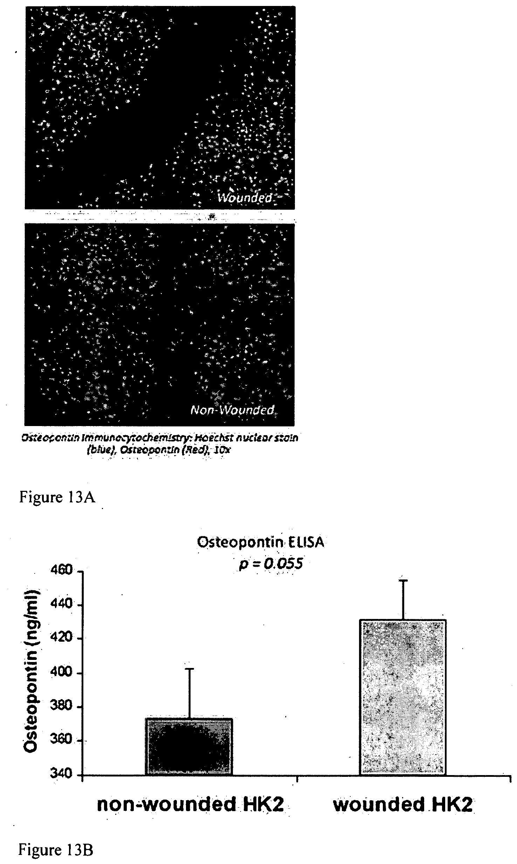

Figure 13A shows that osteopontin is secreted by tubular cells and is upregulated in response to injury (Osteopontin Immunocytochemistry: Hoechst nuclear stain (blue), Osteopontin (Red), 10x). Osteopontin is a secreted phosphorylated glycoprotein (Kelly et al. J Am Soc Soc Nephrol, 1999). Osteopontin is expressed in kidney tubules and is involved in adhesion and migration. Osteopontin is upregulated by injury in established tubular cell monolayers as shown by immunoflluorescence (Figure 13A) and ELISA (Figure 13B).

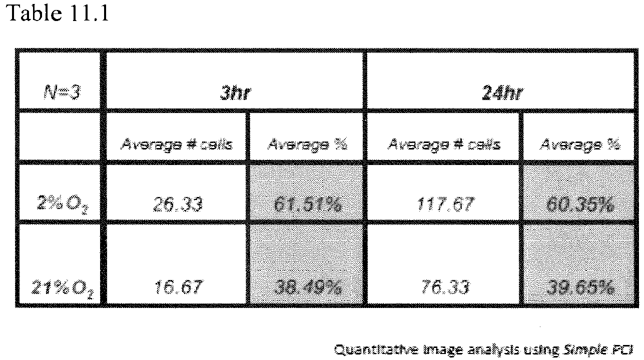

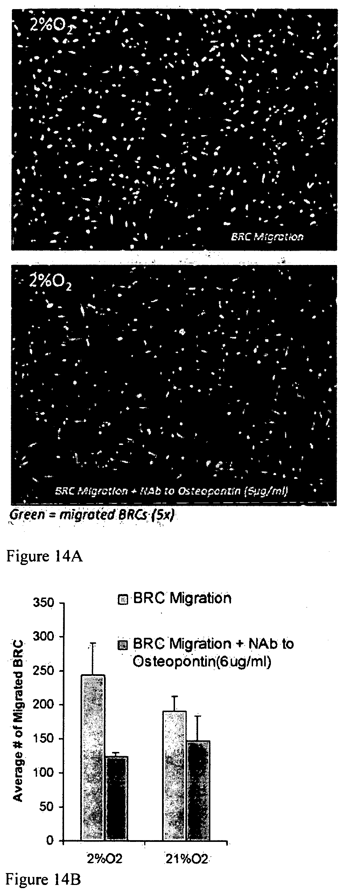

Figure 14A shows that the migratory response of cells is mediated in part by osteopontin (Green = migrated cells (5x)). Figure 14B shows that neutralizing antibodies (NAb) to osteopontin reduce renal cell migration response by 50%.

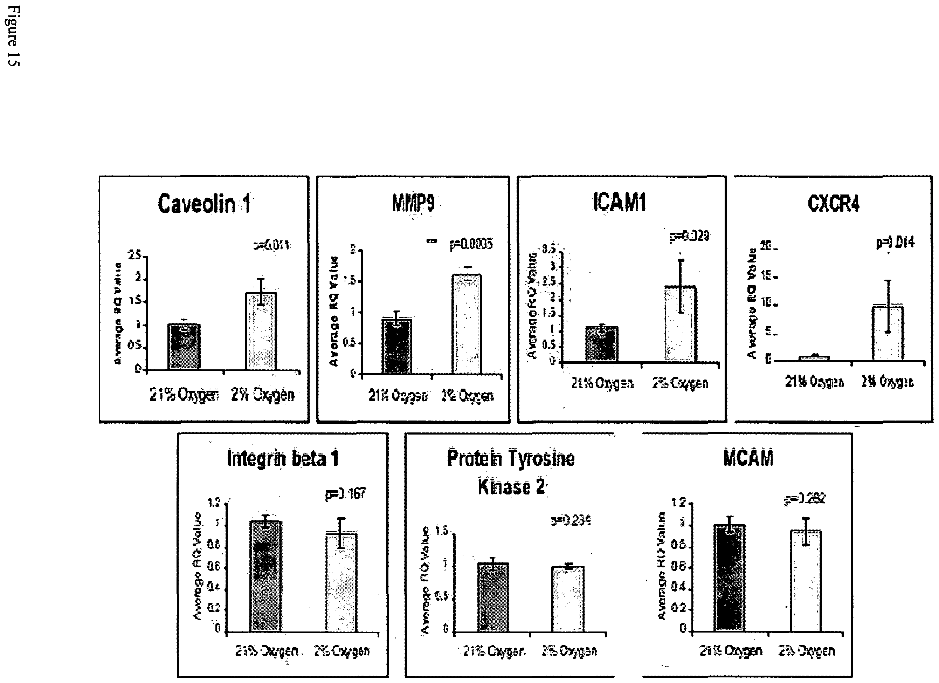

Figure 15 shows that low-oxygen induction of cells modulates expression of tissue remodeling genes. Caveolin 1 is a scaffolding protein involved in modulation of integrin signaling. MMP9 is a metalloproteinase that facilitates migration through extracellular matrix degradation. ICAM1 is an intercellular adhesion molecule associated with epithelial cell motility. CXCR4 is a chemokine surface receptor that mediates cell migration.

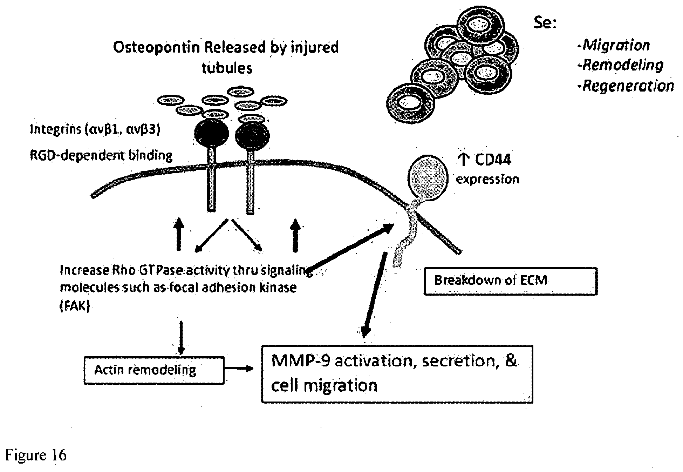

Figure 16 depicts a putative mechanism for low oxygen augmentation of bioactivity of cells leading to renal regeneration.

Taken together, these results suggest that hypoxic exposure facilitates the isolation of a specific renal cell subpopulation with demonstrated bioactivity for repair of tubular injury *in vitro,* and thus may potentially enhance the ability of these cells to migrate and engraft into diseased tissue after *in vivo* delivery. The SRCs demonstrated the ability to stabilize renal function and enhance survival in a rodent model of progressive CKD. The low oxygen levels (2% O2) provided the following: enhanced post-culture recovery of selected regenerative cells; enhanced cellular attachment and monolayer repair in response to tubular injury; and stimulated cellular migration in response to tubular injury. In addition, cellular migration and attachment were mediated in part by osteopontin in vitro, low-oxygen upregulated integrins, secreted proteins, and cell adhesion molecules which mediate tissue remodeling, migration, and cell-cell communication.

### Example 12 - Presto Blue Cell Viability Assay

To determine cell viability, the cell population of the invention is cultured in either a 24 well or 96 well format for 24-48 hours. Upon culture establishment, cells are exposed to NCE for a 24-96-hour period. Viability is assessed by mixing stock Presto blue reagent to KGM at a 1:10 ratio (1 part Presto Blue to 9 parts KGM), which is mixed well. The medium and NCE is then removed from each culture well and Presto blue is added in a small volume to the culture well (300ul/well/24 well) or (100ul/well/96 well) and incubated for 2 hours at 37°C. The medium is then removed and transferred to a 96 well plate and read at 530/590.

### Example 13 - Fluorimetric Caspase Assay

For the quantitative *in vitro* determination of caspases activity, the fluorimetric caspase assay is performed according to the Homogeneous Caspases Assay (Roche, Indianapolis, IN). First, 50µl of double concentrated apoptosis inducing agent is pipetted into the wells of a 96-well plate. The cell population of the invention is then seeded in duplicate onto prediluted apoptosis inducing agents at 4 x 10⁴ cells per well in 50µl of cell culture media. Cells are not seeded into the wells designated for the blank, standards, or positive control. 100µl of cell culture media is then pipetted only in duplicate to the wells designated for the blanks. 100µl of prediluted positive control is pipetted in duplicate to the wells designated for positive control. 100µl of prediluted standard solutions is pipetted in duplicate to the wells designated for the specific concentrations of standard. Then, 100µl of freshly prepared substrate working solution is pipetted onto each well. The 96-well plate is covered with lid and incubated for more than 1 hour at 37°C. Caspase activity is measured fluorimetrically using a plate reader with an excitation filter set at 470-500 nm and emission filter set at 500-560 nm.

### Example 14 - GGT Functional Enzyme Activity Assay

The assays for GGT (L-glutamic acid gamma-p-nitroanalide hydrochloride) enzyme activity can be performed on cells in suspension or on plated cells.

For cells in suspension, 500,000 cells are collected, centrifuged, and resuspended in 100 ul of KSFM. 20 µl of these cells are then transferred in duplicate to a 96-well plates. 20 µl of positive control lysate is added in duplicate to a 96-well plate. 180 µl of GGT reagent is added to test sample wells, 2 blank negative control wells, and 2 positive control wells. The GGT plate is incubated for 30 minutes at room temperature and then read on a plate reader at 405 nm.

For plated cells, 500,000 cells are plated into 6 wells of a 24-well plate in 50:50 KGM and allowed to grow to confluence for 3 days. Cells are washed 1x with PBS. 0.5 ml of ml of GGT reagent is added to each test well, 1 blank/negative control well, and 1 positive control well. 100 µl of positive control lysate is added to the positive control well. GGT designated wells are incubated for 1 hour at room temperature. After the 1 hour incubation, 200 µl of reagent is transferred from each well including reagent blank/negative control and the positive control, in duplicate to a 96-well plate and read at 405 nm.

### Example 15 - Method of Screening Self-generating Human Kidney Spheroid/Organoid Structures with NCE

In the present study, SRCs were exposed to escalating dose ranges of well known nephrotoxic drugs (ex. Aminoglycosides, chemotherapeutics, immuno-supressants, antimicrobials, and anti-retrovirals) and screened for toxicity and function. (Table 15.1) As shown herein, well known nephrotoxic compounds reproducibly and robustly inhibited primary human SRC functional activity in a dose-responsive manner using two- (2D) and three-dimensional (3D) human renal cell constructs that simulate human tissue physiology ex vivo.

**Table 15.1. 2D and 3D Ex-Vivo Screening**

| **Screen** | **Assays** |
|---|---|
| **Function** | (*GGT*, *LAP*, *Albumin uptake*, *migration*, *proliferation*) |
| **Toxicity** | (*Viability*, *Morphology*, *Apoptosis*, *LDH Et inhibition of function*) |
| **Genotypic** | (*DNA integrity* (*ploidy*)), *Gene array*) |
| **Phenotypic** | (*Kim-1*, *Osteopontin*, *NGAL*, *Clusterin*, *NAG*, *Collagen IV*, *GST*, *RPA*, *Timp-1*) |
| **Pharmacokinetics** | (GGT, LAP, OAT, OCT, MRP, MRD1, PKC) |

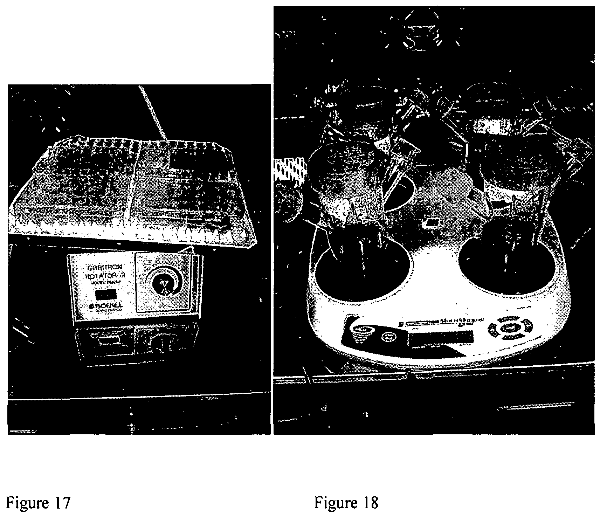

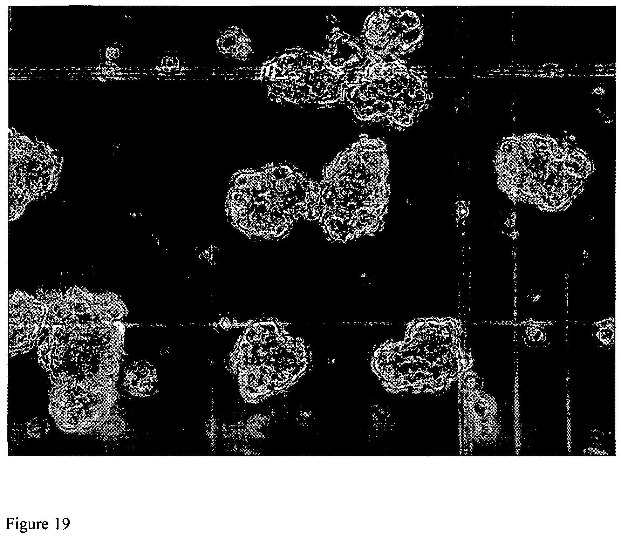

Human kidney cells were isolated using standard operating procedures for generating NKA, as described *supra.* Cells were expanded and sub-cultured through two passages prior to exposing to a low oxygen environment (2%O2) for 18 hours. After exposure, the cells were harvested and subjected to a two-step density gradient (7% and 16% w/v Optiprep) and centrifuged for 20 minutes at 800 x g without brake. The resulting band formed between the 7 and 16% layer was collected and washed (B2,B3,B4). The cells were counted and viability assessed. Spheroids were generated by either culturing cells (20-30 x10³ cells/cm²) in multi-well plates that were poly-HEMA coated to prevent attachment and placed on an orbital rotator in the incubator for 24 hrs (Figure 17). Alternatively, banded cells were resuspended in 75mls of kidney growth medium at a concentration of 1x10⁶ cells per ml and placed into a 125ml spinner flask (BD) onto a magnetic stirrer (4-40 rpm) inside an incubator at 37° C/5% CO₂ (Figure 18). The cells were left to self aggregate to generate spheroids for 24-48 hours prior to exposing to NCEs (Figure 18). The cells can either be exposed within the spinner flasks or can be transferred to smaller poly-HEMA coated mutliwell plates, which maintain spheroids, for dosing studies (Figure 19) over a period ranging from 24-96 hours. (Buzhor ET AL. Kidney Spheroids Recapitulate Tubular Organoids Leading to Enhanced Tubulogenic Potency of Human Kidney-Derived Cells Tissue Engineering Part A, 2011).

Similar assays to measure phenotypic changes (Table 15.2), function, viability, apoptosis can be applied to cells in suspension.

**Table 15.2. Examples of Functional Markers on Kidney Spheroids**

| **Marker** | **Function** |
|---|---|
| NKCC2 (Fig. 20) | Expressed in kidney where active reabsorbtion of sodium chloride is mediated |
| GGT-1 (Fig. 21) | GGT-1 initiates extracellular glutathione breakdown (GSH) |
| Aqp-1 (Fig. 22) | Proximal tubule marker associated with water transport |
| LAP-3 (Fig. 23) | Involved in the processing and turnover of intracellular proteins and amino acids |
| OAT-1 (Fig. 24) | Important in transporting anionic substrates and removing toxins |
| Cubilin (Fig. 25) | Functionally import when bound to Megalin required for internalization of cubilin bound ligands such as Albumin, vitamin B12, an apolipoprotein A1 |

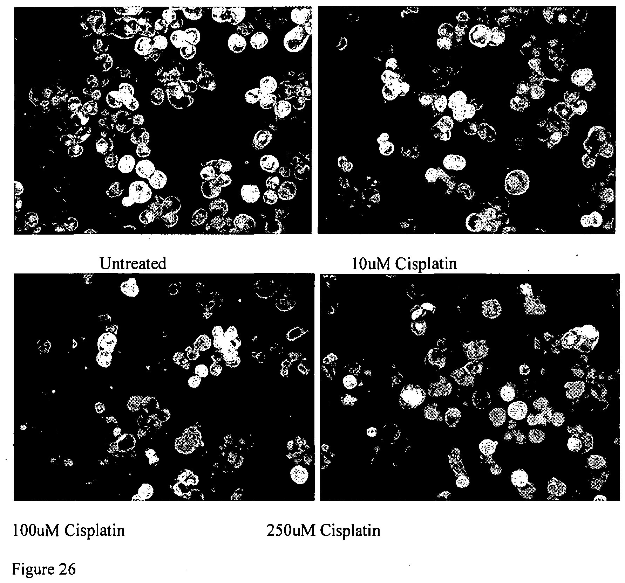

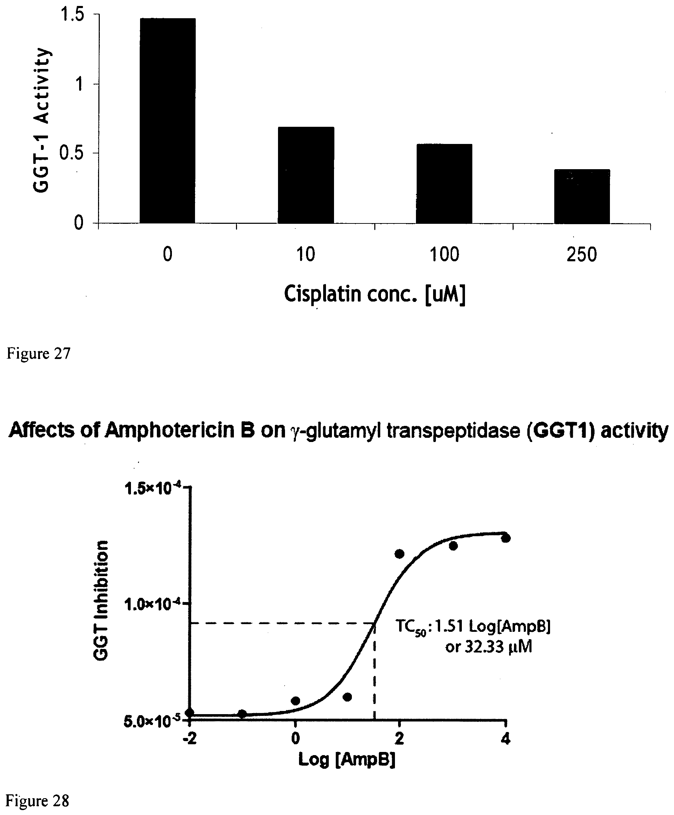

Spheroid viability post-exposure to cisplatin (post 48 hours) was assessed using Invitrogen Live/Dead Kit (L3224) is shown in Figure 26. Following exposure to escalating doses of cisplatin, the SRC tubular organoids displayed green where live cells were present and red where dead cells present. The number of dead cells appeared to increase as the test concentration of cisplatin was increased. Figure 27 depicts an ex-vivo 3D functional analysis based on the brush border enzyme assay for gamma glutamyl transpeptidase (GGT). Following 48 hours of cisplatin exposure at concentrations of either 0, 10, 100 or 250 micromolar (uM), the 3D tubular organoid cultures demonstrated a dose dependent decrease in GGT activity. Thus, cisplatin was shown to have a dose dependent inhibitory effect on SRC spheroid-mediated GGT-1 function and spheroid cell viability after 48 hrs of exposure.

### Example 16 - Morphological and functional changes to SRCs following Amphotericin B (Amp B) exposure

Morphological, genotypic, phenotypic and functional changes to 2D cultured SRC were monitored to 72hrs following exposure to escalating doses of the well known nephrotoxin, Amphotericin B (Amp B). The TC50 was estimated by Amp B knockdown of SRC/NKA ex vivo GGT activity.

GGT inhibition detected by western blot is shown in below in Table16.1.

**Table 16.1. Western blot density analysis; response = band density**

| **[c]** | **response** | **Log[c]** | **inv response** |
|---|---|---|---|
| 0.01 | 18764 | -2 | 5.32935E-05 |
| 0.1 | 18959 | -1 | 5.27454E-05 |
| 1 | 17112 | 0 | 5.84385E-05 |
| 10 | 16611 | 1 | 6.02011E-05 |
| 100 | 8227 | 2 | 0.000121551 |
| 1000 | 8000 | 3 | 0.000125 |
| 10000 | 7800 | 4 | 0.000128205 |

A semi-logrithmic plot of the inhibitory effects of Amp B on the SRC-mediated brush border-based enzymatic activity, γ-glutamyl transpeptidase (GGT), provided a sigmoidal curve with an IC50 of 32.33µM. TC₅₀ from semilogarithmic dose response curve showing the effects of Amphotercin B on GGT1 activity is shown in Figure 28. Results from semilogarithmic dose response analysis are shown below in Table 16.2.

**Table 16.2.**

| **Log(inhibitor) vs response** | |
|---|---|
| BOTTOM | 0.0001307 |
| TOP | 5.204e-005 |
| **LOGIC50** | **1.510** |
| **IC50** | **32.33** |
| Span | -7.862e-005 |
| Std. Error | |
| BOTTOM | 5.722e-006 |
| TOP | 4.608e-006 |
| LOGIC50 | 0.2126 |
| Span | 6.956e-006 |
| 95% Confidence Intervals | |
| BOTTOM | 0.0001148 to 0.0001465 |
| TOP | 3.925e-005 to 6.484e-005 |
| LOGIC 50 | 0.9195 to 2.100 |
| IC50 | 8.307 to 125.8 |
| Span | -9.792e-005 to -5.931e-005 |
| Goodness of Fit | |
| Degrees of Freedom | 4 |
| R² | 0.9702 |
| Absolute Sum of Squares | 2.435e-010 |
| Sy.x | 7.802e-006 |
| Number of points | 7 |

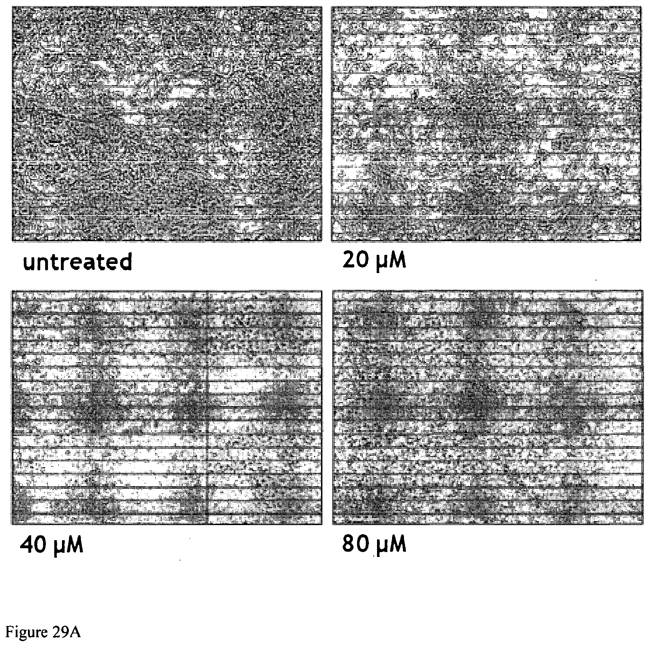

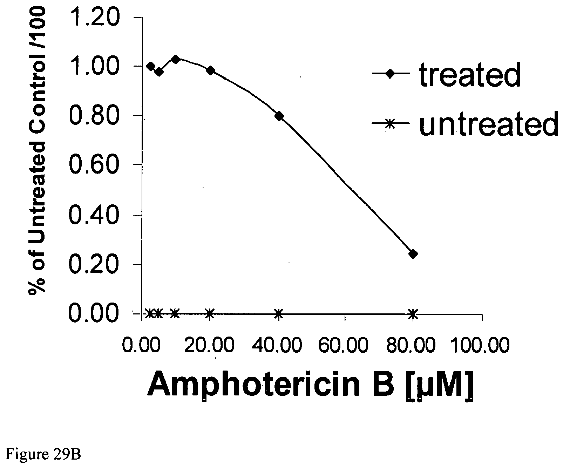

The Amp B IC50 strongly correlated to cell viability using Presto Blue and apoptotic-based caspase detection (described above). Morphological and viability changes post 72hr exposure to Amphotericin B are depicted in Figure 29A and represented by the percent change from untreated control by Presto Blue in Figure 29B.

The functional properties of 2-D and 3-D SRC cultures, with established in vivo regenerative properties, provide a platform for ascertaining drug safety, efficacy and possibly even pharmacokinetic outcomes for new chemical entities (NCEs). Therefore, the functional properties of this ex vivo renal cell platform is highly relevant towards reliably and reproducibly predicting clinical outcomes.

### Example 17 - In Vitro method for identifying a test agent suitable for therapeutic use

Development of a test agent suitable for therapeutic use ultimately requires evaluation *in vivo.* However, such testing can be lengthy and costly, so initial *in vitro* screening of the test agents to be evaluated is often performed. Effective *in vitro* screening must be sensitive enough to relevant responses to eliminate certain test agent candidates, allowing for only the most viable options to be tested *in vivo.* This approach results in a more efficient use of the *in vivo* resources, and potentially reduces the cost and time required to bring a product to market.

For identifying a test agent suitable for therapeutic use in a human subject having a kidney disorder, the cell populations provided herein, formulated as 2-D cultures, spheroids, and/or formulated in a biomaterial, may be subjected to the following assays to determine whether the test agent modulates the expression of a proliferative marker and/or an M2 phenotype of the cell populations, relative to a non-contacted control cell population.

Briefly, for 3-D cultures, the cell populations are cultured within the hydrogels (in 3-D) followed by exposure to a test agent, as described *supra*, and the resulting conditioned media is assayed for proliferative markers on a model human kidney cell line as indicators of trophic effects and for the ability to induce the M2 phenotype in differentiated monocytes as an indicator of remodelling potential.

**Materials and Methods:** Cell populations are isolated from human kidneys and cultured according to methods described *suupra.* Cell populations are mixed with a biomaterial hydrogel in 24-well transwell inserts so that the cells are uniformly suspended in the resulting gel. The hydrogels may include, for example, Matrigel (Growth-factor reduced, BD Biosciences), Alginate (VLVG, FMC BioPolymer), Collagen (rat tail type I, BD Biosciences), HyStem (thiol-modified hyaluronic acid crosslinked with PEGDA, Glycosan), or Extracel (HyStem also containing thiol-modified gelatin, Glycosan). The cells are then contacted with a test agent and cultured for 24h in serum-free media, after which the conditioned media is collected and assayed.

***Evaluation of Proliferative Marker***. Immortalized human kidney cells are treated with the 3-D cell culture supernatants. After 24h or 48h, cell lysates are prepared and analyzed by Western blotting for levels of PCNA (proliferative marker).

***Evaluation of M2 Phenotype***. THP-1 monocytes (ATCC) are treated with phorbol ester to induce differentiation. The macrophage-differentiated monocytes are treated with supernatants from the 3-D cell cultures for 24h, fixed, and stained with antibodies to CD 68 (pan macrophage) and CD163 (M2 macrophage phenotype). DAPI is used to stain the nucleus. High magnification images are collected and used to determine the M2 percentage in the overall macrophage population.

**Results.** The results of the above assays with test agents are analyzed with the non-contacted cell population in determining whether the test agent modulates the expression of a proliferative marker and/or an M2 phenotype of the heterogeneous renal cell population.

### Example 18 - Organoid/tubulogenesis-based potency assay for NKA

*In vitro* organoid/tubulogenesis-based assays have been developed for determining the potential regenerative bioactivity *in vivo* (i.e., potency) of NKA. NKA site specific engraftment and *de novo* regeneration, *i.e*., formation of organoids and/or tubulogenesis and/or glomerulogenesis, may be determined through the use of these *in vitro* potency assays. Formation of renal organoids and tubules has been found to be an indicator of NKA product potency.

### Material and Methods

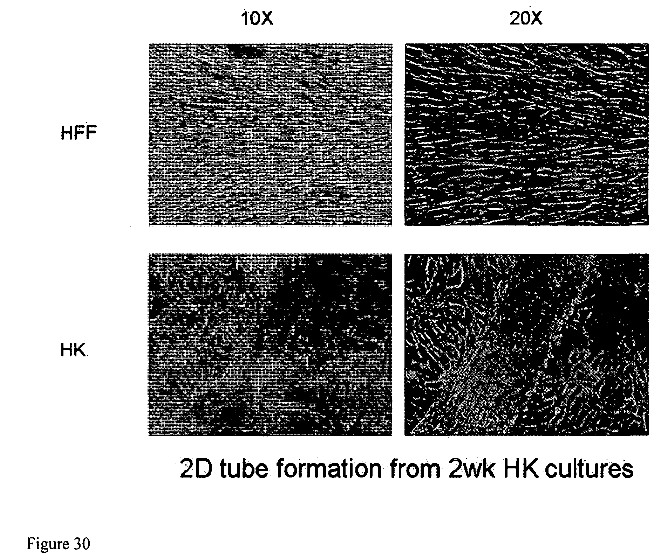

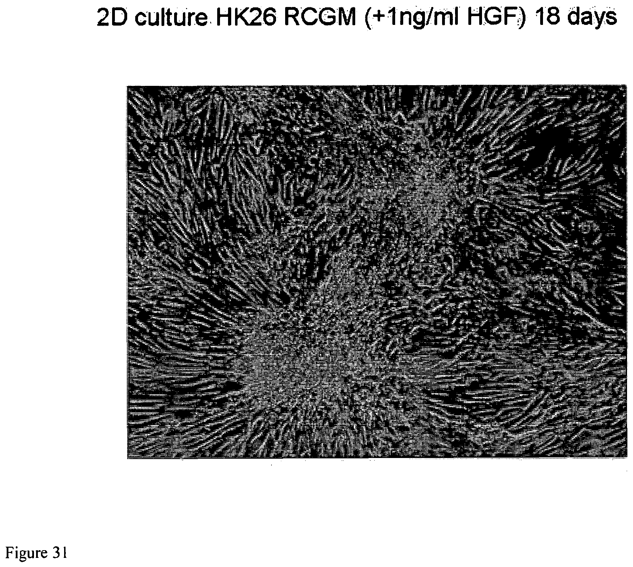

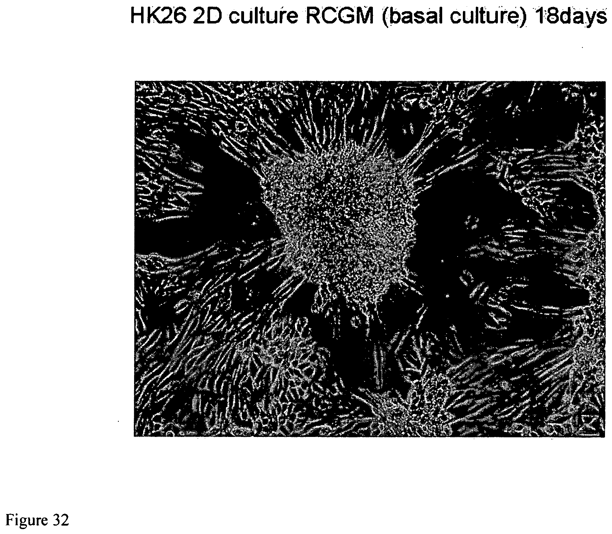

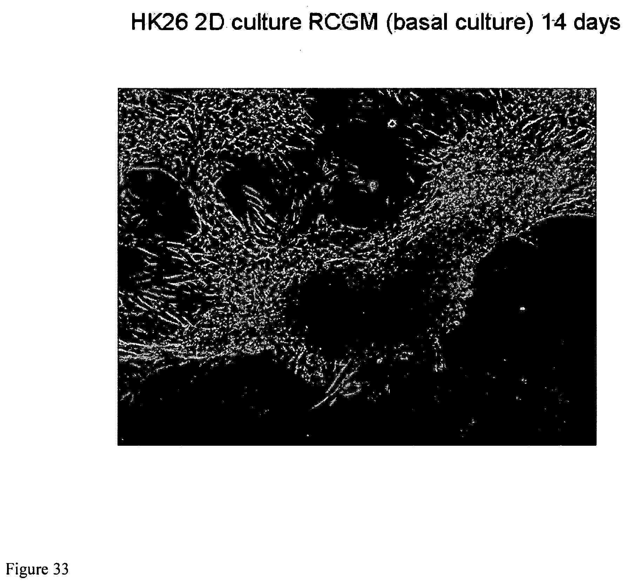

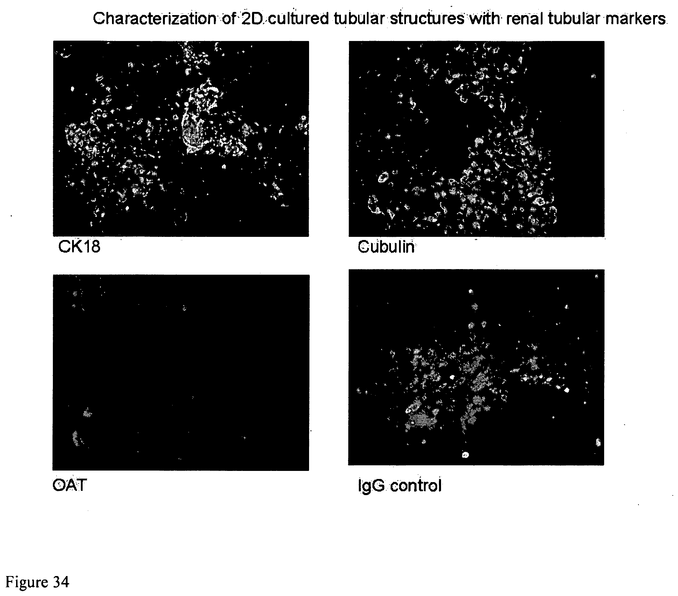

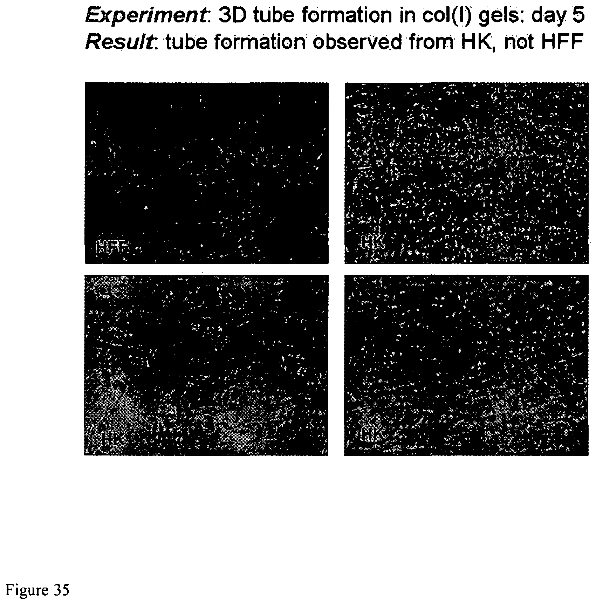

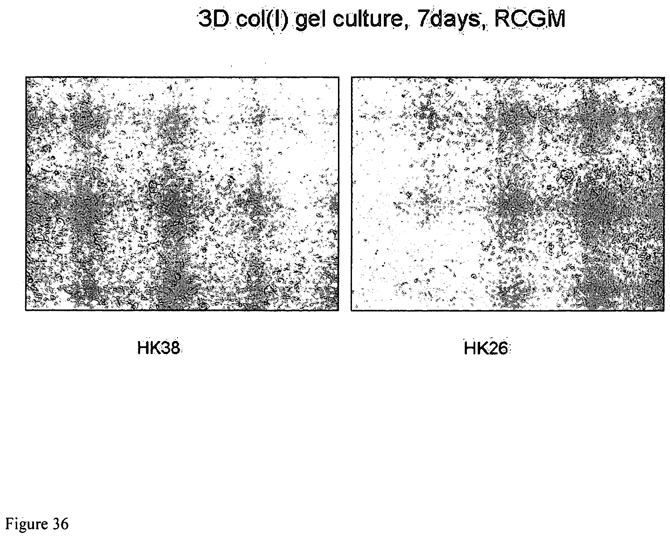

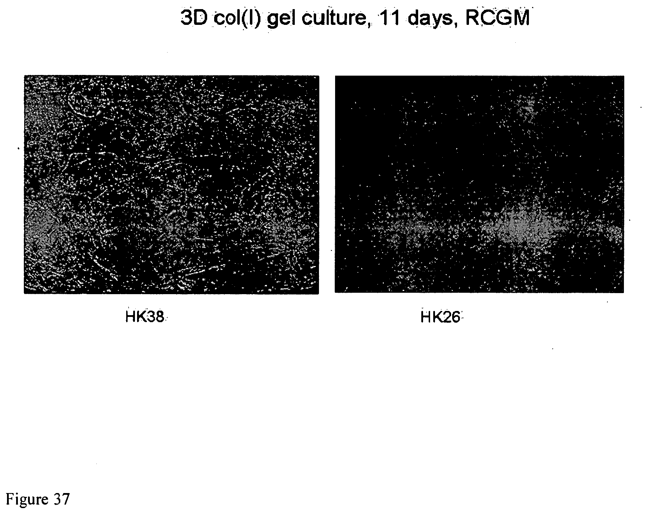

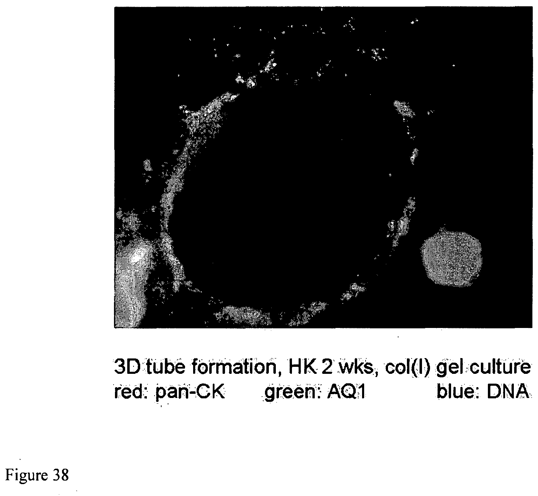

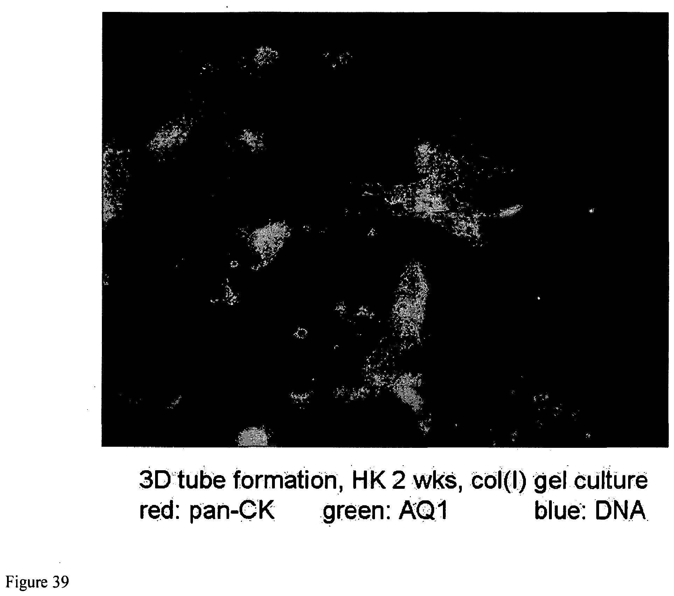

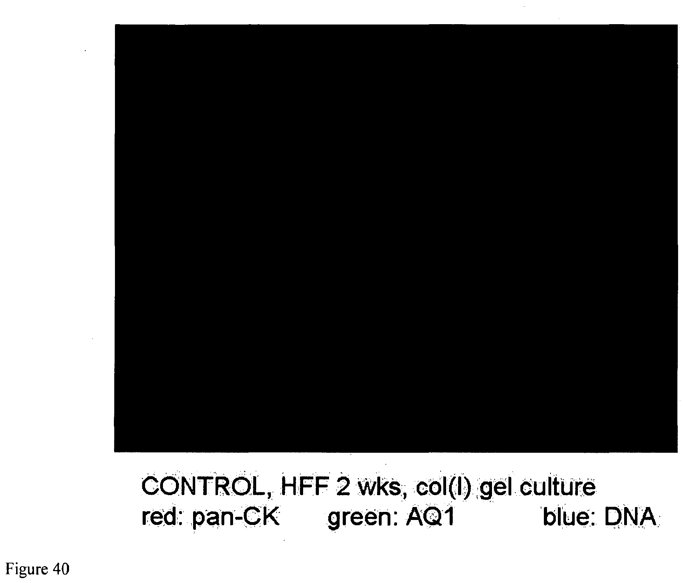

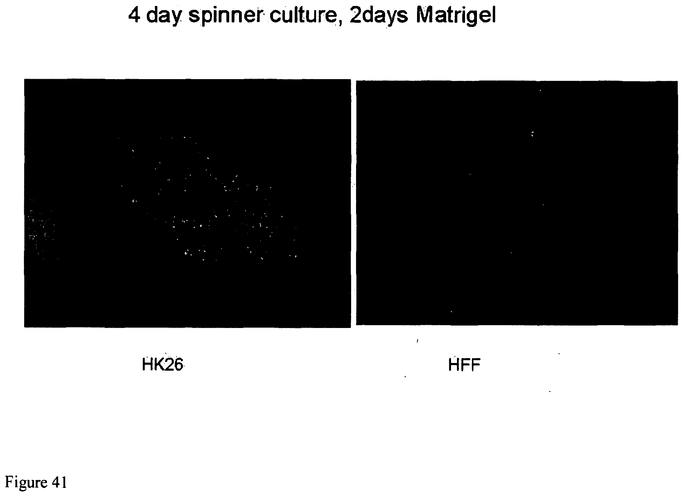

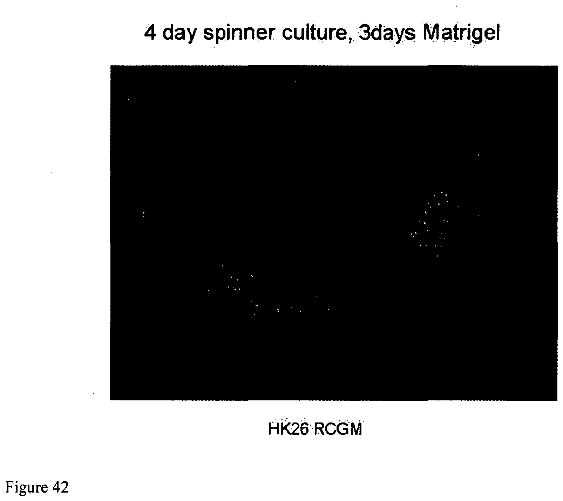

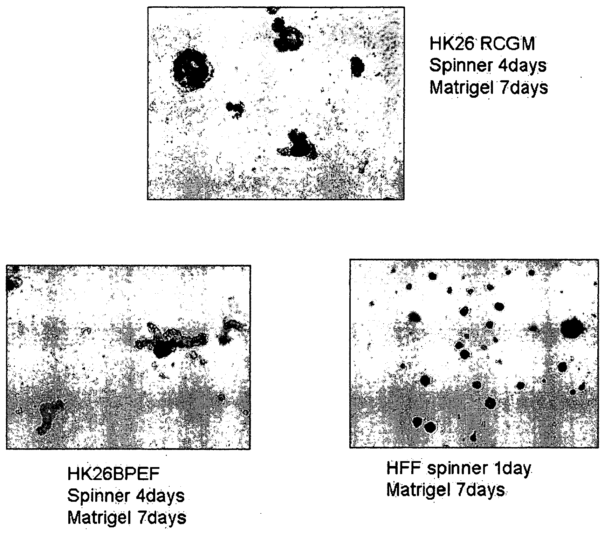

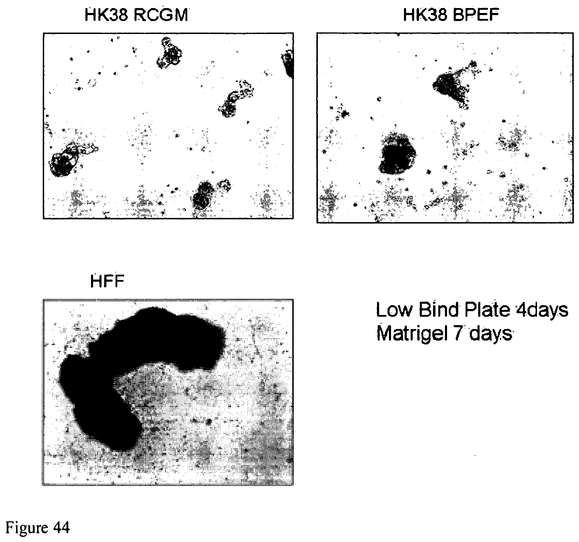

Formation of organoids and tubules from NKA may be induced as follows:
**1) 2D culture**. Formulated NKA were seeded on standard 2D, 6-well cell culture plastic-ware at a density of 5000 cells/cm² in RCGM (Renal Cell Complete Growth Media). Cell populations were grown past confluence for 7-15 days (with regular changes of media every 3-4 days), during which time cells demonstrated spontaneous self-organization into spheroidal structures (referred to as "organoids") and tubules (**Figures 30-33**). These structures were characterized for expression of tubular markers such as cubulin, cytokeratins, OAT (**Figure 34**). Formation of such structures is unique to renal cells as unrelated cell types such as human foreskin fibroblasts (HFF) do not form such structures (**Figure 30**).
**2) 3D culture.** Formulated NKA was incorporated into a collagen (I) gel, collagen (IV) gel, Matrigel or a mixture of any of these as previously described (see Guimaraes-Souza et al., 2012. In vitro reconstitution of human kidney structures for renal cell therapy. Nephrol Dial Transplant 0: 1-9). Briefly, the liquid gel was brought to a neutral pH and NKA mixed in at 1000 cells/ul (this concentration can be varied). The cell/gel mixture was aliquoted into a well of a 24 well plate (200-400ul/well) and allowed to solidify at 37 degrees C for several hours. Cell culture media was then added and the cultures allowed to mature for 4-10 days with regular changes of media. Networks of tubular structures organized as lattices and rings (tubules seen in cross-section) were formed throughout the gel matrix by NKA (see **Figures 35-37**). Formation of these structures is unique to renal cells and is not seen in unrelated cell lines (HFF, see **Figure 35**). These tubules were characterized for expression of tubular markers as shown in **Figures 38-40**.
**3) Spinner culture**. Spheroidal structures loosely referred to as organoids may be formed by suspension culture of many cell types in spinner flasks or low-bind plasticware. Here, 30-40e6 cells (NKA and HFF) were cultured in 45ml media in spinner flasks for up to 4 days (80rpm). Spheroids were then further cultured for 7-10 days on Matrigel coated plates. As shown in **Figures 41-44**, spheroids formed from NKA show tubulogenic potential as shown by de novo budding of tubular structures from cultured spheroids. No such potential is observed from spheroids derived from HFF. Therefore, spheroids derived from NKA present true organogenic potential (potency) unique to renal cell populations.

**Summary:** Taken together, these data demonstrate that:
1) Tubules and spheroidal structures called organoids spontaneously self-assemble from 2D cultures of NKA;
2) Tubules and organoids with tubulogenic potential spontaneously self-assemble from 3D cultures of NKA;
3) Such organogenic bioactivity is restricted to renal cell populations such as NKA (and related cell populations that present branching morphogenesis during development such as mammary gland, lung etc), is not observed from unrelated cells such as HFF, and may therefore be used as an *in vitro* diagnostic indicator of potential regenerative bioactivity *in vivo* (i.e., potency).

The following numbered clauses contain further statements of various aspect of the present invention:-
1. A method of determining a level of renal toxicity of a test agent comprising
   a) culturing a plurality of concentrations of a test agent with a heterogeneous renal cell population comprising a B2 cell population comprising an enriched population of tubular cells, and wherein the heterogeneous renal cell population is depleted of a B1 cell population; and
   b) determining a level of toxicity of said test agent, wherein the presence of at least one toxicity indicator is indicative of the level of renal toxicity of said test agent.
2. The method of clause 1, wherein the heterogeneous renal cell population further comprises a B4 cell population comprising one or more of erythropoietin (EPO)-producing cells, glomerular cells and vascular cells.
3. The method of clause 2, wherein the heterogeneous renal cell population further comprises a B3 cell population.
4. The method of clause 3, wherein the heterogeneous renal cell population further comprises a B5 cell population.
5. The method of clause 1, wherein the cell population is cultured as spheroids.
6. The method of clause 1, wherein the heterogeneous renal cell population is cultured on a matrix.
7. The method of clause 6, wherein the matrix is a three-dimensional (3-D) matrix.
8. The method of any of the preceding clauses, wherein the toxicity indicator is decreased GGT expression.
9. The method of any of the preceding clauses, wherein the toxicity indicator is a change Aquaporin-1 expression relative to control.
10. The method of any of the preceding clauses, wherein the toxicity indicator is a change in Aquaporin-2 expression relative to control.
11. The method of any of the preceding clauses, wherein the toxicity indicator is LDH.
12. The method of any of the preceding clauses, wherein the toxicity indicator is a change in phenotype of the cell population relative to control.
13. The method of clause 1, wherein the determination of the level of renal toxicity of the test agent comprises calculating a TC50 for the test agent.
14. A method for determining metabolism of a test agent comprising
   a) incubating a test agent and an enzyme with a heterogeneous renal cell population comprising a B2 cell population comprising an enriched population of tubular cells, and wherein the heterogeneous renal cell population is depleted of a B1 cell population; and
   b) detecting one or more metabolites of the test agent.
15. An *in vitro* method for identifying a test agent suitable for therapeutic use in a human subject having a kidney disorder comprising
   a) contacting a test agent with a heterogeneous renal cell population comprising a B2 cell population that is characterized by a phenotype selected from the group consisting of expression of a proliferative marker and an M2 phenotype, wherein the B2 cell population comprises an enriched population of tubular cells; and
   b) determining whether the test agent modulates the expression of a proliferative marker and/or an M2 phenotype of the heterogeneous renal cell population relative to a non-contacted control cell population.
16. The method of clause 15, wherein the heterogeneous renal cell population further comprises a B4 cell population comprising one or more of erythropoietin (EPO)-producing cells, glomerular cells and vascular cells.
17. The method of clause 15 or 16, wherein the heterogeneous renal cell population is cultured on a matrix.
18. The method of clause 17, wherein the matrix is a three-dimensional (3-D) matrix.
19. An organoid comprising a heterogeneous renal cell population comprising a B2 cell population comprising an enriched population of tubular cells, and wherein the heterogeneous renal cell population is depleted of a B1 cell population.
20. A method of forming an organoid comprising a heterogeneous renal cell population comprising a B2 cell population comprising an enriched population of tubular cells, and wherein the heterogeneous renal cell population is depleted of a B1 cell population, comprising culturing the heterogenerous renal cell population in a culture system selected from the group consisting of i) 2D culture; ii) 3D culture: COL(I) gel; iii) 3D culture: Matrigel; iv) 3D culture: spinners, followed by COL(I)/Matrigel; and v) 3D culture: COL(IV) gel.
21. A method of determining a regenerative potential of a heterogeneous cell population comprising a B2 cell population comprising an enriched population of tubular cells, and wherein the heterogeneous renal cell population is depleted of a B1 cell population, comprising
   a) culturing the heterogeneous renal cell population; and
   b) determining the regenerative potential of the heterogeneous cell population, wherein the formation of tubules and/or organoids is indicative of a regenerative potential.

## Claims

1. An *in vitro* method for identifying a test agent suitable for therapeutic use in a human subject having a kidney disorder comprising
a) contacting a test agent with a heterogeneous renal cell population enriched for a renal tubular cell population that is **characterized by** a phenotype selected from the group consisting of expression of a proliferative marker and an M2 phenotype; and
b) determining whether the test agent modulates the expression of a proliferative marker and/or an M2 phenotype of the heterogeneous renal cell population relative to a non-contacted control cell population.

2. The method of claim 1, wherein the heterogeneous renal cell population further comprises glomerular cells or vascular cells.

3. The method of claim 1 or 2, wherein the heterogeneous renal cell population comprises cells that express GGT-1.

4. The method of any one of claims 1-3, wherein the heterogeneous renal cell population is cultured on a matrix.

5. The method of claim 4, wherein the matrix is a three-dimensional (3-D) matrix.

6. An *in vitro* method of determining a regenerative potential of an isolated renal cell population comprising
a) culturing the isolated renal cell population in vitro in a culture system selected from a 2D culture system and a 3D culture system; and
b) detecting the presence of tubule and/or organoid formation in the culture system;
wherein the presence of tubule and/or organoid formation is indicative of a regenerative potential of the isolated renal cell population, and wherein the isolated renal cell population is enriched for a renal tubular cell population.

7. The method of claim 6, wherein the 3D culture is selected from the group
a) COL(I) gel;
b) Matrigel;
c) spinners, followed by a COL(I) gel or a Matrigel; and
d) COL (IV) gel.

8. The method of claim 6 or 7, wherein the renal cell population further comprises glomerular cells or vascular cells.

9. The method of any one of claims 6-8, wherein the renal cell population comprises cells that express GGT-1.

10. The method of any one of claims 6-9, wherein the tubule and/or organoid formation is (i) quantitated by determining the total number of tubules/organoids per field or per unit area of 2D cell surface growth or per unit volume of 3D culture, or (ii) detected by staining the tubules/organoids with a fluorescent dye.

11. The method of any one of claims 6-9, wherein the tubule forms lattices and rings.

12. The method of any one of claims 6-9, wherein the tubule and/or organoid expresses tubular markers, and wherein, optionally, the tubular markers is selected from cubulin, cytokeratins, and OAT.
